(19) Europäisches Patentamt
European Patent Office
Office européen des brevets

(11) EP 3 389 603 B1

(12) EUROPEAN PATENT SPECIFICATION

(45) Date of publication and mention of the grant of the patent:
**15.07.2020 Bulletin 2020/29**

(21) Application number: **16804815.5**

(22) Date of filing: **01.12.2016**

(51) Int Cl.:
***A61K 8/29*** *(2006.01)* ***A61K 8/36*** *(2006.01)*
***A61Q 1/02*** *(2006.01)*

(86) International application number:
**PCT/EP2016/079451**

(87) International publication number:
**WO 2017/102359 (22.06.2017 Gazette 2017/25)**

# (54) COMPOSITION OF GEL/GEL TYPE BASED ON HYDROPHOBIC COATED PIGMENTS AND A LIQUID FATTY ACID AND/OR A GLYCOL COMPOUND

ZUSAMMENSETZUNG VOM GEL/GEL-TYP AUF BASIS VON HYDROPHOB BESCHICHTETEN PIGMENTEN UND EINER FLÜSSIGEN FETTSÄURE UND/ODER EINER GLYKOLVERBINDUNG

COMPOSITION DE GEL/DU TYPE GEL BASÉE SUR DES PIGMENTS HYDROPHOBES ENROBÉS ET UN ACIDE GRAS LIQUIDE ET/OU UN COMPOSÉ GLYCOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2015 FR 1562601**
**17.12.2015 FR 1562607**

(43) Date of publication of application:
**24.10.2018 Bulletin 2018/43**

(60) Divisional application:
**19187533.5 / 3 578 160**

(73) Proprietor: **L'Oréal**
**75008 Paris (FR)**

(72) Inventor: **VALVERDE, Elodie**
**94152 Chevilly La Rue (FR)**

(74) Representative: **L'Oreal**
**Service D.I.P.I.**
**9, rue Pierre Dreyfus**
**92110 Clichy (FR)**

(56) References cited:
**WO-A1-2014/161722**
**WO-A1-2014/167543**
**WO-A1-2016/030839**
**US-A1- 2001 044 475**
**US-A1- 2005 112 072**
**US-A1- 2013 071 453**
**US-A1- 2013 156 831**
**US-A1- 2014 356 402**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 389 603 B1

## Description

The present invention is directed towards proposing for the field of caring for and/or making up keratin materials, especially the skin and/or the lips, and in particular the skin and keratin fibres, especially the eyebrows, a novel galenical form that is most particularly advantageous with regard to its technical performance and the sensations it affords the user during its application thereto, in particular to the skin.

The term "keratin materials" especially means the skin, the lips, the eyebrows and/or the eyelashes, in particular the skin and/or the eyebrows, and preferably the skin.

Cosmetic compositions, for example foundations, are commonly used to give the skin an aesthetic colour, but also to hide and/or unify imperfections of the skin relief such as wrinkles and/or fine lines and/or scars. In this regard, many solid or fluid, anhydrous or non-anhydrous formulations have been developed to date.

Multi-phase compositions exist at the present time, which are advantageous as regards the makeup properties they impart, especially a matt effect and coverage, and persistence of the makeup.

"Gel-gel" compositions have already been proposed in the cosmetics field and are particularly advantageous as alternatives to emulsions, which have a tendency to give a substantial greasy and tacky sensation, lack of freshness and lack of lightness for the textures obtained. Formulations of this type combine a gelled aqueous phase with a gelled oily phase. Thus, gel/gel formulations are described in Almeida et al., Pharmaceutical Development and Technology, 2008, 13:487, tables 1 and 2, page 488; WO 99/65455; BRPI 0405758-9; WO 99/62497; JP 2005-112834 and WO 2008/081175, FR 2 984 736, FR 3 002 444, FR 3 004 343, WO 14/128 680, WO2014/128679 and WO 14/128 678. However, these formulations are not entirely satisfactory. Specifically, the use of particular materials such as hydrophobic-treated pigments in high contents in the fatty phase of compositions of gel/gel type has a tendency to increase the viscosity of said fatty phase. A substantial difference in viscosity between the gel of the aqueous phase and the gel of the fatty phase has a tendency to lead to compositions of gel/gel type that are macroscopically non-homogeneous: the highly concentrated pigmented oily gel disperses in macro-domains which are then visible to the naked eye and gives the product an unaesthetic appearance as a lack of smoothness and/or lack of gloss. These macroscopic heterogeneities may lead to degradation of the sensory perception on application (i.e.: freshness, lightness, emollience, comfort, coverage of imperfections) and also to a loss of stability of the composition over time.

There thus remains a need to find novel formulations of the gel-gel type which give good makeup properties such as a matt effect, good coverage, good sensory properties such as freshness and lightness on application and also good persistence over time without the drawbacks mentioned previously as macroscopic heterogeneities.

The Applicant has discovered, surprisingly, that this objective can be achieved with a composition **of gel-gel type,** especially comprising a physiologically acceptable medium, especially for coating keratin materials, more particularly for making up and/or caring for keratin materials, containing:

- at least one aqueous phase gelled with at least one **synthetic** hydrophilic gelling agent selected **from 2-acrylamido-2-methylpropanesulfonic acid polymers and copolymers;** and
- at least one oily phase gelled with at least one lipophilic gelling agent which is an **organopolysiloxane elastomer;** said phases forming therein a macroscopically homogeneous mixture and said composition also comprising:
- at least one hydrophobic-coated pigment; and
- at least one liquid fatty acid of formula (1) below:

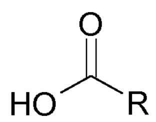

in which R is chosen from:

a) a saturated, branched $C_{14}$-$C_{22}$, preferably C18, alkyl group, or
b) an alkyl group comprising at least one linear or branched $C_{14}$-$C_{22}$, preferably $C_{18}$, double bond; and/

- **optionally** at least one glycol compound of formula (2) below:

$$CH_3\text{--}[CH_2CH_2]_m\text{--}[COO]_n\text{-}CH_2\text{-}CH(OH)\text{-}CH_2OH \qquad (2)$$

in which:

- m is between 2 and 4
- n is equal to 0 or 1;

said gelled oily phase comprising at least one polar oil when the composition comprises at least one glycol compound of formula (2).

This discovery forms the basis of the invention.

Thus, according to one of its aspects, the present invention relates to a composition **of gel-gel type,** especially comprising a physiologically acceptable medium, especially for coating keratin materials, more particularly for making up and/or caring for keratin materials, containing:

- at least one aqueous phase gelled with at least one **synthetic** hydrophilic gelling agent selected **from 2-acrylamido-2-methylpropanesulfonic acid polymers and copolymers;** and
- at least one oily phase gelled with at least one lipophilic gelling agent which is **an organopolysiloxane elastomer;** said phases forming therein a macroscopically homogeneous mixture and said composition also comprising:
- at least one hydrophobic-coated pigment; and
- at least one liquid fatty acid of formula (1) below:

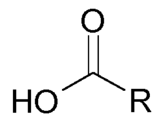

in which R is chosen from:

a) a saturated, branched $C_{14}$-$C_{22}$, preferably C18, alkyl group, or
b) an alkyl group comprising at least one linear or branched $C_{14}$-$C_{22}$, preferably $C_{18}$, double bond; and

- **optionally** at least one glycol compound of formula (2) below:

$$CH_3\text{--}[CH_2CH_2]_m\text{--}[COO]_n\text{-}CH_2\text{-}CH(OH)\text{-}CH_2OH \qquad (2)$$

in which:

- m is between 2 and 4
- n is equal to 0 or 1;

said gelled oily phase comprising at least one polar oil when the composition comprises at least one glycol compound of formula (2).

The invention also relates to a process for coating keratin materials, more particularly for making up and/or caring for keratin materials, such as the skin, characterized in that it comprises the application to the keratin materials of a composition as defined previously.

## Definitions

In the context of the present invention, the term "keratin material" means especially the skin (body, face, area around the eyes), the lips, the eyelashes and the eyebrows. More particularly, the term "keratin material" means the skin.

The term "physiologically acceptable" means compatible with the skin and/or its integuments, which has a pleasant colour, odour and feel, and which does not cause any unacceptable discomfort (stinging, tautness or redness) liable to discourage the consumer from using this composition.

The term "liquid fatty acid of formula (1)" means any compound of formula (1) that is in liquid form at room temperature (25°C) and atmospheric pressure ($10^5$ Pa).

## GEL-GEL

To begin with, it is important to note that a composition according to the invention of gel-gel type is different from an emulsion.

An emulsion is generally constituted by an oily liquid phase and an aqueous liquid phase. It is a dispersion of droplets of one of the two liquid phases in the other. The size of the droplets forming the dispersed phase of the emulsion is typically about a micrometre (0.1 to 100 $\mu$m). Furthermore, an emulsion requires the presence of a surfactant or of an emulsifier to ensure its stability over time.

In contrast, a composition according to the invention consists of a macroscopically homogeneous mixture of two immiscible gelled phases. These two phases both have a gel-type texture. This texture is especially reflected visually by a consistent and/or creamy appearance.

The term *"macroscopically homogeneous mixture"* means a mixture in which each of the gelled phases cannot be individualized by the naked eye.

More precisely, in a composition according to the invention, the gelled aqueous phase and the gelled oily phase interpenetrate and thus form a stable, consistent product. This consistency is achieved by mixing interpenetrated macrodomains. These interpenetrated macrodomains are not measurable objects. Thus, by microscope, the composition according to the invention is very different from an emulsion. A composition according to the invention cannot be characterized either as having a *"sense",* i.e. an O/W or W/O sense.

Thus, a composition according to the invention has a consistency of gel type. The stability of the composition is long-lasting without surfactant. Consequently, a composition, especially a cosmetic composition, according to the invention does not require any surfactant or silicone emulsifier to ensure its stability over time.

It is known practice from the prior art to observe the intrinsic nature of a mixture of aqueous and oily gels in a composition of gel-gel type, for example, by introducing a dyestuff either into the aqueous gelled phase or into the lipophilic gelled phase, before the formation of the composition of gel-gel type. During visual inspection, in a composition of gel-gel type, the dyestuff appears uniformly dispersed, even if the dye is present solely in the gelled aqueous phase or in the gelled oily phase. Specifically, if two different dyes of different colours are introduced, respectively, into the oily phase and into the aqueous phase, before formation of the composition of gel-gel type, the two colours may be observed as being uniformly dispersed throughout the composition of gel-gel type. This is different from an emulsion in which, if a dye, which is soluble in water or soluble in oil, is introduced, respectively, into the aqueous and oily phases, before forming the emulsion, the colour of the dye present will only be observed in the outer phase (Remington: The Science and Practice of Pharmacy, 19th Edition (1995), Chapter 21, page 282).

It is also known practice to distinguish a composition of gel-gel type from an emulsion by performing a "drop test". This test consists in demonstrating the bi-continuous nature of a composition of gel-gel type. Specifically, as mentioned previously, the consistency of a composition is obtained by means of the interpenetration of the aqueous and oily gelled domains. Consequently, the bi-continuous nature of a composition of gel-gel type may be demonstrated by means of a simple test with, respectively, hydrophilic and hydrophobic solvents. This test consists in depositing, firstly, one drop of a hydrophilic solvent on a first sample of the test composition, and, secondly, one drop of a hydrophobic solvent on a second sample of the same test composition, and in analysing the behaviour of the two drops of solvents. In the case of an O/W emulsion, the drop of hydrophilic solvent diffuses into the sample and the drop of hydrophobic solvent remains at the surface of the sample. In the case of a W/O emulsion, the drop of hydrophilic solvent remains at the surface of the sample and the drop of hydrophobic solvent diffuses throughout the sample. Finally, in the case of a composition of gel-gel type (bi-continuous system), the hydrophilic and hydrophobic drops diffuse throughout the sample.

In the case of the present invention, the test that will be preferred for distinguishing a composition of gel-gel type from an emulsion is a dilution test. Specifically, in a composition of gel-gel type, the aqueous and oily gelled domains interpenetrate and form a consistent and stable composition, in which the behaviour in water and in oil is different from the behaviour of an emulsion. Consequently, the behaviour during dilution of a composition of gel-gel type (bi-continuous system) may be compared to that of an emulsion.

More specifically, the dilution test consists in placing 40 g of product and 160 g of dilution solvent (water or oil) in a 500 mL plastic beaker. The dilution is performed with controlled stirring to avoid any emulsification. In particular, this is performed using a planetary mixer: Speed Mixer TM DAC400FVZ®. The speed of the mixer is set at 1500 rpm for 4 minutes. Finally, observation of the resulting sample is performed using a light microscope at a magnification of $\times$ 100 ($\times$10$\times$10). It may be noted that oils such as Parleam® and Xiameter PMX-200 Silicone Fluid 5CS® sold by Dow Corning are suitable as dilution solvent, in the same respect as one of the oils contained in the composition.

In the case of a composition of gel-gel type (bi-continuous system), when it is diluted in oil or in water, a heterogeneous appearance is always observed. When a composition of gel-gel type (bi-continuous system) is diluted in water, pieces of oily gel in suspension are observed, and when a composition of gel-gel type (bi-continuous system) is diluted in oil, pieces of aqueous gel in suspension are observed.

In contrast, during dilution, emulsions have a different behaviour. When an O/W emulsion is diluted in an aqueous solvent, it gradually reduces without having a heterogeneous and lumpy appearance. This same O/W emulsion, on dilution with oil, has a heterogeneous appearance (pieces of O/W emulsion suspended in the oil). When a W/O emulsion is diluted with an aqueous solvent, it has a heterogeneous appearance (pieces of W/O emulsion suspended in the water). This same W/O emulsion, when diluted in oil, gradually reduces without having a heterogeneous and

lumpy appearance.

According to the present invention, the aqueous gelled phase and the oily gelled phase forming a composition according to the invention are present therein in a weight ratio ranging from 95/5 to 5/95. More preferentially, the aqueous phase and the oily phase are present in a weight ratio ranging from 30/70 to 80/20.

The ratio between the two gelled phases is adjusted according to the desired cosmetic properties.

Thus, in the case of a makeup composition, in particular for the face, it will be advantageous to favour an aqueous gelled phase/oily gelled phase weight ratio of greater than 1, especially ranging from 60/40 to 90/10, preferably ranging from 60/40 to 80/20, preferentially from 60/40 to 70/30 and even more preferentially to favour an aqueous gelled phase/oily gelled phase weight ratio of 60/40 or 70/30.

These preferred ratios are particularly advantageous for obtaining fresh and light compositions.

Advantageously, a composition according to the invention may thus be in the form of a creamy gel with a minimum stress below which it does not flow unless it has been subjected to an external mechanical stress.

As emerges from the text hereinbelow, a composition according to the invention may have a minimum threshold stress of 1.5 Pa and in particular greater than 10 Pa.

It may also advantageously have a stiffness modulus G* at least equal to 400 Pa and preferably greater than 1000 Pa.

According to an advantageous embodiment variant, the gelled phases under consideration to form a composition according to the invention may have, respectively, a threshold stress of greater than 1.5 Pa and preferably greater than 10 Pa.

Characterization of the threshold stresses is performed by oscillating rheology measurements. Methodology is proposed in the illustrative chapter of the present text.

In general, the corresponding measurements are taken at 25°C using a Haake® RS600 imposed-stress rheometer equipped with a plate-plate measuring body (60 mm diameter) fitted with an anti-evaporation device (bell jar). For each measurement, the sample is placed delicately in position and the measurements start 5 minutes after placing the sample in the jaws (2 mm). The test composition is then subjected to a stress ramp from $10^{-2}$ to $10^{3}$ Pa at a set frequency of 1 Hz.

A composition according to the invention may also have a certain elasticity. This elasticity may be characterized by a stiffness modulus G* which, under this minimum stress threshold, may be at least equal to 400 Pa and preferably greater than 1000 Pa. The value G* of a composition may be obtained by subjecting the composition under consideration to a stress ramp from $10^{-2}$ to $10^{3}$ Pa at a set frequency of 1 Hz.

## HYDROPHILIC GELLING AGENT

For the purposes of the present invention, the term "hydrophilic gelling agent" means a compound that is capable of gelling the aqueous phase of the compositions according to the invention.

The gelling agent is hydrophilic and is thus present in the aqueous phase of the composition.

The gelling agent may be water-soluble or water-dispersible.

As stated above, the aqueous phase of a composition according to the invention is gelled with at least one hydrophilic gelling agent.

The hydrophilic gelling agent may be chosen from synthetic polymeric gelling agents, polymeric gelling agents that are natural or of natural origin, mixed silicates and fumed silicas, and mixtures thereof.

Preferably, the hydrophilic gelling agent may be chosen from synthetic polymeric gelling agents.

### Synthetic polymeric gelling agents

For the purposes of the invention, the term "synthetic" means that the polymer is neither naturally existing nor a derivative of a polymer of natural origin.

The synthetic polymeric hydrophilic gelling agent under consideration according to the invention may or may not be particulate.

For the purposes of the invention, the term "particulate" means that the polymer is in the form of particles, preferably spherical particles.

As emerges from the text hereinbelow, the polymeric hydrophilic gelling agent is sen from 2-acrylamido-2-methylpropanesulfonic acid polymers and copolymers as defined below.

#### *2-acrylamido-2-methylpropanesulfonic acid polymers and copolymers*

The polymers used that are suitable as aqueous gelling agent for the invention may be crosslinked or non-crosslinked homopolymers or copolymers comprising at least the 2-acrylamidomethylpropanesulfonic acid (AMPS®)

monomer, in a form partially or totally neutralized with a mineral base other than aqueous ammonia, such as sodium hydroxide or potassium hydroxide.

They are preferably totally or almost totally neutralized, i.e. at least 90% neutralized.

These AMPS® polymers according to the invention may be crosslinked or non-crosslinked.

When the polymers are crosslinked, the crosslinking agents may be chosen from the polyolefinically unsaturated compounds commonly used for crosslinking polymers obtained by radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also the allylic esters of phosphoric and/or vinylphosphonic acid derivatives, or mixtures of these compounds.

According to one preferred embodiment of the invention, the crosslinking agent is chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS® polymers that are suitable for use in the invention are water-soluble or water-dispersible. In this case, they are:

- either "homopolymers" comprising only AMPS® monomers and, if they are crosslinked, one or more crosslinking agents such as those defined above;
- or copolymers obtained from AMPS® and from one or more hydrophilic or hydrophobic ethylenically unsaturated monomers and, if they are crosslinked, one or more crosslinking agents such as those defined above. When said copolymers comprise hydrophobic ethylenically unsaturated monomers, these monomers do not comprise a fatty chain and are preferably present in small amounts.

For the purpose of the present invention, the term *"fatty chain"* means any hydrocarbon-based chain comprising at least 7 carbon atoms.

The term *"water-soluble or water-dispersible"* means polymers which, when introduced into an aqueous phase at 25°C, at a mass concentration equal to 1%, make it possible to obtain a macroscopically homogeneous and transparent solution, i.e. a solution with a maximum light transmittance value, at a wavelength equal to 500 nm, through a sample 1 cm thick, of at least 60% and preferably of at least 70%.

The *"homopolymers "* according to the invention are preferably crosslinked and neutralized, and they may be obtained according to the preparation process comprising the following steps:

(a) the monomer such as AMPS in free form is dispersed or dissolved in a solution of tert-butanol or of water and tert-butanol;

(b) the monomer solution or dispersion obtained in (a) is neutralized with one or more mineral or organic bases, preferably aqueous ammonia $NH_3$, in an amount making it possible to obtain a degree of neutralization of the sulfonic acid functions of the polymer ranging from 90% to 100%;

(c) the crosslinking monomer(s) are added to the solution or dispersion obtained in (b);

(d) a standard free-radical polymerization is performed in the presence of free-radical initiators at a temperature ranging from 10 to 150°C; the polymer precipitates in the tert-butanol-based solution or dispersion.

The water-soluble or water-dispersible AMPS® copolymers according to the invention contain water-soluble ethylenically unsaturated monomers, hydrophobic monomers, or mixtures thereof.

The water-soluble comonomers may be ionic or nonionic.

Among the ionic water-soluble comonomers, examples that may be mentioned include the following compounds, and salts thereof:

- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- water-soluble vinyl monomers of formula (A) below:

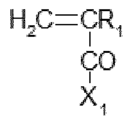

(A)

in which:

- $R_1$ is chosen from H, $-CH_3$, $-C_2H_5$ and $-C_3H_7$,
- $X_1$ is chosen from:
- alkyl oxides of type $-OR_2$ where $R_2$ is a linear or branched, saturated or unsaturated hydrocarbon-based radical containing from 1 to 6 carbon atoms, substituted with at least one sulfonic ($-SO_3^-$) and/or sulfate ($-SO_4^-$) and/or phosphate ($-PO_4H_2$-) group.

Among the nonionic water-soluble comonomers, examples that may be mentioned include:

- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula $CH_2$=CHOH,
- water-soluble vinyl monomers of formula (B) below:

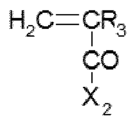

(B)

in which:

- $R_3$ is chosen from H, $-CH_3$, $-C_2H_5$ and $-C_3H_7$,
- $X_2$ is chosen from alkyl oxides of the type $-OR_4$ where $R_4$ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

Mention is made, for example, of glycidyl (meth)acrylate, hydroxyethyl methacrylate, and (meth)acrylates of ethylene glycol, of diethylene glycol or of polyalkylene glycol.

Among the hydrophobic co-monomers without a fatty chain, mention may be made, for example, of:

- styrene and derivatives thereof, such as 4-butylstyrene, $\alpha$-methylstyrene and vinyltoluene;
- vinyl acetate of formula $CH_2$=CH-$OCOCH_3$;
- vinyl ethers of formula $CH_2$=CHOR in which R is a linear or branched, saturated or unsaturated hydrocarbon-based radical containing from 1 to 6 carbons;
- acrylonitrile;
- caprolactone;
- vinyl chloride and vinylidene chloride;
- silicone derivatives, which, after polymerization, result in silicone polymers such as methacryloxypropyltris(trimethylsiloxy)silane and silicone methacrylamides;
- hydrophobic vinyl monomers of formula (C) below:

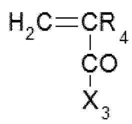

(C)

in which:

- $R_4$ is chosen from H, $-CH_3$, $-C_2H_5$ and $-C_3H_7$;
- $X_3$ is chosen from:
- alkyl oxides of the type $-OR_5$ where $R_5$ is a linear or branched, saturated or unsaturated hydrocarbon-based radical containing from 1 to 6 carbon atoms.

Mention is made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate.

The water-soluble or water-dispersible AMPS® polymers of the invention preferably have a molar mass ranging from 50 000 to 10 000 000 g/mol, preferably from 80 000 to 8 000 000 g/mol, and even more preferably from 100 000 to 7 000 000 g/mol.

As water-soluble or water-dispersible AMPS@ homopolymers suitable for use in the invention, mention may be made, for example, of crosslinked or non-crosslinked polymers of sodium acrylamido-2-methylpropanesulfonate, such as that used in the commercial product Simulgel 800 (CTFA name: Sodium Polyacryloyldimethyl Taurate), crosslinked ammonium acrylamido-2-methylpropanesulfonate polymers (INCI name: Ammonium Polyacryldimethyltauramide) such as those described in patent EP 0 815 928 B1 and such as the product sold under the trade name Hostacerin AMPS® by the company Clariant.

As water-soluble or water-dispersible AMPS copolymers in accordance with the invention, examples that may be mentioned include:

- crosslinked acrylamide/sodium acrylamido-2-methylpropanesulfonate copolymers, such as that used in the commercial product Sepigel 305® (CTFA name: Polyacrylamide/$C_{13}$-$C_{14}$ Isoparaffin/ Laureth-7) or that used in the commercial product sold under the name Simulgel 600® (CTFA name: Acrylamide/Sodium acryloyldimethyltaurate/Isohexadecane/Polysorbate-80) by the company SEPPIC;
- copolymers of AMPS® and of vinylpyrrolidone or vinylformamide, such as that used in the commercial product sold under the name Aristoflex AVC® by the company Clariant (CTFA name: Ammonium Acryloyldimethyltaurate/VP copolymer) but neutralized with sodium hydroxide or potassium hydroxide;
- copolymers of AMPS® and of sodium acrylate, for instance the AMPS®/sodium acrylate copolymer, such as that used in the commercial product sold under the name Simulgel EG® by the company SEPPIC or under the trade name Sepinov EM® (CTFA name: Hydroxyethyl acrylate/Sodium acryloyldimethyltaurate copolymer);
- copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/sodium acryloyldimethyltaurate copolymer (and) squalane (and) polysorbate 60), or such as the product sold under the name Sodium acrylamido-2-methylpropanesulfonate/hydroxyethyl acrylate copolymer, such as the commercial product Sepinov EMT 10® (INCI name: Hydroxyethyl acrylate/Sodium acryloyldimethyltaurate copolymer).

As preferred water-soluble or water-dispersible AMPS® copolymers in accordance with the invention, mention may be made of copolymers of AMPS® and of hydroxyethyl acrylate.

In general, an aqueous phase according to the invention may comprise from 0.1% to 8% by weight, preferably from 0.2% to 5% by weight and more preferentially from 0.7% to 5% by weight of solids of crosslinked and/or neutralized 2-acrylamido-2-methylpropanesulfonic acid polymer(s) and copolymer(s) relative to its total weight.

## LIPOPHILIC GELLING AGENT

For the purposes of the present invention, the term *"lipophilic gelling agent"* means a compound that is capable of gelling the oily phase of the compositions according to the invention.

The gelling agent is lipophilic and is thus present in the oily phase of the composition.

The gelling agent is liposoluble or lipodispersible.

As emerges from the text hereinbelow, the lipophilic gelling agent is advantageously chosen organopolysiloxane elastomers.

### Organopolysiloxane elastomer

The organopolysiloxane elastomer which can be used as lipophilic gelling agent has the advantage of conferring good application properties on the composition according to the invention. It affords a very soft and mattifying feel after application, which is advantageous in particular for application to the skin. It may also allow efficient filling of the hollows

present on keratin materials.

The term *"organopolysiloxane elastomer"* or *"silicone elastomer"* means a supple, deformable organopolysiloxane with viscoelastic properties and especially with the consistency of a sponge or a supple sphere. Its modulus of elasticity is such that this material withstands deformation and has a limited ability to extend and to contract. This material is capable of regaining its original shape after stretching.

It is more particularly a crosslinked organopolysiloxane elastomer.

Thus, the organopolysiloxane elastomer can be obtained by a crosslinking addition reaction of diorganopolysiloxane containing at least one hydrogen bonded to silicon and of diorganopolysiloxane having ethylenically unsaturated groups bonded to silicon, in particular in the presence of a platinum catalyst; or by a dehydrogenation crosslinking condensation reaction between a diorganopolysiloxane comprising hydroxyl end groups and a diorganopolysiloxane containing at least one hydrogen bonded to silicon, in particular in the presence of an organotin compound; or by a crosslinking condensation reaction of a diorganopolysiloxane comprising hydroxyl end groups and of a hydrolysable organopolysilane; or by thermal crosslinking of organopolysiloxane, in particular in the presence of an organic peroxide catalyst; or by crosslinking of organopolysiloxane via high-energy radiation, such as gamma rays, ultraviolet rays or an electron beam.

Preferably, the organopolysiloxane elastomer is obtained by crosslinking addition reaction (A) of diorganopolysiloxane containing at least two hydrogens each bonded to a silicon, and (B) of diorganopolysiloxane containing at least two ethylenically unsaturated groups bonded to silicon, especially in the presence (C) of a platinum catalyst, as described, for instance, in patent application EP-A-295 886.

In particular, the organopolysiloxane elastomer may be obtained by reaction of dimethylpolysiloxane bearing dimethylvinylsiloxy end groups and of methylhydrogenopolysiloxane bearing trimethylsiloxy end groups, in the presence of a platinum catalyst.

Compound (A) is the base reactant for the formation of elastomeric organopolysiloxane, and the crosslinking takes place via an addition reaction of compound (A) with compound (B) in the presence of the catalyst (C).

Compound (A) is in particular an organopolysiloxane containing at least two hydrogen atoms bonded to different silicon atoms in each molecule.

Compound (A) may have any molecular structure, especially a linear-chain or branched-chain structure or a cyclic structure.

Compound (A) may have a viscosity at 25°C ranging from 1 to 50 000 centistokes, especially so as to be readily miscible with compound (B).

The organic groups bonded to the silicon atoms of compound (A) may be alkyl groups such as methyl, ethyl, propyl, butyl, octyl; substituted alkyl groups such as 2-phenylethyl, 2-phenylpropyl or 3,3,3-trifluoropropyl; aryl groups such as phenyl, tolyl, xylyl; substituted aryl groups such as phenylethyl; and substituted monovalent hydrocarbon-based groups such as an epoxy group, a carboxylate ester group or a mercapto group.

Compound (A) may thus be chosen from trimethylsiloxy-terminated methylhydrogenopolysiloxanes, trimethylsiloxy-terminated dimethylsiloxane/methylhydrogenosiloxane copolymers, and dimethylsiloxane/methylhydrogenosiloxane cyclic copolymers.

Compound (B) is advantageously a diorganopolysiloxane containing at least two lower alkenyl groups (for example $C_2$-$C_4$); the lower alkenyl group may be chosen from vinyl, allyl and propenyl groups. These lower alkenyl groups may be located in any position on the organopolysiloxane molecule, but are preferably located at the ends of the organopolysiloxane molecule. The organopolysiloxane (B) may have a branched-chain, linear-chain, cyclic or network structure, but the linear-chain structure is preferred. Compound (B) may have a viscosity ranging from the liquid state to the gum state. Preferably, compound (B) has a viscosity of at least 100 centistokes at 25°C.

Besides the abovementioned alkenyl groups, the other organic groups bonded to the silicon atoms in compound (B) may be alkyl groups such as methyl, ethyl, propyl, butyl or octyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon-based groups such as an epoxy group, a carboxylate ester group or a mercapto group.

The organopolysiloxanes (B) can be chosen from methylvinylpolysiloxanes, methylvinylsiloxane-dimethylsiloxane copolymers, dimethylpolysiloxanes comprising dimethylvinylsiloxy end groups, dimethylsiloxane-methylphenylsiloxane copolymers comprising dimethylvinylsiloxy end groups, dimethylsiloxane-diphenylsiloxane-methylvinylsiloxane copolymers comprising dimethylvinylsiloxy end groups, dimethylsiloxane-methylvinylsiloxane copolymers comprising trimethylsiloxy end groups, dimethylsiloxane-methylphenylsiloxane-methylvinylsiloxane copolymers comprising trimethylsiloxy end groups, methyl(3,3,3-trifluoropropyl)polysiloxanes comprising dimethylvinylsiloxy end groups, and dimethylsiloxane-methyl(3,3,3-trifluoropropyl)siloxane copolymers comprising dimethylvinylsiloxy end groups.

In particular, the organopolysiloxane elastomer can be obtained by reaction of dimethylpolysiloxane comprising dimethylvinylsiloxy end groups and of methylhydropolysiloxane comprising trimethylsiloxy end groups, in the presence of a platinum catalyst.

Advantageously, the sum of the number of ethylenic groups per molecule in compound (B) and of the number of hydrogen atoms bonded to silicon atoms per molecule in compound (A) is at least 5.

It is advantageous for compound (A) to be added in an amount such that the molecular ratio between the total amount of hydrogen atoms bonded to silicon atoms in compound (A) and the total amount of all the ethylenically unsaturated groups in compound (B) is within the range from 1.5/1 to 20/1.

Compound (C) is the catalyst for the crosslinking reaction, and is especially chloroplatinic acid, chloroplatinic acid-olefin complexes, chloroplatinic acid-alkenylsiloxane complexes, chloroplatinic acid-diketone complexes, platinum black and platinum on a support.

Catalyst (C) is preferably added in an amount of from 0.1 to 1000 parts by weight and better still from 1 to 100 parts by weight, as clean platinum metal, per 1000 parts by weight of the total amount of compounds (A) and (B).

The elastomer is advantageously a non-emulsifying elastomer.

The term *"non-emulsifying"* defines organopolysiloxane elastomers not containing a hydrophilic chain and in particular not containing polyoxyalkylene units (especially polyoxyethylene or polyoxypropylene units) or a polyglyceryl unit. Thus, according to a specific form of the invention, the composition comprises an organopolysiloxane elastomer devoid of polyoxyalkylene units and of polyglyceryl unit.

In particular, the silicone elastomer used in the present invention is chosen from Dimethicone Crosspolymer (INCI name), Vinyl Dimethicone Crosspolymer (INCI name), Dimethicone/Vinyl Dimethicone Crosspolymer (INCI name) or Dimethicone Crosspolymer-3 (INCI name).

The organopolysiloxane elastomer particles may be conveyed in the form of a gel formed from an elastomeric organopolysiloxane included in at least one hydrocarbon-based oil and/or one silicone oil. In these gels, the organopolysiloxane particles are often non-spherical particles.

Non-emulsifying elastomers are especially described in patents EP 242 219, EP 285 886 and EP 765 656 and in patent application JP-A-61-194 009.

The silicone elastomer is generally provided in the form of a gel, a paste or a powder but advantageously in the form of a gel in which the silicone elastomer is dispersed in a linear silicone oil (dimethicone) or cyclic silicone oil (e.g.: cyclopentasiloxane), advantageously in a linear silicone oil.

Non-emulsifying elastomers that may more particularly be used include those sold under the names KSG-6®, KSG-15®, KSG-16®, KSG-18®, KSG-41®, KSG-42®, KSG-43® and KSG-44® by the company Shin-Etsu, DC9040® and DC9041® by Dow Corning and SFE 839® by the company General Electric.

According to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone oil chosen from polydimethylsiloxanes (PDMS) or dimethicones with a viscosity at 25°C ranging from 1 to 500 cSt, optionally modified with optionally fluorinated aliphatic groups, or with functional groups such as hydroxyl, thiol and/or amine groups.

Mention may be made especially of the compounds having the following INCI names:

- dimethicone/vinyl dimethicone crosspolymer, such as USG-105® and USG-107A® from the company Shin-Etsu; DC9506® and DC9701® from the company Dow Corning;
- dimethicone/vinyl dimethicone crosspolymer (and) dimethicone, such as KSG-6® and KSG-16® from the company Shin-Etsu;
- dimethicone/vinyl dimethicone crosspolymer (and) cyclopentasiloxane, such as KSG-15®;
- cyclopentasiloxane (and) dimethicone crosspolymer, such as DC9040®, DC9045® and DC5930® from the company Dow Corning;
- dimethicone (and) dimethicone crosspolymer, such as DC9041® from the company Dow Corning.
- dimethicone (and) dimethicone crosspolymer, such as Dow Corning EL-9240® Silicone Elastomer Blend from the company Dow Corning (mixture of polydimethylsiloxane crosslinked with hexadiene/polydimethylsiloxane (2 cSt));
- $C_{4-24}$ alkyl dimethicone/divinyl dimethicone crosspolymer, such as NuLastic Silk MA® from the company Alzo.

Mention may in particular be made, as examples of silicone elastomers dispersed in a linear silicone oil which can advantageously be used according to the invention, of the following references:

- dimethicone/vinyl dimethicone crosspolymer (and) dimethicone, such as KSG-6® and KSG-16® from the company Shin-Etsu;
- dimethicone (and) dimethicone crosspolymer, such as DC9041®, Dow Corning EL-9240® Silicone Elastomer Blend from the company Dow Corning.

The organopolysiloxane elastomer particles may also be used in powder form: mention may be made especially of the powders sold under the names Dow Corning 9505 Powder® and Dow Corning 9506 Powder® by the company

Dow Corning, these powders having the INCI name: dimethicone/vinyl dimethicone crosspolymer.

The organopolysiloxane powder may also be coated with silsesquioxane resin, as described, for example, in patent US 5 538 793. Such elastomeric powders are sold under the names KSP-100®, KSP-101®, KSP-102®, KSP-103®, KSP-104® and KSP-105® by the company Shin-Etsu, and have the INCI name: vinyl dimethicone/methicone silsesquioxane crosspolymer.

As examples of organopolysiloxane powders coated with silsesquioxane resin that may advantageously be used according to the invention, mention may be made especially of the reference KSP-100® from the company Shin-Etsu.

As preferred lipophilic gelling agent of organopolysiloxane elastomer type, mention may be made especially of crosslinked organopolysiloxane elastomers chosen from Dimethicone Crosspolymer (INCI name), Dimethicone (and) Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name), and in particular Dimethicone Crosspolymer (INCI name).

The organopolysiloxane elastomer may be present in a composition of the present invention in a content ranging from 0.1% to 35% by weight of solids, especially from 1% to 20% and more particularly from 2% to 10% by weight relative to the total weight of the composition.

## HYDROPHILIC GELLING AGENT/LIPOPHILIC GELLING AGENT SYSTEM

As preferred synthetic polymeric hydrophilic gelling agents, mention may be made more particularly of 2-acrylamido-2-methylpropanesulfonic acid polymers, for instance AMPS@, such as the ammonium 2-acrylamido-2-methylpropanesulfonate polymer sold under the trade name Hostacerin AMPS® by the company Clariant, and 2-acrylamido-2-methyl propanesulfonic acid copolymers and in particular copolymers of AMPS® and of hydroxyethyl acrylate, for instance the AMPS®/hydroxyethyl acrylate copolymer such as that used in the commercial product sold under the name Simulgel NS® by the company SEPPIC (CTFA name: Hydroxyethyl acrylate/sodium acryloyldimethyltaurate copolymer (and) squalane (and) polysorbate 60), or such as the product sold under the name Sodium acrylamido-2-methylpropanesulfonate/hydroxyethyl acrylate copolymer, such as the commercial product Sepinov EMT 10® (INCI name: Hydroxyethyl acrylate/Sodium acryloyldimethyltaurate copolymer).

As preferred lipophilic gelling agents, mention may be made of organopolysiloxane elastomers preferably chosen from Dimethicone Crosspolymer (INCI name), Dimethicone (and) Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name), and in particular Dimethicone Crosspolymer (INCI name) and Dimethicone (and) Dimethicone Crosspolymer (INCI name).

According to a preferred mode, as preferred lipophilic gelling agents, mention may be made more particularly of gels of silicone elastomer dispersed in a silicone oil and/or powders of organopolysiloxane elastomer coated with silsesquioxane resin.

Thus, according to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone oil chosen from polydimethylsiloxanes (PDMS) or dimethicones with a viscosity at 25°C ranging from 1 to 500 cSt at 25°C, especially the following references:

- dimethicone/vinyl dimethicone crosspolymer (and) dimethicone, such as KSG-6® and KSG-16® from the company Shin-Etsu;
- dimethicone (and) dimethicone crosspolymer, such as DC9041® from the company Dow Corning; and
- dimethicone (and) dimethicone crosspolymer, such as Dow Corning EL-9240® Silicone Elastomer Blend from the company Dow Corning.

As non-limiting illustrations of hydrophilic gelling agent/lipophilic gelling agent systems that are most particularly suitable for use in the invention, mention may be made especially of the polymer or copolymer system of 2-acrylamido-2-methylpropanesulfonic acid/organopolysiloxane elastomer.

Thus, a composition according to the invention may advantageously comprise as hydrophilic gelling agent/lipophilic gelling agent system, a polymer system of 2-acrylamido-2-methylpropanesulfonic acid/organopolysiloxane elastomer(s) or copolymer of 2-acrylamido-2-methylpropanesulfonic acid and of hydroxyethyl acrylate/organopolysiloxane elastomer(s).

Preferably, a composition according to the invention may comprise as hydrophilic gelling agent/lipophilic gelling agent system, a copolymer system of 2-acrylamido-2-methylpropanesulfonic acid and of hydroxyethyl acrylate/organopolysiloxane elastomer powder.

## AQUEOUS PHASE

The aqueous phase of a composition according to the invention comprises water and optionally a water-soluble solvent.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms such as ethanol and isopropanol, glycols containing from 2 to 8 carbon atoms such as ethylene glycol, propylene glycol, 1,3-butylene glycol and dipropylene glycol, $C_3$ and $C_4$ ketones and $C_2$-$C_4$ aldehydes.

The aqueous phase (water and optionally the water-miscible solvent) may be present in the composition in a content ranging from 5% to 95%, better still from 30% to 80% by weight and preferably from 40% to 75% by weight relative to the total weight of said composition.

According to another embodiment variant, the aqueous phase of a composition according to the invention may comprise at least one $C_2$-$C_{32}$ polyol.

For the purposes of the present invention, the term "*polyol*" should be understood as meaning any organic molecule comprising at least two free hydroxyl groups.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are advantageously suitable for formulating a composition according to the present invention are those especially containing from 2 to 32 carbon atoms and preferably 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols, such as glycerol oligomers, for instance diglycerol, and polyethylene glycols, and mixtures thereof.

According to a preferred embodiment of the invention, said polyol is chosen from ethylene glycol, pentaerythritol, trimethylolpropane, propylene glycol, glycerol, polyglycerols, polyethylene glycols and mixtures thereof.

According to a particular mode, the composition of the invention may comprise at least propylene glycol.

According to another particular mode, the composition of the invention may comprise at least glycerol.

## OILY PHASE

For the purposes of the invention, an oily phase comprises at least one oil.

The term "oil" means any fatty substance that is in liquid form at room temperature (25°C) and atmospheric pressure (760 mmHg).

An oily phase that is suitable for preparing the compositions, especially cosmetic compositions, according to the invention may comprise hydrocarbon-based oils, silicone oils, fluoro oils or non-fluoro oils, or mixtures thereof.

The oils may be volatile or non-volatile.

They may be of animal, plant, mineral or synthetic origin. According to one embodiment variant, oils of silicone origin are preferred.

For the purposes of the present invention, the term "non-volatile oil" means an oil with a vapour pressure of less than 0.13 Pa.

For the purposes of the present invention, the term "silicone oil" means an oil comprising at least one silicon atom, and especially at least one Si-O group.

The term "fluoro oil" means an oil comprising at least one fluorine atom.

The term "hydrocarbon-based oil" means an oil mainly containing hydrogen and carbon atoms.

The oils may optionally comprise oxygen, nitrogen, sulfur and/or phosphorus atoms, for example in the form of hydroxyl or acid radicals.

For the purposes of the invention, the term "volatile oil" means any oil that is capable of evaporating on contact with the skin in less than one hour, at room temperature and atmospheric pressure.

The volatile oil is a volatile cosmetic compound, which is liquid at room temperature, especially having a non-zero vapour pressure, at room temperature and atmospheric pressure, especially having a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

Volatile oils

The volatile oils may be hydrocarbon-based oils or silicone oils.

Among the volatile hydrocarbon-based oils containing from 8 to 16 carbon atoms, mention may be made especially of branched C8-C16 alkanes, for instance C8-C16 isoalkanes (also known as isoparaffins), isododecane, isodecane, isohexadecane and, for example, the oils sold under the trade names Isopar or Permethyl, branched C8-C16 esters, for instance isohexyl neopentanoate, and mixtures thereof. Preferably, the volatile hydrocarbon-based oil is chosen from volatile hydrocarbon-based oils containing from 8 to 16 carbon atoms, and mixtures thereof, in particular from isododecane, isodecane and isohexadecane, and is in particular isohexadecane.

Mention may also be made of volatile linear alkanes comprising from 8 to 16 carbon atoms, in particular from 10 to 15 carbon atoms and more particularly from 11 to 13 carbon atoms, for instance n-dodecane (C12) and n-tetradecane (C14) sold by Sasol under the respective references Parafol 12-97® and Parafol 14-97®, and also mixtures thereof, the undecane-tridecane mixture, mixtures of n-undecane (C11) and of n-tridecane (C13) obtained in Examples 1 and 2 of patent application WO 2008/155 059 from the company Cognis, and mixtures thereof.

Volatile silicone oils that may be mentioned include linear volatile silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane and dodecamethylcyclohexasiloxane.

Non-volatile oils

The non-volatile oils may, in particular, be selected from non-volatile hydrocarbon-based, fluoro and/or silicone oils.

Non-volatile hydrocarbon-based oils that may especially be mentioned include:

- hydrocarbon-based oils of animal origin,
- hydrocarbon-based oils of plant origin, synthetic ethers containing from 10 to 40 carbon atoms, such as dicaprylyl ether,
- synthetic esters, for instance the oils of formula $R_1COOR_2$, in which R1 represents a linear or branched fatty acid residue containing from 1 to 40 carbon atoms and R2 represents a hydrocarbon-based chain, which is especially branched, containing from 1 to 40 carbon atoms, on condition that $R1 + R2 \geq 10$. The esters may be chosen especially from alcohol fatty acid esters, for instance cetostearyl octanoate, isopropyl alcohol esters, such as isopropyl myristate, isopropyl palmitate, ethyl palmitate, 2-ethylhexyl palmitate, isopropyl stearate, octyl stearate, hydroxylated esters, for instance isostearyl lactate, octyl hydroxystearate, alcohol or polyalcohol ricinoleates, hexyl laurate, neopentanoic acid esters, for instance isodecyl neopentanoate, isotridecyl neopentanoate, and isononanoic acid esters, for instance isononyl isononanoate and isotridecyl isononanoate,
- polyol esters and pentaerythritol esters, for instance dipentaerythritol tetrahydroxystearate/tetraisostearate,
- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- C12-C22 higher fatty acids, such as oleic acid, linoleic acid and linolenic acid, and mixtures thereof,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, and trimethyl-pentaphenyl-trisiloxane, and mixtures thereof; and also mixtures of these various oils.

Preferably, a composition according to the invention comprises volatile and/or non-volatile silicone oils. Such silicone oils are particularly appreciated when the lipophilic gelling agent is an organopolysiloxane elastomer.

A composition according to the invention may comprise from 5% to 95% by weight, better still from 5% to 40% by weight and preferably from 7% to 35% by weight of oil(s) relative to the total weight of said composition.

As mentioned above, the gelled oily phase according to the invention may have a threshold stress of greater than 1.5 Pa and preferably greater than 10 Pa. This threshold stress value reflects a gel-type texture of this oily phase.

## POLAR OILS

According to a particular form of the invention, when the composition comprises at least one glycol compound of formula (2), the gelled oily phase comprises at least one polar oil.

The term "polar oil" means any oil which has solubility parameters in the Hansen solubility space such that 13 < $\delta_d$ < 22.

The definition of the solubility parameters in the Hansen three-dimensional solubility space are described in the article by C.M. Hansen: The three-dimensional solubility parameters, J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a=(\delta_p{}^2 + \delta_h{}^2)^{½}$.

The parameters $\delta_p$, $\delta_h$, $\delta_D$ and $\delta_a$ are expressed in $(J/cm^3)^{½}$.

The solubility parameters are calculated with the HSPiP v4.1 software.

More preferentially, the polar oil(s) in accordance with the invention will also be characterized by 1 < $\delta_a$ < 10.

The polar oil(s) in accordance with the invention may be of plant, mineral or synthetic origin.

They may be chosen from hydrocarbon-based oils and silicone oils, and mixtures thereof.

A) Polar hydrocarbon-based oils

Among the hydrocarbon-based polar oils that may be used in the oily phase of the compositions of the invention, mention may be made of:

- phytostearyl esters, such as phytostearyl oleate, phytostearyl isostearate and lauroyl/octyldodecyl/phytostearyl glutamate (Ajinomoto, Eldew PS203®),
- triglycerides constituted of fatty acid esters of glycerol, in particular the fatty acids of which may have chain lengths ranging from C4 to C36, and in particular from C18 to C36, these oils possibly being linear or branched, and saturated or unsaturated; these oils may in particular be heptanoic or octanoic triglycerides, wheatgerm oil, sunflower oil, grapeseed oil, sesame seed oil (820.6 g/mol), corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil or musk rose oil; shea oil; or alternatively caprylic/capric acid triglycerides, for instance those sold by the company Stearineries Dubois or those sold under the names Miglyol 810®, 812® and 818® by the company Dynamit Nobel;
- synthetic ethers containing from 10 to 40 carbon atoms, such as dicaprylyl ether,
- hydrocarbon-based esters of formula RCOOR' in which RCOO represents a carboxylic acid residue comprising from 2 to 40 carbon atoms, and R' represents a hydrocarbon-based chain containing from 1 to 40 carbon atoms, such as cetostearyl octanoate, isopropyl alcohol esters, such as isopropyl myristate or isopropyl palmitate, ethyl palmitate, 2-ethylhexyl palmitate, isopropyl stearate or isostearate, isostearyl isostearate, octyl stearate, diisopropyl adipate, heptanoates, and in particular isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethyl hexanoate, and mixtures thereof, C12 to C15 alcohol benzoates, hexyl laurate, neopentanoic acid esters, for instance isodecyl neopentanoate, isotridecyl neopentanoate, isostearyl neopentanoate and 2-octyldodecyl neopentanoate, isononanoic acid esters, for instance isononyl isononanoate, isotridecyl isononanoate and octyl isononanoate, oleyl erucate, isopropyl lauroyl sarcosinate, diisopropyl sebacate, isocetyl stearate, isodecyl neopentanoate, isostearyl behenate, and myristyl myristate;
- polyesters obtained by condensation of an unsaturated fatty acid dimer and/or trimer and of diol, such as those described in patent application FR 0 853 634, in particular such as dilinoleic acid and 1,4-butanediol. Mention may in particular be made in this respect of the polymer sold by Biosynthis under the name Viscoplast 14436H® (INCI name: Dilinoleic Acid/Butanediol Copolymer), or copolymers of polyols and of diacid dimers, and esters thereof, such as Hailuscent ISDA®,
- polyol esters and pentaerythritol esters, for instance dipentaerythritol tetrahydroxystearate/tetraisostearate,
- fatty alcohols containing from 12 to 26 carbon atoms, for instance octyldodecanol, 2-butyloctanol, 2-hexyldecanol, 2-undecylpentadecanol and oleyl alcohol,
- dialkyl carbonates, the two alkyl chains possibly being identical or different, such as dicaprylyl carbonate sold under the name Cetiol CC® by Cognis; and

- vinylpyrrolidone copolymers such as the vinylpyrrolidone/1-hexadecene copolymer, Antaron V-216® sold or manufactured by the company ISP,
- linear fatty acid esters with a total carbon number ranging from 35 to 70, such as pentaerythrityl tetrapelargonate,
- hydroxylated esters such as polyglyceryl-2 triisostearate,
- aromatic esters such as tridecyl trimellitate, $C_{12}$-$C_{15}$ alcohol benzoate, the 2-phenylethyl ester of benzoic acid, and butyloctyl salicylate,
- $C_{24}$-$C_{28}$ esters of branched fatty alcohols or fatty acids such as those described in patent application EP-A-0 955 039, and in particular triisoarachidyl citrate, pentaerythrityl tetraisononanoate, glyceryl triisostearate, glyceryl tris(2-decyl)tetradecanoate, pentaerythrityl tetraisostearate, polyglyceryl-2 tetraisostearate or pentaerythrityl tetrakis(2-decyl)tetradecanoate,
- esters and polyesters of dimer diol and of monocarboxylic or dicarboxylic acid, such as esters of dimer diol and of fatty acid and esters of dimer diol and of dimer dicarboxylic acid, such as Lusplan DD-DA5® and Lusplan DD-DA7® sold by the company Nippon Fine Chemical and described in patent application US 2004-175 338, the content of which is incorporated into the present application by reference,
- and mixtures thereof.

According to one particular mode, the non-volatile hydrocarbon-based oil may be chosen from liquid lipophilic organic UV-screening agents.

The term "liquid lipophilic organic screening agent" means any organic chemical molecule that is capable of absorbing at least UV radiation in the wavelength range between 280 and 400 nm, said molecule being in liquid form at room temperature (20 - 25°C) and at atmospheric pressure (760 mmHg) and capable of being miscible in an oily phase.

The liquid organic UV-screening agents that can be used according to the invention may be chosen from

- liquid lipophilic β,β-diphenylacrylate compounds,
- liquid lipophilic salicylate compounds,
- liquid lipophilic cinnamate compounds,
- and mixtures thereof.

i) β,β-Diphenylacrylate compounds

Among the liquid lipophilic organic UVB-screening agents that can be used according to the invention, mention may be made of the liquid lipophilic alkyl β,β-diphenylacrylate or α-cyano-β,β-diphenylacrylate compounds of formula (I) below:

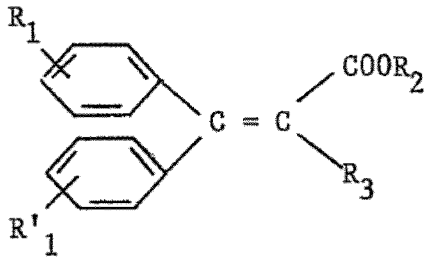

(I)

where $R_1$ to $R_3$ can have the following meanings:

- $R_1$ and $R'_1$, which may be identical or different, represent a hydrogen atom, a straight-chain or branched-chain $C_1$-$C_8$ alkoxy radical or a straight-chain or branched-chain $C_1$-$C_4$ alkyl radical;
- $R_1$ and $R'_1$ being in the para meta position;
- $R_2$ represents a straight-chain or branched-chain $C_1$-$C_{12}$ alkyl radical;
- $R_3$ represents a hydrogen atom or the CN radical.

Among the straight-chain or branched-chain $C_1$-$C_8$ alkoxy radicals, mention may for example be made of methoxy, ethoxy, n-propoxy, isopropoxy, n-butoxy, isobutoxy, tert-butoxy, n-amyloxy, isoamyloxy, neopentyloxy, n-hexyloxy, n-heptyloxy, n-octyloxy and 2-ethylhexyloxy radicals.

Among the straight-chain or branched-chain $C_1$-$C_4$ alkyl radicals, mention may more particularly be made of methyl, ethyl, n-propyl, isopropyl, n-butyl, isobutyl and tert-butyl radicals. For the $C_1$-$C_{12}$ alkyl radicals, mention may be made, by way of example, in addition to those mentioned above, of n-amyl, isoamyl, neopentyl, n-hexyl, n-heptyl, n-octyl, 2-ethylhexyl, decyl and lauryl radicals.

Among the compounds of general formula (I), the following compounds are more particularly preferred:

- 2-ethylhexyl α-cyano-β,β-diphenylacrylate or Octocrylene, sold in particular under the trade name UVINUL N539® by BASF;
- ethyl α-cyano-β,β-diphenylacrylate such as Etocrylene, sold in particular under the trade name UVINUL N35® by BASF;
- 2-ethylhexyl β,β-diphenylacrylate;
- ethyl β,β-di(4'-methoxyphenyl)acrylate.

Among the compounds of general formula (I), the compound 2-ethylhexyl 2-cyano-3,3-diphenylacrylate or Octocrylne is even more particularly preferred.

ii) Salicylate compounds

Among the liquid lipophilic salicylate compounds that can be used according to the invention, mention may be made of:

- Homosalate, sold under the name Eusolex HMS® by Rona/EM Industries,
- Ethylhexyl salicylate, sold under the name Neo Heliopan OS® by Symrise.

iii) Cinnamate compounds

Among the liquid lipophilic cinnamate compounds that can be used according to the invention, mention may be made of:

- Ethylhexyl Methoxycinnamate, sold in particular under the trade name Parsol MCX® by DSM Nutritional Products,
- Isopropyl Methoxycinnamate,
- Isoamyl Methoxycinnamate, sold under the trade name Neo Heliopan E 1000 by Symrise.

Among the liquid lipophilic screening agents according to the invention, use will more particularly be made of the compound Ethylhexyl Methoxycinnamate.

Preferentially, the polar hydrocarbon-based oil will be Ethylhexyl Methoxycinnamate.

B) Polar silicone oils

The polar silicone oils that may be used in the oily phase of the compositions of the invention may be chosen from certain non-phenyl silicone oils, phenyl silicone oils, and mixtures thereof.

The term "phenyl silicone" (also referred to as phenyl silicone oil) is understood to mean an organopolysiloxane substituted with at least one phenyl group.

a) Non-phenyl silicone oils

Among the polar non-phenyl silicone oils that may be used in the oily phase of the compositions of the invention, mention may be made of:

- volatile cyclic silicone oils having a viscosity at room temperature of less than 8 cSt and containing in particular from 4 to 7 silicone atoms, these silicones optionally comprising alkyl or alkoxy groups containing from 1 to 10 carbon atoms, such as hexamethylcyclotrisiloxane, octamethylcyclotetrasiloxane, decamethylcyclopentasiloxane and dodecamethylcyclohexasiloxane (cyclohexasiloxane);
- polydimethylsiloxanes comprising aliphatic groups, in particular alkyl groups, or alkoxy groups, which are pendent and/or at the end of the silicone chain; these groups each comprising from 6 to 24 carbon atoms, for instance caprylyl methicone, such as the commercial product Dow Corning FZ-3196® from the company Dow Corning.

b) Phenyl silicone oils

The phenyl silicone oil may be chosen from phenyl trimethicones, phenyl dimethicones, phenyltrimethylsiloxydiphenylsiloxanes, diphenyl dimethicones, diphenylmethyldiphenyltrisiloxanes, trimethylpentaphenyltrisiloxane and 2-phenylethyl trimethylsiloxysilicates.

The silicone oil can correspond to the formula:

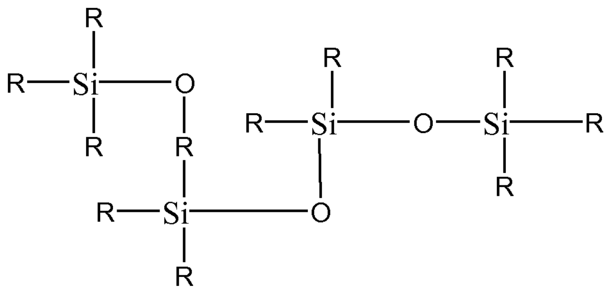

in which the R groups represent, independently of each other, a methyl or a phenyl. Preferably, in this formula, the silicone oil comprises at least three phenyl groups, for example at least four, at least five or at least six.

According to another embodiment, the silicone oil corresponds to the formula:

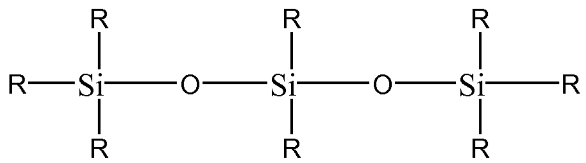

in which the R groups represent, independently of each other, a methyl or a phenyl. Preferably, in this formula, said organopolysiloxane comprises at least three phenyl groups, for example at least four or at least five.

Mixtures of the phenyl organopolysiloxanes described above can be used.

Mention may be made, for example, of mixtures of triphenylated, tetraphenylated or pentaphenylated organopolysiloxane.

According to another embodiment, the silicone oil corresponds to the formula:

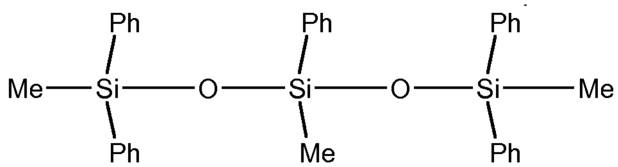

in which Me represents methyl and Ph represents phenyl. Such a phenyl silicone is especially manufactured by Dow Corning under the reference Dow Corning 555 Cosmetic Fluid® (INCI name: trimethyl pentaphenyl trisiloxane). The reference Dow Corning 554 Cosmetic Fluid® may also be used.

According to another embodiment, the silicone oil corresponds to the formula:

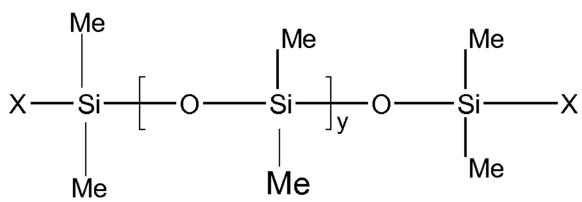

in which Me represents methyl, y is between 1 and 1000 and X represents $-CH_2-CH(CH_3)(Ph)$.

According to another embodiment, the silicone oil corresponds to the formula:

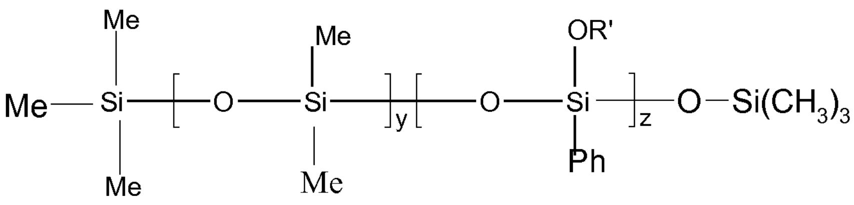

in which -OR' represents -O-SiMe$_3$, y is between 1 and 1000 and z is between 1 and 1000.

The phenyl silicone oil can be chosen from the phenyl silicones of following formula (VI):

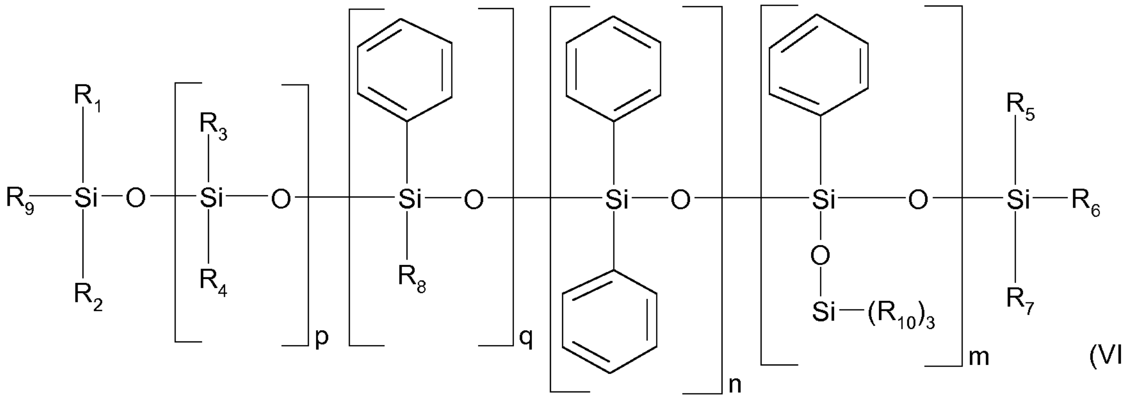

(VI)

in which:

- $R_1$ to $R_{10}$, independently of each other, are saturated or unsaturated, linear, cyclic or branched $C_1$-$C_{30}$ hydrocarbon-based radicals,
- m, n, p and q are, independently of each other, integers between 0 and 900, with the proviso that the sum m+n+q is other than 0.

Preferably, the sum "m+n+p+q" is between 1 and 100. Preferably, the sum "m+n+q" is between 1 and 900 and better still between 1 and 800. Preferably, q is equal to 0.

The phenyl silicone oil can be chosen from the phenyl silicones of following formula (VII):

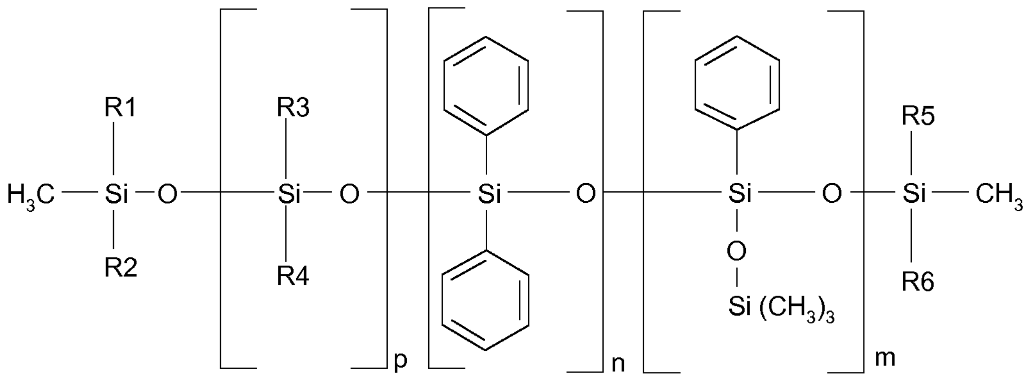

(VII)

in which:

- $R_1$ to $R_6$, independently of each other, are saturated or unsaturated, linear, cyclic or branched $C_1$-$C_{30}$ hydrocarbon-based radicals,
- m, n and p are, independently of each other, integers between 0 and 100, with the proviso that the sum n+m is between 1 and 100.

Preferably, $R_1$ to $R_6$, independently of each other, represent a saturated, linear or branched $C_1$-$C_{30}$ and espe-

cially $C_1$-$C_{12}$ hydrocarbon-based radical and in particular a methyl, ethyl, propyl or butyl radical.

$R_1$ to $R_6$ may especially be identical, and in addition may be a methyl radical.

Preferably, it is possible to have m = 1 or 2 or 3, and/or n = 0 and/or p = 0 or 1, in the formula (VII).

Use may be made of a phenylated silicone oil of formula (VI) having a viscosity at 25°C of between 5 and 1500 $mm^2/s$ (i.e. 5 to 1500 cSt) and preferably having a viscosity of between 5 and 1000 $mm^2/s$ (i.e. 5 to 1000 cSt).

As phenyl silicone oils of formula (VII), it is especially possible to use phenyl trimethicones such as DC556 from Dow Corning (22.5 cSt), the oil Silbione 70663V30 from Rhone-Poulenc (28 cSt) or diphenyl dimethicones such as Belsil oils, especially Belsil PDM1000® (1000 cSt), Belsil PDM 200® (200 cSt) and Belsil PDM 20 (20 cSt) from Wacker. The values in brackets represent the viscosities at 25°C.

Preferentially, the polar oil will be chosen from ethylhexyl methoxycinnamate, dodecamethylcyclohexasiloxane and caprylyl methicone, and mixtures thereof.

The polar oil(s) are preferably present in the composition in concentrations ranging from 0.5% to 60% and more preferentially from 2% to 30% by weight relative to the total weight of the composition.

The polar oil(s) are preferably present in the composition in concentrations ranging from 2% to 95% and more preferentially from 5% to 80% by weight relative to the total weight of the oily phase.

**LIQUID FATTY ACID:**

The liquid fatty acids in accordance with the invention correspond to formula (1) below:

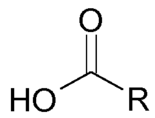

(1)

in which R is chosen from:

a) a saturated, branched $C_{14}$-$C_{22}$, preferably C18, alkyl group, or
b) an alkyl group comprising at least one linear or branched $C_{14}$-$C_{22}$, preferably $C_{18}$, double bond.

Among the compounds of formula (1) for which R denotes a saturated branched $C_{14}$-$C_{22}$ alkyl, mention may be made in particular of isostearic acid ($C_{18}$), such as the products sold under the trade names

- AEC Isostearic Acid® by the company A & E Connock (Perfumery & Cosmetics) Ltd.
- Isostearic Acid EX® by the company Kokyu Alcohol Kogyo Co., Ltd;
- Liponate ISA® by the company Lipo Chemicals, Inc.;
- Prisorine 3505® by the company Croda Europe, Ltd.

Among the compounds of formula (1) for which R represents a linear or branched $C_{14}$-$C_{22}$ alkyl group comprising at least one double bond, mention may be made in particular of:

- oleic acid ($C_{18}$)
- linoleic acid ($C_{18}$)
- linolenic acid ($C_{18}$); and
- mixtures thereof.

The liquid fatty acids in accordance with the invention are more particularly chosen from

- isostearic acid ($C_{18}$)
- oleic acid ($C_{18}$)
- linoleic acid ($C_{18}$)
- linolenic acid ($C_{18}$); and
- mixtures thereof.

Isostearic acid will be used more particularly.

The liquid fatty acid(s) in accordance with the invention are present in the compositions of the invention preferably

in concentrations ranging from 0.1% to 5% by weight and more preferentially from 0.2% to 3% by weight relative to the total weight of the composition.

## GLYCOL COMPOUND

**The compositions of the invention may further contain at least one glycol compound** corresponding to formula (2) below:

$$CH_3\text{--}[CH_2CH_2]_m\text{--}[COO]_n\text{-}CH_2\text{-}CH(OH)\text{-}CH_2OH \qquad (2)$$

in which:

- m is between 2 and 4
- n is equal to 0 or 1.

Among the preferential compounds of formula (2) that may be mentioned are:

- caprylyl glycol of formula $CH_3(CH_2)_4CH_2CH(OH)CH_2OH$ (m = 2 and n= 0) such as the commercial products sold under the trade names

    Botanistat CG® (Botanigenics, Inc.)
    Dermosoft Octiol® (Dr. Straetmans)
    Hydrolite-8 109169® (Symrise)
    199602 Hydrolite CG® (Symrise)
    Jeecide CAP® (Jeen International Corporation)
    Lexgard O® (Inolex Inc.)
    OriStar CPG® (Orient Stars LLC)

ParaPure (Paradigm Science, Inc;

- glyceryl caprylate of formula $CH_3(CH_2)_6COOCH_2CH(OH)CH_2OH$ (m = 3, n= 1) such as the commercial products sold under the trade names:

    AEC Glyceryl Caprylate (A & E Connock (Perfumery & Cosmetics) Ltd.)
    Capmul 708G (Abitec Corporation)
    CremerCOOR GC 8 (Cremer Oleo)
    Dermosoft GMCY (Dr. Straetmans)
    Dub 8G (Stearinerie Dubois Fils)
    Imwitor 308 (Cremer Oleo)
    Imwitor 988 (Cremer Oleo)
    Lexgard GMCY (Inolex Inc.)
    OriStar GCC (Orient Stars LLC)
    Sunsoft 707 (Taiyo Kagaku Company, Ltd.)
    Sunsoft 700 P-2 (Taiyo Kagaku Company, Ltd.)

- glyceryl caprate of formula $CH_3(CH_2)_8COOCH_2CH(OH)CH_2OH$ (m = 4 and n= 1) such as the commercial products sold under the trade names:

    AEC Glyceryl Caprate® (A & E Connock (Perfumery & Cosmetics) Ltd.)
    Capmul MCM C-10® (Abitec Corporation)
    Dermosoft GMC® (Dr. Straetmans)
    Lexgard GMC® (Inolex Inc.)
    Sunsoft 760® (Taiyo Kagaku Company, Ltd.)

- and mixtures thereof.

Mention may also be made of glyceryl caprylate/caprate mixtures such as the commercial products sold under the trade names:

AEC Glyceryl Caprylate/Caprate® (A & E Connock (Perfumery & Cosmetics) Ltd.) CremerCOOR GC810® (Cremer Oleo)
Dub 810 G® (Stearinerie Dubois Fils)

Use will be made more particularly of caprylyl glycol, glyceryl caprylate, and mixtures thereof.
The compound(s) of formula (2) are present in the compositions of the invention preferably in concentrations ranging from 0.1% to 5% by weight and more preferentially from 0.2% to 3% by weight relative to the total weight of the composition.

## HYDROPHOBIC-COATED PIGMENTS

The term *"pigments"* means white or coloured, mineral or organic particles, which are insoluble in an aqueous medium, and which are intended to colour and/or opacify the resulting composition and/or film. These pigments may be white or coloured, and mineral and/or organic.
The term "hydrophobic-coated pigment" means any pigment coated with at least one lipophilic or hydrophobic compound.
The term "lipophilic compound" means any compound that is soluble or dispersible in an oily phase.
The term "hydrophobic compound" means any compound that is insoluble in water.
According to a particular embodiment, the hydrophobic modified pigments used according to the invention are chosen from mineral pigments.
The term *"mineral pigment"* means any pigment that satisfies the definition in Ullmann's encyclopaedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminium powder or copper powder. The following mineral pigments may also be used: $Ta_2O_5$, $Ti_3O_5$, $Ti_2O_3$, TiO, $ZrO_2$ as a mixture with $TiO_2$, $ZrO_2$, $Nb_2O_5$, $CeO_2$, ZnS.
The particular size of the coated pigment is strictly greater than 100 nm.
For the purpose of the invention, the "size" of a particle means its D50. The D50, or volume average size, corresponds to the particle size defined such that 50% by volume of the particles have a size greater than D50.
The volume average size may be assessed by light diffraction using a Malvern MasterSizer laser particle size analyser, said particles to be evaluated being dispersed in a liquid medium, for instance octyldodecyl neopentanoate.
According to one embodiment, the size of the pigment particles according to the invention ranges from 100 nm to 25 $\mu$m, preferably from 200 nm to 10 $\mu$m.
In the context of the present invention, the hydrophobic modified mineral pigments are more particularly hydrophobic modified pigments of iron oxide and/or titanium dioxide.
They may also be nacres and/or particles with metallic glints.
The term *"nacres"* should be understood as meaning iridescent or non-iridescent coloured particles of any shape, especially produced by certain molluscs in their shell or alternatively synthesized, which have a colour effect via optical interference.
The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic dyestuffs.
Examples of nacres that may also be mentioned include natural mica covered with titanium oxide, with iron oxide, with natural pigment or with bismuth oxychloride.
The nacres may more particularly have a yellow, pink, red, bronze, orange, brown, gold and/or coppery colour or glint.
Advantageously, the nacres in accordance with the invention are micas covered with titanium dioxide or with iron oxide, and also bismuth oxychloride.
For the purposes of the present invention, the term *"particles with a metallic glint"* means any compound whose nature, size, structure and surface finish allow it to reflect the incident light, especially in a non-iridescent manner.
The particles with a metallic glint that may be used in the invention are in particular chosen from:

- particles of at least one metal and/or of at least one metal derivative;
- particles comprising a monomaterial or multimaterial organic or mineral substrate, at least partially coated with at least one layer with a metallic glint comprising at least one metal and/or at least one metal derivative, and
- mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals.
The term *"metal derivatives"* denotes compounds derived from metals, especially oxides, fluorides, chlorides and sulfides.

Coating of the pigment

The composition according to the invention advantageously comprises at least one pigment coated with at least one lipophilic or hydrophobic compound.
The coating may also comprise at least one additional non-lipophilic compound.
For the purposes of the invention, the *"coating"* of a pigment according to the invention generally denotes the total or partial surface treatment of the pigment with a surface agent, absorbed, adsorbed or grafted onto said pigment.
The surface-treated pigments may be prepared according to surface treatment techniques of chemical, electronic, mechanochemical or mechanical nature that are well known to those skilled in the art. Commercial products may also be used.
The surface agent may be absorbed, adsorbed or grafted onto the pigments by evaporation of solvent, chemical reaction and creation of a covalent bond.
According to one variant, the surface treatment is constituted of a coating of the pigments.
The coating may represent from 0.1% to 20% by weight and in particular from 0.5% to 5% by weight relative to the total weight of the coated pigment.
The coating may be performed, for example, by adsorption of a liquid surface agent onto the surface of the solid particles by simple mixing with stirring of the particles and of said surface agent, optionally with heating, prior to the incorporation of the particles into the other ingredients of the makeup or care composition.
The coating may be performed, for example, by chemical reaction of a surface agent with the surface of the solid pigment particles and creation of a covalent bond between the surface agent and the particles. This method is especially described in patent US 4 578 266.
The chemical surface treatment may consist in diluting the surface agent in a volatile solvent, dispersing the pigments in this mixture and then slowly evaporating off the volatile solvent, so that the surface agent is deposited at the surface of the pigments.

Lipophilic or hydrophobic treatment agent

According to a particular embodiment of the invention, the pigments may be coated according to the invention with at least one compound chosen from silicone surface agents; fluoro surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

*Silicone surface agent*

According to a particular embodiment, the pigments may be totally or partially surface-treated with a compound of silicone nature.
The silicone surface agents may be chosen from organopolysiloxanes, silane derivatives, silicone-acrylate copolymers, silicone resins, and mixtures thereof.
The term *"organopolysiloxane compound"* means a compound having a structure comprising an alternance of silicon atoms and oxygen atoms and comprising organic radicals linked to silicon atoms.

*i) Non-elastomeric organopolysiloxane*

Non-elastomeric organopolysiloxanes that may in particular be mentioned include polydimethylsiloxanes, polymethylhydrogenosiloxanes and polyalkoxydimethylsiloxanes.
The alkoxy group may be represented by the radical R-O- such that R represents methyl, ethyl, propyl, butyl or octyl, 2-phenylethyl, 2-phenylpropyl or 3,3,3-trifluoropropyl radicals, aryl radicals such as phenyl, tolyl or xylyl, or substituted aryl radicals such as phenylethyl.
One method for surface-treating pigments with a polymethylhydrogenosiloxane consists in dispersing the pigments in an organic solvent and then in adding the silicone compound. On heating the mixture, covalent bonds are created between the silicone compound and the surface of the pigment.
According to a preferred embodiment, the silicone surface agent may be a non-elastomeric organopolysiloxane,

especially chosen from polydimethylsiloxanes. According to one particular form, use may be made of triethoxysilylethyl polydimethylsiloxyethyl dimethicone, such as the commercial product sold under the name KF9908® from Shin-Etsu.

*ii) Alkylsilanes and alkoxysilanes*

Silanes bearing alkoxy functionality are especially described by Witucki in "A silane primer, Chemistry and applications of alkoxy silanes, Journal of Coatings Technology, 65, 822, pages 57-60, 1993*".*

Alkoxysilanes such as the alkyltriethoxysilanes and the alkyltrimethoxysilanes sold under the references Silquest A-137 (OSI Specialities) and Prosil 9202 (PCR) may be used for coating the pigments.

The use of alkylpolysiloxanes bearing a reactive end group such as alkoxy, hydroxyl, halogen, amino or imino is described in patent application JP H07-196946. They are also suitable for treating the pigments.

*iii) Silicone-acrylate polymers*

Grafted silicone-acrylic polymers having a silicone backbone as described in patents US 5 725 882, US 5 209 924, US 4 972 037, US 4 981 903, US 4 981 902 and US 5 468 477 and in patents US 5 219 560 and EP 0 388 582 may be used.

Other silicone-acrylate polymers may be silicone polymers comprising in their structure the unit of formula (I) below:

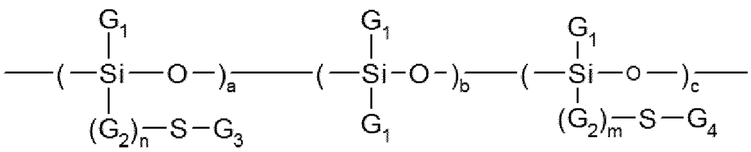

(I)

in which the radicals $G_1$, which may be identical or different, represent hydrogen or a $C_1$-$C_{10}$ alkyl radical or alternatively a phenyl radical; the radicals $G_2$, which may be identical or different, represent a $C_1$-$C_{10}$ alkylene group; $G_3$ represents a polymeric residue resulting from the (homo)polymerization of at least one ethylenically unsaturated anionic monomer; $G_4$ represents a polymeric residue resulting from the (homo)polymerization of at least one ethylenically unsaturated hydrophobic monomer; m and n are equal to 0 or 1; a is an integer ranging from 0 to 50; b is an integer that may be between 10 and 350, c is an integer ranging from 0 to 50; with the proviso that one of the parameters a and c is other than 0.

Preferably, the unit of formula (I) above has at least one, and even more preferentially all, of the following characteristics:

- the radicals $G_1$ denote an alkyl radical, preferably a methyl radical;
- n is non-zero, and the radicals $G_2$ represent a divalent $C_1$-$C_3$ radical, preferably a propylene radical;
- $G_3$ represents a polymeric radical resulting from the (homo)polymerization of at least one monomer of the ethylenically unsaturated carboxylic acid type, preferably acrylic acid and/or methacrylic acid;
- $G_4$ represents a polymeric radical resulting from the (homo)polymerization of at least one monomer of the ($C_1$-$C_{10}$)alkyl (meth)acrylate type, preferably such as isobutyl or methyl (meth)acrylate.

Examples of silicone polymers corresponding to formula (I) are especially polydimethylsiloxanes (PDMS) onto which are grafted, via a connecting chain unit of thiopropylene type, mixed polymer units of the poly(meth)acrylic acid type and of the polymethyl (meth)acrylate type.

Other examples of silicone polymers corresponding to formula (I) are especially polydimethylsiloxanes (PDMS) onto which are grafted, via a connecting chain unit of thiopropylene type, polymer units of the polyisobutyl (meth)acrylate type.

*iv) Silicone resins*

The silicone surface agent may be chosen from silicone resins.

The term *"resin"* means a three-dimensional structure.

The silicone resins may be soluble or swellable in silicone oils. These resins are crosslinked polyorganosiloxane polymers.

The nomenclature of silicone resins is known under the name "MDTQ", the resin being described as a function of the various siloxane monomer units that it comprises, each of the letters "MDTQ" characterizing a type of unit.

The letter M represents the monofunctional unit of formula $(CH_3)_3SiO_{1/2}$, the silicon atom being connected to only one oxygen atom in the polymer comprising this unit.

The letter D signifies a difunctional unit $(CH_3)_2SiO_{2/2}$ in which the silicon atom is bonded to two oxygen atoms.

The letter T represents a trifunctional unit of formula $(CH_3)SiO_{3/2}$.

In the units M, D and T defined above, at least one of the methyl groups may be substituted with a group R other than a methyl group, such as a hydrocarbon-based radical (especially alkyl) containing from 2 to 10 carbon atoms or a phenyl group, or alternatively a hydroxyl group.

Finally, the letter Q means a tetrafunctional unit $SiO_{4/2}$ in which the silicon atom is bonded to four hydrogen atoms, which are themselves bonded to the rest of the polymer.

Various resins with different properties may be obtained from these different units, the properties of these polymers varying as a function of the type of monomers (or units), of the type and number of substituted radicals, of the length of the polymer chain, of the degree of branching and of the size of the side chains.

Examples of these silicone resins that may be mentioned include:

- siloxysilicates, which may be trimethyl siloxysilicates of formula $[(CH_3)_3XSiXO]_xX(SiO_{4/2})_y$ (MQ units) in which x and y are integers ranging from 50 to 80;
- polysilsesquioxanes of formula $(CH_3SiO_{3/2})_x$ (T units) in which x is greater than 100 and at least one of the methyl radicals of which may be substituted with a group R as defined above;
- polymethylsilsesquioxanes, which are polysilsesquioxanes in which none of the methyl radicals is substituted with another group. Such polymethylsilsesquioxanes are described in document US 5 246 694.

As examples of commercially available polymethylsilsesquioxane resins, mention may be made of those sold:

- by the company Wacker under the reference Resin MK®, such as Belsil PMS MK®: polymer comprising $CH_3SiO_{3/2}$ repeating units (T units), which may also comprise up to 1% by weight of $(CH_3)_2SiO_{2/2}$ units (D units) and having an average molecular weight of about 10 000;
- by the company Shin-Etsu under the references KR-220L®, which are composed of T units of formula $CH_3SiO_{3/2}$ and contain Si-OH (silanol) end groups, under the reference KR-242A, which comprise 98% of T units and 2% of dimethyl D units and contain Si-OH end groups, or also under the reference KR-251, comprising 88% of T units and 12% of dimethyl D units and contain Si-OH end groups.

Siloxysilicate resins that may be mentioned include trimethyl siloxysilicate (TMS) resins, optionally in the form of powders. Such resins are sold under the references SR1000®, E 1 170-002® or SS 4230®, by the company General Electric or under the references TMS 803®, Wacker 803® and 804® by the company Wacker Silicone Corporation.

Mention may also be made of trimethylsiloxysilicate resins sold in a solvent such as cyclomethicone, sold under the name KF-7312J® by the company Shin-Etsu or DC 749® and DC 593® by the company Dow Corning.

As examples of commercial references of pigments treated with a silicone compound, mention may be made of:

- red iron oxide/dimethicone sold under the reference SA-C 338075-10® by the company Miyoshi Kasei; and

*Fluoro surface agent*

The pigments may be totally or partially surface-treated with a compound of fluoro nature.

The fluoro surface agents may be chosen from perfluoroalkyl phosphates, perfluoropolyethers, polytetrafluoropolyethylenes (PTFE), perfluoroalkanes, perfluoroalkyl silazanes, polyhexafluoropropylene oxides, and polyorganosiloxanes comprising perfluoroalkyl perfluoropolyether groups.

The term *"perfluoroalkyl radical"* means an alkyl radical in which all of the hydrogen atoms have been replaced with fluorine atoms.

Perfluoropolyethers are in particular described in patent application EP 0 486 135, and sold under the trade name Fomblin® by the company Montefluos.

Perfluoroalkyl phosphates are described in particular in patent application JP H05-86984. The perfluoroalkyl diethanolamine phosphates sold by Asahi Glass under the reference AsahiGuard AG530® may be used.

Among the linear perfluoroalkanes that may be mentioned are perfluorocycloalkanes, perfluoro(alkylcycloalkanes), perfluoropolycycloalkanes, aromatic perfluoro hydrocarbons (perfluoroarenes) and hydrocarbon-based perfluoro organic compounds comprising at least one heteroatom.

Among the perfluoroalkanes, mention may be made of the linear alkane series such as perfluorooctane, perfluorononane or perfluorodecane.

Among the perfluorocycloalkanes and perfluoro(alkylcycloalkanes), mention may be made of perfluorodecalin sold under the name Flutec PP5 GMP® by the company Rhodia, perfluoro(methyldecalin) and perfluoro($C_3$-$C_5$ alkylcy-

clohexanes) such as perfluoro(butylcyclohexane).

Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives such as tetracosafluorotetradecahydrophenanthrene.

Among the perfluoroarenes, mention may be made of perfluoronaphthalene derivatives, for instance perfluoronaphthalene and perfluoromethyl-1-naphthalene.

As examples of commercial references of pigments treated with a fluoro compound, mention may be made of:

- yellow iron oxide/perfluoroalkyl phosphate sold under the reference PF 5 Yellow 601® by the company Daito Kasei;
- red iron oxide/perfluoroalkyl phosphate sold under the reference PF 5 Red R 516L® by the company Daito Kasei;
- black iron oxide/perfluoroalkyl phosphate sold under the reference PF 5 Black BL100® by the company Daito Kasei;
- titanium dioxide/perfluoroalkyl phosphate sold under the reference PF 5 $TiO_2$ CR 50® by the company Daito Kasei;
- yellow iron oxide/perfluoropolymethyl isopropyl ether sold under the reference Iron oxide yellow BF-25-3® by the company Toshiki;
- DC Red 7/perfluoropolymethyl isopropyl ether sold under the reference D&C Red 7 FHC® by the company Cardre Inc.; and
- DC Red 6/PTFE sold under the reference T 9506® by the company Warner-Jenkinson.

*Fluorosilicone surface agent*

The pigments may be totally or partially surface-treated with a compound of fluorosilicone nature.

The fluorosilicone compound may be chosen from perfluoroalkyl dimethicones, perfluoroalkyl silanes and perfluoroalkyl trialkoxysilanes.

Perfluoroalkyl silanes that may be mentioned include the products LP-IT® and LP-4T® sold by Shin-Etsu Silicone.

The perfluoroalkyl dimethicones may be represented by the following formula:

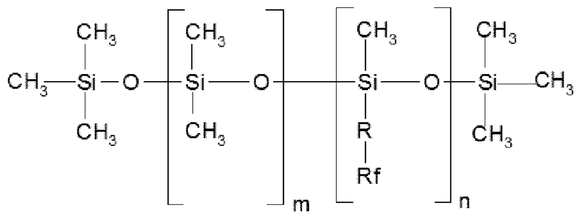

in which:

- R represents a linear or branched divalent alkyl group containing from 1 to 6 carbon atoms, preferably a divalent methyl, ethyl, propyl or butyl group;
- Rf represents a perfluoroalkyl radical containing 1 to 9 carbon atoms and preferably 1 to 4 carbon atoms;
- m is chosen between 0 and 150 and preferably from 20 to 100; and
- n is chosen between 1 and 300 and preferably from 1 to 100.

As examples of commercial references of pigments treated with a fluorosilicone compound, mention may be made of titanium dioxide/fluorosilicone sold under the reference Fluorosil Titanium dioxide 100TA® by the company Advanced Dermaceuticals International Inc.

*Other lipophilic surface agents*

The hydrophobic treatment agent may also be chosen from:

i) metal soaps such as aluminium dimyristate and the aluminium salt of hydrogenated tallow glutamate;

Metal soaps that may especially be mentioned include metal soaps of fatty acids containing from 12 to 22 carbon atoms and in particular those containing from 12 to 18 carbon atoms.

The metal of the metal soap may especially be zinc or magnesium.

Metal soaps that may be used include zinc laurate, magnesium stearate, magnesium myristate and zinc stearate, and mixtures thereof;

ii) fatty acids such as lauric acid, myristic acid, stearic acid and palmitic acid;
iii) N-acylamino acids or salts thereof, which may comprise an acyl group containing from 8 to 22 carbon atoms, for instance a 2-ethylhexanoyl, caproyl, lauroyl, myristoyl, palmitoyl, stearoyl or cocoyl group.

The amino acid may be, for example, lysine, glutamic acid or alanine.
The salts of these compounds may be the aluminium, magnesium, calcium, zirconium, zinc, sodium or potassium salts.
Thus, according to a particularly preferred embodiment, an N-acylamino acid derivative may especially be a glutamic acid derivative and/or a salt thereof, and more particularly a stearoyl glutamate, for instance aluminium stearoyl glutamate. It is, for example, the NAI surface treatment sold by Miyoshi;

iv) lecithin and derivatives thereof, such as hydrogenated lecithin, for instance the HLC surface treatment sold by LCW;

v) isopropyl triisostearyl titanate (INCI name: Isopropyl Titanium Triisostearate).

As examples of isopropyl titanium triisostearate (ITT)-treated pigments, mention may be made of those sold under the commercial references BTD-401® (titanium dioxide CI177891 and isopropyl titanium triisostearate), BBO-12® (iron oxide CI77499 and isopropyl titanium triisostearate), BYO-12® (Iron oxide CI77492 and isopropyl titanium triisostearate) and BRO-12® (Iron oxide CI77491 and isopropyl titanium triisostearate) by the company Kobo;

vi) isostearyl sebacate;
vii) natural plant or animal waxes or polar synthetic waxes;
viii) fatty esters, in particular jojoba esters;
ix) phospholipids; and
x) mixtures thereof.

The waxes mentioned in the compounds mentioned previously may be those generally used in cosmetics, as defined hereinbelow.
They may especially be hydrocarbon, silicone and/or fluoro waxes, optionally comprising ester or hydroxyl functions. They may also be of natural or synthetic origin.
The term *"polar wax"* means a wax containing chemical compounds comprising at least one polar group. Polar groups are well known to those skilled in the art; they may be, for example, alcohol, ester or carboxylic acid groups. Polyethylene waxes, paraffin waxes, microcrystalline waxes, ozokerite and Fischer-Tropsch waxes are not included among polar waxes.
In particular, the polar waxes have a mean Hansen solubility parameter $\delta_a$ at 25°C such that $\delta_a > 0$ $(J/cm^3)^{1/2}$ and better still $\delta_a > 1$ $(J/cm^3)^{1/2}$:

$$\delta_a = \sqrt{\delta_p^2 + \delta_h^2}$$

in which $\delta_p$ and $\delta_h$ are, respectively, the polar contributions and contributions of interaction types specific to the Hansen solubility parameters.
The definition of solvents in the three-dimensional solubility space according to Hansen is described in the article by C. M. Hansen: "The three-dimensional solubility parameters", J. Paint Technol. 39, 105 (1967):

- $\delta_h$ characterizes the specific interaction forces (such as hydrogen bonding, acid/base, donor/acceptor, etc.);
- $\delta_p$ characterizes the Debye interaction forces between permanent dipoles and also the Keesom interaction forces between induced dipoles and permanent dipoles.

The solubility parameters are calculated with the HSPiP v4.1 software.
The parameters $\delta_p$ and $\delta_h$ are expressed in $(J/CM^3)^{1/2}$.
A polar wax is especially formed from molecules comprising, besides carbon and hydrogen atoms in their chemical structure, heteroatoms (such as O, N and P).
Non-limiting illustrations of these polar waxes that may especially be mentioned include natural polar waxes, such as beeswax, lanolin wax, orange wax, lemon wax and Chinese insect waxes, rice bran wax, carnauba wax, candelilla wax, ouricury wax, cork fibre wax, sugarcane wax, Japan wax, sumac wax and montan wax.

According to one particular embodiment, the pigments may be coated with at least one compound chosen from N-acylamino acids or salts thereof, isopropyl triisostearyl titanate; silicone surface agents; natural plant or animal waxes; hydrogenated lecithin, fatty esters; and mixtures thereof.

According to a more particularly preferred embodiment, the pigments may be coated with an N-acylamino acid and/or a salt thereof, in particular with a glutamic acid derivative and/or a salt thereof, especially a stearoyl glutamate, for instance aluminium stearoyl glutamate.

According to one more particularly preferred embodiment, use will be made of hydrophobic coated pigments chosen from titanium dioxides and iron oxides coated with aluminium stearoyl glutamate, sold, for example, under the reference NAI® by Miyoshi Kasei.

According to a more particularly preferred embodiment, use will be made of hydrophobic coated pigments chosen from titanium dioxides and iron dioxides coated with isopropyl titanium triisostearate (ITT); mention may be made of those sold under the commercial references BTD-401® (titanium dioxide CI177891 and isopropyl titanium triisostearate), BBO-I2® (iron oxide CI77499 and isopropyl titanium triisostearate), BYO-I2® (iron oxide CI77492 and isopropyl titanium triisostearate), and BRO-I2® (iron oxide CI77491 and isopropyl titanium triisostearate) by the company Kobo.

The pigments coated with at least one hydrophobic compound are present in a composition of the invention in a proportion preferably of at least 5% by weight, more preferentially ranging from 5% to 25% by weight and even more preferentially ranging from 8% to 15%, relative to the total weight of the composition.

## ADDITIONAL DYESTUFFS

A composition according to the invention may also comprise at least one additional dyestuff, preferably in a proportion of at least 0.01% by weight relative to the total weight of the composition.

For obvious reasons, this amount is liable to vary significantly with regard to the intensity of the desired colour effect and of the colour intensity afforded by the dyestuffs under consideration, and its adjustment clearly falls within the competence of those skilled in the art.

A composition according to the invention may comprise from 0.01% to 25% by weight, especially from 0.1% to 25% by weight, in particular from 1% to 20% by weight and preferably from 5% to 15% by weight of dyestuffs relative to the total weight of said composition.

As specified above, the colorants that are suitable for use in the invention may be water-soluble, but may also be liposoluble.

For the purposes of the invention, the term *"water-soluble dyestuff"* means any natural or synthetic, generally organic compound, which is soluble in an aqueous phase or water-miscible solvents and which is capable of imparting colour.

As water-soluble dyes that are suitable for use in the invention, mention may be made in particular of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophylline, methylene blue, anthocyanins (enocianin, black carrot, hibiscus and elder), caramel and riboflavin.

The water-soluble dyes are, for example, beetroot juice and caramel.

For the purposes of the invention, the term *"liposoluble dyestuff"* means any natural or synthetic, generally organic compound, which is soluble in an oily phase or in solvents that are miscible with a fatty substance, and which is capable of imparting colour.

As liposoluble dyes that are suitable for use in the invention, mention may be made in particular of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

They may in particular be non-hydrophobic-coated pigments, non-hydrophobic-coated nacres and/or non-hydrophobic-coated particles with metallic glints.

The term *"non-hydrophobic-coated pigment"* means any pigment which is not coated with at least one lipophilic or hydrophobic compound.

The additional pigments may be white or coloured, and mineral and/or organic.

As non-hydrophobic-coated mineral pigments that may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

They may also be a pigment having a structure that may be, for example, of sericite/brown iron oxide/titanium dioxide/silica type. Such a pigment is sold, for example, under the reference Coverleaf NS® or JS® by the company Chemicals and Catalysts, and has a contrast ratio in the region of 30.

They may also be pigments having a structure that may be, for example, of silica microsphere type containing iron oxide. An example of a pigment having this structure is the product sold by the company Miyoshi under the reference

PC Ball PC-LL-100 P®, this pigment being constituted of silica microspheres containing yellow iron oxide.

Advantageously, the additional pigments in accordance with the invention are iron oxides and/or titanium dioxides.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic dyestuffs.

Examples of nacres that may also be mentioned include natural mica covered with titanium oxide, with iron oxide, with natural pigment or with bismuth oxychloride.

Among the nacres available on the market, mention may be made of the nacres Timica, Flamenco® and Duochrome® (based on mica) sold by the company Engelhard, the Timiron nacres sold by the company Merck, the Prestige mica-based nacres, sold by the company Eckart, and the Sunshine synthetic mica-based nacres, sold by the company Sun Chemical.

The nacres may more particularly have a yellow, pink, red, bronze, orange, brown, gold and/or coppery colour or glint.

Advantageously, the nacres in accordance with the invention are micas covered with titanium dioxide or with iron oxide, and also bismuth oxychloride.

Illustrations of these additional particles with metallic glints that may be mentioned include aluminium particles, such as those sold under the names Starbrite 1200 EAC® by the company Siberline and Metalure® by the company Eckart and glass particles coated with a metallic layer, in particular those described in documents JP-A-09188830, JP-A-10158450, JP-A-10158541, JP-A-07258460 and JP-A-05017710.

## FILLERS

Advantageously, a composition according to the invention may also comprise one or more fillers conventionally used in care and/or makeup compositions.

These fillers are colourless or white solid particles of any form, which are in a form that is insoluble and dispersed in the medium of the composition.

These fillers, of mineral or organic, natural or synthetic nature, give the composition containing them softness and give the makeup result a matt effect and uniformity. In addition, these fillers advantageously make it possible to combat various attacking factors such as sebum or sweat.

As illustrations of these fillers, mention may be made of talc, mica, silica, kaolin, poly-p-alanine powder and polyethylene powder, powders of tetrafluoroethylene polymers (Teflon®), lauroyllysine, starch, boron nitride, hollow polymer microspheres such as those of polyvinylidene chloride/acrylonitrile, for instance Expancel® (Nobel Industrie), acrylic acid copolymer microspheres, silicone resin microbeads (for example Tospearls® from Toshiba), polyorganosiloxane elastomer particles, precipitated calcium carbonate, magnesium carbonate, magnesium hydrogen carbonate, hydroxyapatite, barium sulfate, aluminium oxides, polyurethane powders, composite fillers, hollow silica microspheres, and glass or ceramic microcapsules. Use may also be made of particles which are in the form of hollow sphere portions, as described in patent applications JP-2003 128 788 and JP-2000 191 789.

## (Meth)acrvlic polymer particles

According to a particular form of the invention, the compositions according to the invention may contain (meth)acrylic polymer particles.

The (meth)acrylic polymer particles are especially particles of polymethyl methacrylate, poly(methyl methacrylate/ethylene glycol dimethacrylate), poly(allyl methacrylate/ethylene glycol dimethacrylate), ethylene glycol dimethacrylate/lauryl methacrylate copolymer;

(Meth)acrylic polymer powders that may be mentioned include:

- the polymethyl methacrylate powders sold under the name Covabead LH85® by the company Wackherr;
- the polymethyl methacrylate/ethylene glycol dimethacrylate powders sold under the name Dow Corning 5640 Microsponge Skin Oil Adsorber® by the company Dow Corning and the name Ganzpearl GMP-0820® by the company Ganz Chemical;
- the polyallyl methacrylate/ethylene glycol dimethacrylate powders sold under the names Polypore L200® and Polypore E200® by the company Amcol Health and Beauty Solutions Inc.;
- ethylene glycol dimethacrylate/lauryl methacrylate copolymer powders (INCI name: Lauryl methacrylate/glycol dimethacrylate crosspolymer sold under the name Polytrap 6603 Adsorber® by the company Amcol Health & Beauty Solutions.

Preferably, the (meth)acrylic polymer particles have a number-average size ranging from 50 nm to 350 microns, better still from 100 nm to 100 microns and even more preferentially from 0.5 to 100 microns.

The (meth)acrylic polymer particles are present in the composition according to the invention in a content ranging from 0.01% to 30% by weight, preferably ranging from 0.05% to 20% by weight and most preferentially ranging from 0.2% to 10% by weight relative to the total weight of the composition.

In particular, such fillers may be present in a composition according to the invention in a content ranging from 0.01% to 25% by weight, especially from 0.1% to 20% by weight, in particular from 1% to 10% by weight, relative to the total weight of the composition.

## ACTIVE AGENT

A composition according to the invention may comprise at least one moisturizer (also known as a humectant), in particular for a care application.

Preferably, the moisturizer is glycerol.

The moisturizer(s) may be present in the composition in a content ranging from 0.1% to 15% by weight, especially from 0.5% to 10% by weight or even from 1% to 6% by weight relative to the total weight of said composition.

As other active agents that may be used in the composition of the invention, examples that may be mentioned include vitamins and sunscreens other than liquid lipophilic organic UV-screening agents, and mixtures thereof.

Preferably, a composition according to the invention comprises at least one active agent.

## HYDROPHOBIC FILM-FORMING POLYMERS

According to a particularly preferred form of the invention, the compositions according to the invention comprise at least one hydrophobic film-forming polymer.

This type of polymer is particularly advantageous insofar as it makes it possible to significantly increase the persistence of the matt effect over time. As indicated previously, the performance of these polymers is advantageously increased by means of using them in a composition according to the invention.

For the purposes of the invention, the term "polymer" means a compound corresponding to the repetition of one or more units (these units resulting from compounds known as monomers). This or these unit(s) are repeated at least twice and preferably at least three times.

For the purposes of the present invention, the term "hydrophobic film-forming polymer" is intended to denote a film-forming polymer that has no affinity for water and, in this respect, does not lend itself to a formulation in the form of a solute in an aqueous medium. In particular, the term "hydrophobic polymer" means a polymer which has a solubility in water at 25°C of less than 1% by weight.

The term "film-forming polymer" means a polymer that is capable of forming, by itself or in the presence of an auxiliary film-forming agent, a macroscopically continuous film on a support, especially on keratin materials, and preferably a cohesive film, and better still a film whose cohesion and mechanical properties are such that said film may be isolable and manipulable in isolation, for example when said film is prepared by pouring onto a non-stick surface, for instance a Teflon-coated or silicone-coated surface.

In particular, the hydrophobic film-forming polymer is a polymer chosen from the group comprising:

- film-forming polymers that are soluble in an organic solvent medium, in particular liposoluble polymers; this means that the polymer is soluble or miscible in the organic medium and forms a single homogeneous phase when it is incorporated into the medium;
- film-forming polymers that are dispersible in an organic solvent medium, which means that the polymer forms an insoluble phase in the organic medium, the polymer remaining stable and/or compatible once incorporated into this medium. In particular, such polymers may be in the form of non-aqueous dispersions of polymer particles, preferably dispersions in silicone oils or hydrocarbon-based oils; in one embodiment, the non-aqueous polymer dispersions comprise polymer particles stabilized on their surface with at least one stabilizer; these non-aqueous dispersions are often referred to as NADs;
- film-forming polymers in the form of aqueous dispersions of polymer particles, which means that the polymer forms an insoluble phase in water, the polymer remaining stable and/or compatible once incorporated into the water, the polymer particles possibly being stabilized at their surface with at least one stabilizer. These polymer particles are often known as *latices.*

Hydrophobic film-forming polymers that may especially be mentioned include homopolymers and copolymers of a compound bearing an ethylenic unit, acrylic polymers and copolymers, polyurethanes, polyesters, polyureas, cellulose-based polymers such as nitrocellulose, silicone polymers such as silicone resins, silicone polyamides, polymers

bearing a non-silicone organic backbone grafted with monomers containing a polysiloxane, polyamide polymers and copolymers, and polyisoprenes.

A composition according to the invention may comprise from 0.1% to 30% by weight, preferably from 0.2% to 20% by weight and even more preferentially from 0.5% to 15% by weight of hydrophobic film-forming polymer(s) relative to the total weight of the composition.

In particular, said hydrophobic film-forming polymer(s) are present totally or partially, and preferably solely, in the gelled oily phase.

As hydrophobic film-forming polymers that are most particularly suitable for use in the invention, mention may be made especially of block ethylenic polymers, vinyl polymers comprising at least one carbosiloxane dendrimer derivative and silicone resins (T resin, MQ resin).

## I. Silicone resins

According to one embodiment variant, a composition according to the invention may comprise, as hydrophobic film-forming polymer, at least one silicone resin.

More generally, the term "resin" is understood to mean a compound, the structure of which is three-dimensional. "Silicone resins" are also referred to as "siloxane resins". Thus, within the meaning of the present invention, a polydimethylsiloxane is not a silicone resin.

The nomenclature of silicone resins (also known as siloxane resins) is known under the name "MDTQ", the resin being described as a function of the various siloxane monomer units it comprises, each of the letters "MDTQ" characterizing a type of unit.

The letter "M" represents the Monofunctional unit of formula $R_1R_2R_3SiO_{1/2}$, the silicon atom being connected to only one oxygen atom in the polymer comprising this unit.

The letter "D" signifies a Difunctional unit $R_1R_2SiO_{2/2}$ in which the silicon atom is connected to two oxygen atoms.

The letter "T" represents a Trifunctional unit of formula $R_1SiO_{3/2}$.

Such resins are described, for example, in the Encyclopedia of Polymer Science and Engineering, vol. 15, John Wiley and Sons, New York, (1989), pp. 265-270, and US 2 676 182, US 3 627 851, US 3 772 247, US 5 248 739 or else US 5 082 706, US 5 319 040, US 5 302 685 and US 4 935 484.

In the units M, D and T defined previously, R, namely $R_1$ and $R_2$, represents a hydrocarbon-based radical (in particular alkyl) containing from 1 to 10 carbon atoms, a phenyl group, a phenylalkyl group or even a hydroxyl group.

Finally, the letter Q means a tetrafunctional unit $SiO_{4/2}$ in which the silicon atom is bonded to four hydrogen atoms, which are themselves bonded to the rest of the polymer.

Various silicone resins with different properties may be obtained from these different units, the properties of these polymers varying as a function of the type of monomer (or unit), the nature and number of the radical R, the length of the polymer chain, the degree of branching and the size of the pendent chains.

As silicone resins that may be used in the compositions according to the invention, use may be made, for example, of silicone resins of MQ type, of T type or of MQT type.

### MQ resins

As examples of silicone resins of MQ type, mention may be made of the alkyl siloxysilicates of formula $[(R_1)_3SiO_{1/2}]_x(SiO_{4/2})_y$ (MQ units) in which x and y are integers ranging from 50 to 80, and such that the group $R_1$ represents a radical as defined previously, and is preferably an alkyl group containing from 1 to 8 carbon atoms or a hydroxyl group, preferably a methyl group.

As examples of solid silicone resins of MQ type of trimethyl siloxysilicate type, mention may be made of those sold under the reference SR1000® by the company General Electric, under the reference TMS 803® by the company Wacker, or under the name KF-7312J® by the company Shin-Etsu or DC749® or DC593® by the company Dow Corning.

As silicone resins comprising MQ siloxysilicate units, mention may also be made of phenylalkylsiloxysilicate resins, such as phenylpropyldimethylsiloxysilicate (Silshine 151® sold by the company General Electric). The preparation of such resins is described especially in patent US 5 817 302.

### T resins

Examples of silicone resins of type T that may be mentioned include the polysilsesquioxanes of formula $(RSiO_{3/2})_x$ (units T) in which x is greater than 100 and such that the group R is an alkyl group containing from 1 to 10 carbon atoms, said polysilsesquioxanes also possibly comprising Si-OH end groups.

Polymethylsilsesquioxane resins that may preferably be used are those in which R represents a methyl group, for instance those sold:

- by the company Wacker under the reference Resin MK®, such as Belsil PMS MK®: polymer comprising $CH_3SiO_{3/2}$ repeating units (units T), which may also comprise up to 1% by weight of $(CH_3)_2SiO_{2/2}$ units (units D) and having an average molecular weight of about 10 000 g/mol, or
- by the company Shin-Etsu under the reference KR220L®, which are composed of units T of formula $CH_3SiO_{3/2}$ and have Si-OH (silanol) end groups, under the reference KR242A, which comprise 98% of units T and 2% of dimethyl units D and have Si-OH end groups, or alternatively under the reference KR251® comprising 88% of units T and 12% of dimethyl units D and have Si-OH end groups.

MQT resins

Resins comprising MQT units that are especially known are those mentioned in document US 5 110 890.
A preferred form of resins of MQT type are MQT-propyl (also known as MQTpr) resins. Such resins that may be used in the compositions according to the invention are especially the resins described and prepared in patent application WO 2005/075 542.
The MQ-T-propyl resin preferably comprises the following units:

(i) $(R1_3SiO_{1/2})_a$
(ii) $(R2_2SiO_{2/2})_b$
(iii) $(R3SiO_{3/2})_c$ and
(iv) $(SiO_{4/2})_d$

with:

- $R_1$, $R_2$ and $R_3$ independently representing a hydrocarbon-based (in particular alkyl) radical containing from 1 to 10 carbon atoms, a phenyl group, a phenylalkyl group or alternatively a hydroxyl group and preferably an alkyl radical containing from 1 to 8 carbon atoms or a phenyl group,
- a being between 0.05 and 0.5,
- b being between 0 and 0.3,
- c being greater than zero,
- d being between 0.05 and 0.6,
- a + b + c + d = 1, and a, b, c and d being mole fractions,

on condition that more than 40 mol% of the groups R3 of the siloxane resin are propyl groups.
Preferably, the siloxane resin comprises the following units:

(i) $(R1_3SiO_{1/2})_a$,
(iii) $(R3SiO_{3/2})_c$; and
(iv) $(SiO_{4/2})$d

with:

- R1 and R3 independently representing an alkyl group containing from 1 to 8 carbon atoms, R1 preferably being a methyl group and R3 preferably being a propyl group,
- a being between 0.05 and 0.5 and preferably between 0.15 and 0.4,
- c being greater than zero, preferably between 0.15 and 0.4,
- d being between 0.05 and 0.6, preferably between 0.2 and 0.6 or alternatively between 0.2 and 0.55,
- a + b + c + d = 1, and a, b, c and d being mole fractions,

on condition that more than 40 mol% of the groups R3 of the siloxane resin are propyl groups.
The siloxane resins that may be used according to the invention may be obtained via a process comprising the reaction of:

A) an MQ resin comprising at least 80 mol% of units $(R1_3SiO_{1/2})_a$ and $(SiO_{4/2})_d$; with

- R1 representing an alkyl group containing from 1 to 8 carbon atoms, an aryl group, a carbinol group or an amino group,
- a and d being greater than zero,
- the ratio a/d being between 0.5 and 1.5;

and:
B) a T-propyl resin comprising at least 80 mol% of units $(R3SiO_{3/2})_c$; with

- R3 representing an alkyl group containing from 1 to 8 carbon atoms, an aryl group, a carbinol group or an amino group,
- c being greater than zero,

on condition that at least 40 mol% of the groups R3 are propyl groups, in which the mass ratio A/B is between 95/5 and 15/85, and preferably mass ratio A/B is 30/70.
Advantageously, the A/B weight ratio is between 95/5 and 15/85. Preferably, the A/B ratio is less than or equal to 70/30. These preferred ratios have proven to allow comfortable deposits due to the absence of percolation of the rigid particles of MQ resin in the deposit.
Thus, preferably, the silicone resin is chosen from the group comprising:

a) a resin of MQ type, chosen especially from (i) alkyl siloxysilicates, which may be trimethyl siloxysilicates, of formula $[R1_3SiO_{1/2}]_x(SiO_{4/2})_y$, in which x and y are integers ranging from 50 to 80, and such that the group R1 represents a hydrocarbon-based radical containing from 1 to 10 carbon atoms, a phenyl group, a phenylalkyl group or a hydroxyl group, and preferably is an alkyl group containing from 1 to 8 carbon atoms, preferably a methyl group, and (ii) phenylalkyl siloxysilicate resins, such as phenylpropyldimethyl siloxysilicate, and/or
b) a resin of T type, chosen especially from the polysilsesquioxanes of formula $(RSiO_{3/2})_x$, in which x is greater than 100 and the group R is an alkyl group containing from 1 to 10 carbon atoms, for example a methyl group, said polysilsesquioxanes also possibly comprising Si-OH end groups, and/or
c) a resin of MQT type, especially of MQT-propyl type, which may comprise units (i) $(R1_3SiO_{1/2})_a$, (ii) $(R2_2SiO_{2/2})_b$, (iii) $(R3SiO_{3/2})_c$ and (iv) $(SiO_{4/2})_d$,

- with R1, R2 and R3 independently representing a hydrocarbon-based radical, especially alkyl, containing from 1 to 10 carbon atoms, a phenyl group, a phenylalkyl group or a hydroxyl group and preferably an alkyl radical containing from 1 to 8 carbon atoms or a phenyl group,
- a being between 0.05 and 0.5,
- b being between 0 and 0.3,
- c being greater than zero,
- d being between 0.05 and 0.6,
- a + b + c + d = 1, and a, b, c and d being mole fractions,

on condition that more than 40 mol% of the groups R3 of the siloxane resin are propyl groups.
Advantageously, a composition according to the invention may comprise, as hydrophobic film-forming polymer, at least one trimethyl siloxysilicate resin.

## II. **Lipodispersible film-forming polymers in the form of non-aqueous dispersions of polymer particles, also known as NADs**

According to another embodiment variant, a composition according to the invention may comprise, as hydrophobic film-forming polymer, at least one polymer chosen from lipodispersible film-forming polymers in the form of non-aqueous dispersions of polymer particles, also known as NADs.
Non-aqueous dispersions of hydrophobic film-forming polymer that may be used include dispersions of particles of a grafted ethylenic polymer, preferably an acrylic polymer, in a liquid oily phase:

- either in the form of ethylenic polymer particles dispersed in the absence of additional stabilizer at the surface of the particles, as described especially in document WO 04/055 081,
- or in the form of surface-stabilized particles dispersed in the liquid fatty phase. The dispersion of surface-stabilized polymer particles may be manufactured as described in document EP-A-749 747. The polymer particles may in particular be surface-stabilized by means of a stabilizer that may be a block polymer, a grafted polymer and/or a random polymer, alone or as a mixture. Dispersions of film-forming polymer in the liquid fatty phase, in the presence of stabilizers, are especially described in documents EP-A-748 746, EP-A-923 928, EP-A-930 060.

Advantageously, dispersions of ethylenic polymer particles dispersed in the absence of additional stabilizer at the surface of said particles are used.
Examples of polymers of NAD type that may be mentioned more particularly include acrylic dispersions in

isododecane, for instance Mexomer PAP® (acrylic copolymer as a dispersion in isododecane (25%) with pyrene/isoprene copolymer) sold by the company Chimex.

### III. Block ethylenic copolymer

According to a first embodiment of the invention, the hydrophobic film-forming polymer is a block ethylenic copolymer, containing at least one first block with a glass transition temperature (Tg) of greater than or equal to 40°C and being totally or partly derived from one or more first monomers, which are such that the homopolymer prepared from these monomers has a glass transition temperature of greater than or equal to 40°C, and at least one second block with a glass transition temperature of less than or equal to 20°C and being derived totally or partly from one or more second monomers, which are such that the homopolymer prepared from these monomers has a glass transition temperature of less than or equal to 20°C, said first block and said second block being connected together via a random intermediate segment comprising at least one of said first constituent monomers of the first block and at least one of said second constituent monomers of the second block, and said block copolymer having a polydispersity index I of greater than 2.

The block polymer used according to the invention thus comprises at least one first block and at least one second block.

The term "at least one block" means one or more blocks.

The term "block" polymer means a polymer comprising at least two different blocks and preferably at least three different blocks.

The term "ethylenic polymer" means a polymer obtained by polymerization of monomers comprising an ethylenic unsaturation.

The block ethylenic polymer used according to the invention is prepared exclusively from monofunctional monomers.

This means that the block ethylenic polymer used according to the present invention does not contain any multifunctional monomers, which make it possible to break the linearity of a polymer so as to obtain a branched or even crosslinked polymer, as a function of the content of multifunctional monomer. The polymer used according to the invention does not, either, contain any macromonomers (the term "macromonomer" means a monofunctional monomer containing pendent groups of polymeric nature, and preferably having a molecular mass of greater than 500 g/mol, or alternatively a polymer comprising on only one of its ends a polymerizable (or ethylenically unsaturated) end group), which are used in the preparation of a grafted polymer.

It is pointed out that, in the text hereinabove and hereinbelow, the terms "first" and "second" blocks do not in any way condition the order of said blocks in the structure of the polymer.

The first block and the second block of the polymer used in the invention may be advantageously mutually incompatible.

The term "mutually incompatible blocks" means that the mixture formed from a polymer corresponding to the first block and from a polymer corresponding to the second block is not miscible in the polymerization solvent that is in major amount by weight for the block polymer, at room temperature (25°C) and atmospheric pressure ($10^5$ Pa), for a content of the mixture of said polymers of greater than or equal to 5% by weight, relative to the total weight of the mixture of said polymers and of said polymerization solvent, it being understood that:

- i) said polymers are present in the mixture in a content such that the respective weight ratio ranges from 10/90 to 90/10, and that
- ii) each of the polymers corresponding to the first and second blocks has an average (weight-average or number-average) molar mass equal to that of the block polymer ± 15%.

In the case of a mixture of polymerization solvents, and in the event that two or more solvents are present in identical mass proportions, said polymer mixture is immiscible in at least one of them.

Needless to say, in the case of a polymerization performed in a single solvent, this solvent is the solvent that is in major amount.

The block polymer according to the invention comprises at least one first block and at least one second block that are connected together via an intermediate segment comprising at least one constituent monomer of the first block and at least one constituent monomer of the second block. The intermediate segment (also known as the intermediate block) has a glass transition temperature Tg that is between the glass transition temperatures of the first and second blocks.

The intermediate segment is a block comprising at least one constituent monomer of the first block and at least one constituent monomer of the second block of the polymer allowing these blocks to be "compatibilized".

Advantageously, the intermediate segment comprising at least one constituent monomer of the first block and

at least one constituent monomer of the second block of the polymer is a statistical polymer.

Preferably, the intermediate block is derived essentially from constituent monomers of the first block and of the second block.

The term "essentially" means at least 85%, preferably at least 90%, better still 95% and even better still 100%.

The block polymer according to the invention is advantageously a film-forming block ethylenic polymer.

The term "ethylenic polymer" means a polymer obtained by polymerization of monomers comprising an ethylenic unsaturation.

The term "film-forming polymer" means a polymer that is capable of forming, by itself or in the presence of an auxiliary film-forming agent, a continuous deposit on a support, especially on keratin materials.

Preferentially, the polymer according to the invention does not comprise any silicon atoms in its backbone. The term "backbone" means the main chain of the polymer, as opposed to the pendent side chains.

Preferably, the polymer according to the invention is not water-soluble, i.e. the polymer is not soluble in water or in a mixture of water and linear or branched lower monoalcohols containing from 2 to 5 carbon atoms, for instance ethanol, isopropanol or n-propanol, without modifying the pH, at the solids content of at least 1% by weight, at room temperature (25°C).

Preferably, the polymer according to the invention is not an elastomer.

The term "non-elastomeric polymer" means a polymer which, when it is subjected to a constraint intended to pull it (for example by 30% relative to its initial length), does not return to a length substantially identical to its initial length when the constraint ceases.

More specifically, the term "non-elastomeric polymer" denotes a polymer with an instantaneous recovery Ri < 50% and a delayed recovery R2h < 70% after having been subjected to a 30% elongation. Preferably, Ri is < 30% and R2h < 50%.

More specifically, the non-elastomeric nature of the polymer is determined according to the following protocol: A polymer film is prepared by pouring a solution of the polymer in a Teflon-coated mould, followed by drying for 7 days in an environment conditioned at 23 ± 5°C and 50 ± 10% relative humidity.

A film about 100 μm thick is thus obtained, from which are cut rectangular test specimens (for example using a punch) 15 mm wide and 80 mm long.

This sample is subjected to a tensile stress using a machine sold under the reference Zwick, under the same temperature and humidity conditions as for the drying.

The specimens are stretched at a speed of 50 mm/min and the distance between the jaws is 50 mm, which corresponds to the initial length (l0) of the specimen.

The instantaneous recovery Ri is determined in the following manner:

- the specimen is stretched by 30% ($\varepsilon_{max}$), i.e. approximately 0.3 times its initial length (l0);
- the stress is released by applying a return speed equal to the pull speed, i.e. 50 mm/min, and the residual elongation of the test specimen is measured as a percentage, after returning to zero stress load ($\varepsilon_i$).

The percentage instantaneous recovery (Ri) is given by the formula below:

$$R_i = ((\varepsilon_{max} - \varepsilon_i)/\varepsilon_{max}) \text{ x } 100$$

To determine the delayed recovery, the percentage residual elongation of the test specimen ($\varepsilon_{2h}$) is measured after 2 hours, 2 hours after returning to zero stress load.

The percentage delayed recovery (R2h) is given by the following formula:

$$R_{2h} = ((\varepsilon_{max} - \varepsilon_{2h})/\varepsilon_{max}) \text{ x } 100$$

Purely as a guide, a polymer according to one embodiment of the invention preferably has an instantaneous recovery Ri of 10% and a delayed recovery $R_{2h}$ of 30%.

The polydispersity index of the polymer of the invention is greater than 2.

Advantageously, the block polymer used in the compositions according to the invention has a polydispersity index I of greater than 2, for example ranging from 2 to 9, preferably greater than or equal to 2.5, for example ranging from 2.5 to 8 and better still greater than or equal to 2.8, and in particular ranging from 2.8 to 6.

The polydispersity index I of the polymer is equal to the ratio of the weight-average molar mass Mw to the number-average molar mass Mn.

The weight-average molar mass (Mw) and number-average molar mass (Mn) are determined by gel permeation

liquid chromatography (THF solvent, calibration curve established with linear polystyrene standards, refractometric detector).

The weight-average mass (Mw) of the polymer according to the invention is preferably less than or equal to 300 000; it ranges, for example, from 35 000 to 200 000 and better still from 45 000 to 150 000 g/mol.

The number-average mass (Mn) of the polymer according to the invention is preferably less than or equal to 70 000; it ranges, for example, from 10 000 to 60 000 and better still from 12 000 to 50 000 g/mol.

Preferably, the polydispersity index of the polymer according to the invention is greater than 2, for example ranging from 2 to 9, preferably greater than or equal to 2.5, for example ranging from 2.5 to 8, and better still greater than or equal to 2.8, and in particular ranging from 2.8 to 6.

First block with a Tg of greater than or equal to 40°C

The block with a Tg of greater than or equal to 40°C has, for example, a Tg ranging from 40 to 150°C, preferably greater than or equal to 50°C, for example ranging from 50°C to 120°C and better still greater than or equal to 60°C, for example ranging from 60°C to 120°C.

The glass transition temperatures indicated for the first and second blocks may be theoretical Tg values determined from the theoretical Tg values of the constituent monomers of each of the blocks, which may be found in a reference manual such as the Polymer Handbook, 3rd Edition, 1989, John Wiley, according to the following relationship, known as Fox's law:

$$1/Tg = \Sigma (\omega_i / Tg_i), i$$

$\omega_i$ being the mass fraction of the monomer i in the block under consideration and Tgi being the glass transition temperature of the homopolymer of the monomer i. Unless otherwise indicated, the Tg values indicated for the first and second blocks in the present patent application are theoretical Tg values.

The difference between the glass transition temperatures of the first and second blocks is generally greater than 10°C, preferably greater than 20°C and better still greater than 30°C.

In the present invention, the expression: "between ... and ..." is intended to denote a range of values for which the limits mentioned are excluded, and "from ... to ..." and "ranging from ... to ..." are intended to denote a range of values for which the limits are included.

The block with a Tg of greater than or equal to 40°C may be a homopolymer or a copolymer.

The block with a Tg of greater than or equal to 40°C may be derived totally or partially from one or more monomers which are such that the homopolymer prepared from these monomers has a glass transition temperature of greater than or equal to 40°C. This block may also be referred to as a "rigid block".

In the case where this block is a homopolymer, it is derived from monomers which are such that the homopolymers prepared from these monomers have glass transition temperatures of greater than or equal to 40°C. This first block may be a homopolymer consisting of only one type of monomer (for which the Tg of the corresponding homopolymer is greater than or equal to 40°C).

In the case where the first block is a copolymer, it may be totally or partially derived from one or more monomers, the nature and concentration of which are chosen such that the Tg of the resulting copolymer is greater than or equal to 40°C.

The copolymer may comprise, for example:

- monomers which are such that the homopolymers prepared from these monomers have Tg values of greater than or equal to 40°C, for example a Tg ranging from 40 to 150°C, preferably greater than or equal to 50°C, for example ranging from 50°C to 120°C and better still greater than or equal to 60°C, for example ranging from 60°C to 120°C, and
- monomers which are such that the homopolymers prepared from these monomers have Tg values of less than 40°C, chosen from monomers with a Tg of between 20°C and 40°C and/or monomers with a Tg of less than or equal to 20°C, for example a Tg ranging from -100°C to 20°C, preferably less than 15°C, especially ranging from -80°C to 15°C and better still less than 10°C, for example ranging from -50°C to 0°C, as described later.

The first monomers whose homopolymers have a glass transition temperature of greater than or equal to 40°C are chosen, preferably, from the following monomers, also known as the main monomers:

- the methacrylates of formula $CH_2 = C(CH_3)\text{-}COOR_1$,
  in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group, preferably a $C_8$ to $C_{12}$

cycloalkyl, such as isobornyl methacrylate,

- the acrylates of formula $CH_2 = CH\text{-}COOR_2$
  in which $R_2$ represents a $C_4$ to $C_{12}$ cycloalkyl group such as an isobornyl group or a tert-butyl group,
- the (meth)acrylamides of formula:

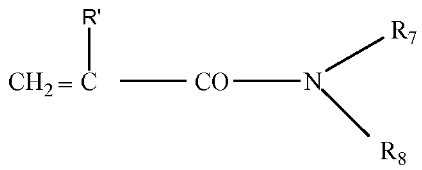

in which $R_7$ and $R_8$, which may be identical or different, each represent a hydrogen atom or a linear or branched C1 to C12 alkyl group such as an n-butyl, t-butyl, isopropyl, isohexyl, isooctyl or isononyl group; or $R_7$ represents H and $R_8$ represents a 1,1-dimethyl-3-oxobutyl group, and
R' denotes H or methyl.

Examples of monomers that may be mentioned include N-butylacrylamide, N-tert-butylacrylamide, N-isopropylacrylamide, N,N-dimethylacrylamide and N,N-dibutylacrylamide, and mixtures thereof.

The first block is advantageously obtained from at least one acrylate monomer of formula $CH_2=CH\text{-}COOR_2$ and from at least one methacrylate monomer of formula $CH_2=C(CH_3)\text{-}COOR_2$ in which $R_2$ represents a $C_4$ to $C_{12}$ cycloalkyl group, preferably a $C_8$ to $C_{12}$ cycloalkyl, such as isobornyl. The monomers and the proportions thereof are preferably chosen such that the glass transition temperature of the first block is greater than or equal to 40°C.

According to one embodiment, the first block is obtained from:

i) at least one acrylate monomer of formula $CH_2=CH\text{-}COOR_2$ in which $R_2$ represents a $C_4$ to $C_{12}$ cycloalkyl group, preferably a $C_8$ to $C_{12}$ cycloalkyl group, such as isobornyl,
ii) and at least one methacrylate monomer of formula $CH_2 = C(CH_3)\text{-}COOR'_2$ in which $R'_2$ represents a $C_4$ to $C_{12}$ cycloalkyl group, preferably a $C_8$ to $C_{12}$ cycloalkyl group, such as isobornyl.

According to one embodiment, the first block is obtained from at least one acrylate monomer of formula $CH_2=CH\text{-}COOR_2$ in which $R_2$ represents a $C_8$ to $C_{12}$ cycloalkyl group, such as isobornyl, and from at least one methacrylate monomer of formula $CH_2=C(CH_3)\text{-}COOR'_2$ in which $R'_2$ represents a $C_8$ to $C_{12}$ cycloalkyl group, such as isobornyl.

Preferably, $R_2$ and $R'_2$ represent, independently or simultaneously, an isobornyl group.

Preferably, the block copolymer comprises from 50% to 80% by weight of isobornyl methacrylate/acrylate, from 10% to 30% by weight of isobutyl acrylate and from 2% to 10% by weight of acrylic acid.

The first block may be obtained exclusively from said acrylate monomer and from said methacrylate monomer.

The acrylate monomer and the methacrylate monomer are preferably in mass proportions of between 30/70 and 70/30, preferably between 40/60 and 60/40 and in particular of the order of 50/50.

The proportion of the first block advantageously ranges from 20% to 90%, better still from 30% to 80% and even better still from 60% to 80% by weight of the polymer.

According to one embodiment, the first block is obtained by polymerization of isobornyl methacrylate and isobornyl acrylate.

Second block with a glass transition temperature of less than 20°C

The second block advantageously has a glass transition temperature Tg of less than or equal to 20°C, for example, a Tg ranging from -100°C to 20°C, preferably less than or equal to 15°C, especially ranging from -80°C to 15°C and better still less than or equal to 10°C, for example ranging from -100°C to 10°C, especially ranging from -30°C to 10°C.

The second block is totally or partially derived from one or more second monomers, which are such that the homopolymer prepared from these monomers has a glass transition temperature of less than or equal to 20°C.

This block may also be referred to as a "flexible block".

The monomer with a Tg of less than or equal to 20°C (known as the second monomer) is preferably chosen from the following monomers:

- the acrylates of formula $CH_2=CHCOOR_3$, $R_3$ representing a linear or branched $C_1$ to $C_{12}$ unsubstituted alkyl group, with the exception of the tert-butyl group, in which one or more heteroatoms chosen from O, N and S are optionally

intercalated,

- the methacrylates of formula $CH_2=C(CH_3)-COOR_4$, $R_4$ representing a linear or branched $C_6$ to $C_{12}$ unsubstituted alkyl group, in which one or more heteroatoms chosen from O, N and S are optionally intercalated,
- the vinyl esters of formula $R_5-CO-O-CH=CH_2$ in which $R_5$ represents a linear or branched $C_4$ to $C_{12}$ alkyl group,
- ethers of vinyl alcohol and of a $C_4$ to $C_{12}$ alcohol,
- N-($C_4$ to $C_{12}$)alkyl acrylamides, such as N-octylacrylamide,
- and mixtures thereof.

The preferred monomers with a Tg of less than or equal to 20°C are isobutyl acrylate, 2-ethylhexyl acrylate or mixtures thereof in all proportions.

Each of the first and second blocks may contain in small proportion at least one constituent monomer of the other block.

Thus, the first block may contain at least one constituent monomer of the second block, and vice versa.

Each of the first and/or second blocks may comprise, in addition to the monomers indicated above, one or more other monomers known as additional monomers, which are different from the main monomers mentioned above.

The nature and amount of this or these additional monomer(s) are chosen such that the block in which they are present has the desired glass transition temperature.

This additional monomer is chosen, for example, from:

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof,
- the methacrylates of formula $CH_2 = C(CH_3)-COOR_6$, in which $R_6$ represents a linear or branched alkyl group containing from 1 to 4 carbon atoms, such as a methyl, ethyl, propyl or isobutyl group, said alkyl group being substituted with one or more substituents chosen from hydroxyl groups (for instance 2-hydroxypropyl methacrylate and 2-hydroxyethyl methacrylate) and halogen atoms (Cl, Br, I or F), such as trifluoroethyl methacrylate,
- the methacrylates of formula $CH_2=C(CH_3)-COOR_9$, $R_9$ representing a linear or branched C6 to C12 alkyl group in which one or more heteroatoms chosen from O, N and S are optionally intercalated, said alkyl group being substituted with one or more substituents chosen from hydroxyl groups and halogen atoms (Cl, Br, I and F),
- the acrylates of formula $CH_2=CHCOOR_{10}$, $R_{10}$ representing a linear or branched $C_1$ to $C_{12}$ alkyl group substituted with one or more substituents chosen from hydroxyl groups and halogen atoms (Cl, Br, I or F), such as 2-hydroxypropyl acrylate and 2-hydroxyethyl acrylate, or $R_{10}$ represents a C1 to C12 alkyl-O-POE (polyoxyethylene) with repetition of the oxyethylene unit 5 to 10 times, for example methoxy-POE, or $R_{10}$ represents a polyoxyethylenated group comprising from 5 to 10 ethylene oxide units.

In particular, the first block may comprise as additional monomer:

- (meth)acrylic acid, preferably acrylic acid,
- tert-butyl acrylate,
- the methacrylates of formula $CH_2 = C(CH_3)-COOR_1$, in which $R_1$ represents a linear or branched unsubstituted alkyl group containing from 1 to 4 carbon atoms, such as a methyl, ethyl, propyl or isobutyl group,
- the (meth)acrylamides of formula:

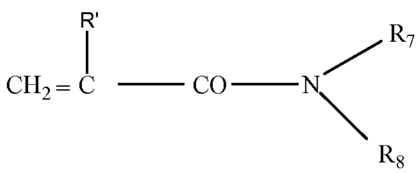

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or R7 represents H and R8 represents a 1,1-dimethyl-3-oxobutyl group, and
R’ denotes H or methyl. Examples of monomers that may be mentioned include N-butylacrylamide, N-tert-butylacrylamide, N-isopropylacrylamide, N,N-dimethylacrylamide and N,N-dibutylacrylamide,

- and mixtures thereof.

The additional monomer may represent 0.5% to 30% by weight relative to the weight of the polymer. According to one embodiment, the polymer of the invention does not contain any additional monomer.

Preferably, the polymer of the invention comprises at least isobornyl acrylate and isobornyl methacrylate monomers in the first block and isobutyl acrylate and acrylic acid monomers in the second block.

Preferably, the polymer comprises at least isobornyl acrylate and isobornyl methacrylate monomers in equivalent weight proportion in the first block and isobutyl acrylate and acrylic acid monomers in the second block.

Preferably, the polymer comprises at least isobornyl acrylate and isobornyl methacrylate monomers in equivalent weight proportion in the first block, and isobutyl acrylate and acrylic acid monomers in the second block, the first block representing 70% by weight of the polymer.

Preferably, the polymer comprises at least isobornyl acrylate and isobornyl methacrylate monomers in equivalent weight proportion in the first block and isobutyl acrylate and acrylic acid monomers in the second block. Preferably, the block with a Tg of greater than 40°C represents 70% by weight of the polymer, and acrylic acid represents 5% by weight of the polymer.

According to one embodiment, the first block does not comprise any additional monomer.

According to a preferred embodiment, the second block comprises acrylic acid as additional monomer. In particular, the second block is advantageously obtained from an acrylic acid monomer and from at least one other monomer with a Tg of less than or equal to 20°C.

The block copolymer may advantageously comprise more than 2% by weight of acrylic acid monomers, and in particular from 2% to 15% by weight, for example from 3% to 15% by weight, in particular from 4% to 15% by weight or even from 4% to 10% by weight of acrylic acid monomers, relative to the total weight of said copolymer.

The constituent monomers of the second block and the proportions thereof are chosen such that the glass transition temperature of the second block is less than or equal to 20°C.

Intermediate segment

The intermediate segment (also known as the intermediate block) connects the first block and the second block of the polymer used according to the present invention. The intermediate segment results from the polymerization:

i) of the first monomer(s), and optionally of the additional monomer(s), which remain available after their polymerization to a maximum degree of conversion of 90% to form the first block,
ii) and of the second monomer(s), and optionally of the additional monomer(s), added to the reaction mixture.

The formation of the second block is initiated when the first monomers no longer react or are no longer incorporated into the polymer chain either because they are all consumed or because their reactivity no longer allows them to be.

Thus, the intermediate segment comprises the first available monomers, resulting from a degree of conversion of these first monomers of less than or equal to 90%, during the introduction of the second monomer(s) during the synthesis of the polymer.

The intermediate segment of the block polymer is a statistical polymer (which may also be referred to as a statistical block). This means that it comprises a statistical distribution of the first monomer(s) and of the second monomer(s) and also of the additional monomer(s) that may be present.

Thus, the intermediate segment is a statistical block, as are the first block and the second block if they are not homopolymers (i.e. if they are both formed from at least two different monomers).

Process for preparing the copolymer

The block ethylenic copolymer according to the invention is prepared by free radical polymerization, according to the techniques that are well known for this type of polymerization.

The free radical polymerization is performed in the presence of an initiator, the nature of which is adapted, in a known manner, as a function of the desired polymerization temperature and of the polymerization solvent. In particular, the initiator may be chosen from initiators bearing a peroxide function, redox couples or other free radical polymerization initiators known to those skilled in the art.

In particular, examples of initiators bearing a peroxide function that may be mentioned include:

- peroxyesters such as tert-butyl peroxyacetate, tert-butyl perbenzoate, tert-butyl peroxy-2-ethylhexanoate (Trigonox 21S from Akzo Nobel) or 2,5-bis(2-ethylhexanoylperoxy)-2,5-dimethylhexane (Trigonox 141 from Akzo Nobel);
- peroxydicarbonates such as diisopropyl peroxydicarbonate;
- peroxy ketones such as methyl ethyl ketone peroxide;
- hydroperoxides such as aqueous hydrogen peroxide solution (H2O2) or tert-butyl hydroperoxide;

- diacyl peroxides such as acetyl peroxide or benzoyl peroxide;
- dialkyl peroxides such as di-tert-butyl peroxide;
- mineral peroxides such as potassium peroxodisulfate (K2S2O8).

As initiator in the form of a redox couple, mention may be made of the potassium thiosulfate + potassium peroxodisulfate couple, for example.

According to a preferred embodiment, the initiator is chosen from organic peroxides comprising from 8 to 30 carbon atoms. Preferably, the initiator used is 2,5-bis(2-ethylhexanoylperoxy)-2,5-dimethylhexane sold under the reference Trigonox® 141 by the company Akzo Nobel.

The block copolymer used according to the invention is prepared by free radical polymerization and not by controlled or living polymerization. In particular, the polymerization of the block ethylenic copolymer is performed in the absence of control agents, and in particular in the absence of control agents conventionally used in living or controlled polymerization processes, such as nitroxides, alkoxyamines, dithioesters, dithiocarbamates, dithiocarbonates or xanthates, trithiocarbonates or copper-based catalysts, for example.

As indicated previously, the intermediate segment is a statistical block, as are the first block and the second block if they are not homopolymers (i.e. if they are both formed from at least two different monomers).

The block copolymer may be prepared by free radical polymerization, and in particular by a process that consists in mixing, in the same reactor, a polymerization solvent, an initiator, at least one monomer with a glass transition temperature of greater than or equal to 40°C, and at least one monomer with a glass transition temperature of less than or equal to 20°C, according to the following sequence:

- some of the polymerization solvent and optionally some of the initiator and of the monomers for the first addition are poured into the reactor, and the mixture is heated to a reaction temperature of between 60°C and 120°C,
- said at least one first monomer with a Tg of greater than or equal to 40°C and optionally some of the initiator are then poured in, in a first addition, and the mixture is left to react for a time T corresponding to a maximum degree of conversion of said monomers of 90%,
- further polymerization initiator and said at least one second monomer with a glass transition temperature of less than or equal to 20°C are then poured into the reactor, in a second addition, and the mixture is left to react for a time T' after which the degree of conversion of said monomers reaches a plateau,
- the reaction mixture is cooled to room temperature.

Preferably, the copolymer may be prepared by free radical polymerization, in particular by a process that consists in mixing, in the same reactor, a polymerization solvent, an initiator, an acrylic acid monomer, at least one monomer with a glass transition temperature of less than or equal to 20°C, at least one acrylate monomer of formula $CH_2$=CH-$COOR_2$ in which R2 represents a $C_4$ to $C_{12}$ cycloalkyl group and at least one methacrylate monomer of formula $CH_2$=C($CH_3$)-COOR'$_2$ in which R'$_2$ represents a $C_4$ to $C_{12}$ cycloalkyl group, according to the following sequence of steps:

- some of the polymerization solvent and optionally some of the initiator and of the monomers for the first addition are poured into the reactor, and the mixture is heated to a reaction temperature of between 60°C and 120°C,
- said at least one acrylate monomer of formula $CH_2$ = CH-$COOR_2$ and said at least one methacrylate monomer of formula $CH_2$ = C($CH_3$)-COOR'$_2$, as monomers with a Tg of greater than or equal to 40°C, and optionally some of the initiator, are then poured in, in a first addition, and the mixture is left to react for a time T corresponding to a maximum degree of conversion of said monomers of 90%,
- further polymerization initiator, the acrylic acid monomer and said at least one monomer with a glass transition temperature of less than or equal to 20°C are then poured into the reactor, in a second addition, and the mixture is left to react for a time T' after which the degree of conversion of said monomers reaches a plateau,
- the reaction mixture is cooled to room temperature.

The term "polymerization solvent" means a solvent or a mixture of solvents.

In particular, as polymerization solvents that may be used, mention may be made of:

- ketones that are liquid at room temperature, such as methyl ethyl ketone, methyl isobutyl ketone, diisobutyl ketone, isophorone, cyclohexanone or acetone;
- propylene glycol ethers that are liquid at room temperature, such as propylene glycol monomethyl ether, propylene glycol monomethyl ether acetate or dipropylene glycol n-butyl monoether;
- short-chain esters (having from 3 to 8 carbon atoms in total), such as ethyl acetate, methyl acetate, propyl acetate, n-butyl acetate or isopentyl acetate;
- ethers that are liquid at room temperature, such as diethyl ether, dimethyl ether or dichlorodiethyl ether;

- alkanes that are liquid at room temperature, such as decane, heptane, dodecane, isododecane, cyclohexane and isohexadecane;
- aromatic cyclic compounds that are liquid at room temperature, such as toluene and xylene; aldehydes that are liquid at room temperature, such as benzaldehyde and acetaldehyde, and mixtures thereof.

Conventionally, the polymerization solvent is a volatile oil with a flash point of less than 80°C. The flash point is measured in particular according to standard ISO 3679.

The polymerization solvent may be chosen especially from ethyl acetate, butyl acetate, alcohols such as isopropanol or ethanol, and aliphatic alkanes such as isododecane, and mixtures thereof. Preferably, the polymerization solvent is a mixture of butyl acetate and isopropanol or isododecane.

According to another embodiment, the copolymer may be prepared by free radical polymerization according to a preparation process that consists in mixing, in the same reactor, a polymerization solvent, an initiator, at least one monomer with a glass transition temperature of less than or equal to 20°C and at least one monomer with a Tg of greater than or equal to 40°C, according to the following sequence of steps:

- some of the polymerization solvent and optionally some of the initiator and of the monomers for the first addition are poured into the reactor, and the mixture is heated to a reaction temperature of between 60°C and 120°C,
- said at least one monomer with a glass transition temperature of less than or equal to 20°C and optionally some of the initiator are then poured in, in a first addition, and the mixture is left to react for a time T corresponding to a maximum degree of conversion of said monomers of 90%,
- further polymerization initiator and said at least one monomer with a Tg of greater than or equal to 40°C are then poured into the reactor, in a second addition, and the mixture is left to react for a time T' after which the degree of conversion of said monomers reaches a plateau, and the reaction mixture is cooled to room temperature.

According to one preferred embodiment, the copolymer may be prepared by free radical polymerization according to a preparation process that consists in mixing, in the same reactor, a polymerization solvent, an initiator, an acrylic acid monomer, at least one monomer with a glass transition temperature of less than or equal to 20°C, at least one monomer with a Tg of greater than or equal to 40°C, and in particular, as monomers with a Tg of greater than or equal to 40°C, at least one acrylate monomer of formula $CH_2=CH-COOR_2$ in which $R_2$ represents a $C_4$ to $C_{12}$ cycloalkyl group and at least one methacrylate monomer of formula $CH_2=C(CH_3)-COOR'_2$ in which $R'_2$ represents a $C_4$ to $C_{12}$ cycloalkyl group, according to the following sequence of steps:

- some of the polymerization solvent and optionally some of the initiator and of the monomers for the first addition are poured into the reactor, and the mixture is heated to a reaction temperature of between 60°C and 120°C,
- the acrylic acid monomer and said at least one monomer with a glass transition temperature of less than or equal to 20°C and optionally some of the initiator are then poured in, in a first addition, and the mixture is left to react for a time T corresponding to a maximum degree of conversion of said monomers of 90%,
- further polymerization initiator, said at least one acrylate monomer of formula $CH_2=CH-COOR_2$ and said at least one methacrylate monomer of formula $CH_2=C(CH_3)-COOR'_2$, as monomer with a Tg of greater than or equal to 40°C, are then poured into the reactor, in a second addition, and the mixture is left to react for a time T' after which the degree of conversion of said monomers reaches a plateau,
- the reaction mixture is cooled to room temperature.

The polymerization temperature is preferably about 90°C. The reaction time after the second addition is preferably between 3 and 6 hours.

Preferably, the block ethylenic copolymer is present in the composition in a solids content ranging from 0.1% to 60%, better still from 0.5% to 50%, better still from 1% to 30% and even better still from 1% to 40% by weight relative to the total weight of the composition.

<u>Distillation of the synthesis solvent</u>

It is possible to perform a step of total or partial removal of said volatile oil or solvent (conventionally isododecane). This is then performed in particular by distillation, optionally under vacuum, and optional addition of non-volatile hydrocarbon-based ester oil comprising at least 16 carbon atoms and having a molar mass of less than 650 g/mol, such as octyldodecyl neopentanoate (especially 2-octyldodecyl neopentanoate).

This step is performed at elevated temperature and optionally under vacuum to distil off a maximum amount of volatile synthesis solvent, and is known to those skilled in the art.

## III. Polyamide silicone block polymer

According to another embodiment variant, a composition according to the invention comprises, as hydrophobic film-forming polymer, at least one polyamide silicone block polymer, also known as a silicone polyamide.
The silicone polyamides are preferably solid at room temperature (25°C) and atmospheric pressure (760 mmHg).
For the purposes of the invention, the term "polymer" means a compound containing at least two repeating units, preferably at least three repeating units and better still
ten repeating units.
The silicone polyamides of the composition of the invention may be polymers of the polyorganosiloxane type, for instance those described in documents US-A-5 874 069, US-A-5 919 441, US-A-6 051 216 and US-A-5 981 680. According to the invention, the silicone polymers may belong to the following two families:

(1) polyorganosiloxanes comprising at least two amide groups, these two groups being located in the polymer chain, and/or
(2) polyorganosiloxanes comprising at least two amide groups, these two groups being located on grafts or branches.

According to a first variant, the silicone polymers are polyorganosiloxanes as defined above in which the units capable of establishing hydrogen interactions are located in the polymer chain.
The silicone polymers may be more particularly polymers comprising at least one unit corresponding to the general formula I:

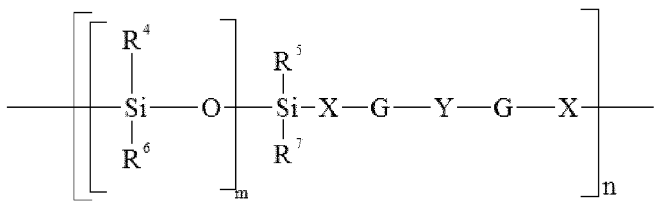

(I)

in which:

- $R^4$, $R^5$, $R^6$ and $R^7$, which may be identical or different, represent a group chosen from:
- saturated or unsaturated, C1 to C40 linear, branched or cyclic hydrocarbon-based groups, which may contain in their chain one or more oxygen, sulfur and/or nitrogen atoms, and which may be partially or totally substituted with fluorine atoms,
- $C_6$ to $C_{10}$ aryl groups, optionally substituted with one or more $C_1$ to $C_4$ alkyl groups,
- polyorganosiloxane chains possibly containing one or more oxygen, sulfur and/or nitrogen atoms,
- the groups X, which may be identical or different, represent a linear or branched $C_1$ to $C_{30}$ alkylenediyl group, which may contain in its chain one or more oxygen and/or nitrogen atoms;
- Y is a saturated or unsaturated C1 to C50 linear or branched alkylene, arylene, cycloalkylene, alkylarylene or arylalkylene divalent group, which may comprise one or more oxygen, sulfur and/or nitrogen atoms, and/or may bear as substituent one of the following atoms or groups of atoms: fluorine, hydroxyl, $C_3$ to $C_8$ cycloalkyl, $C_1$ to $C_{40}$ alkyl, $C_5$ to $C_{10}$ aryl, phenyl optionally substituted with 1 to 3 $C_1$ to $C_3$ alkyl, $C_1$ to $C_3$ hydroxyalkyl and $C_1$ to $C_6$ aminoalkyl groups, or
- Y represents a group corresponding to the formula:

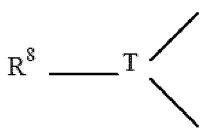

in which:

- T represents a linear or branched, saturated or unsaturated, $C_3$ to $C_{24}$ trivalent or tetravalent hydrocarbon-based group optionally substituted with a polyorganosiloxane chain, and possibly containing one or more atoms chosen

from O, N and S, or T represents a trivalent atom chosen from N, P and Al, and

- $R^8$ represents a linear or branched $C_1$-$C_{50}$ alkyl group or a polyorganosiloxane chain, possibly comprising one or more ester, amide, urethane, thiocarbamate, urea, thiourea and/or sulfonamide groups, which may possibly be linked to another chain of the polymer;
- the groups G, which may be identical or different, represent divalent groups chosen from:

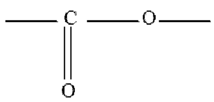 ; 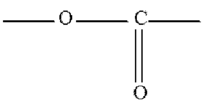 ; 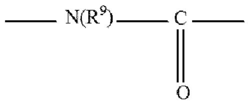 ;

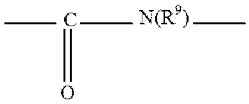 ; 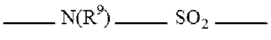 ; 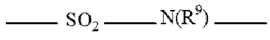 ;

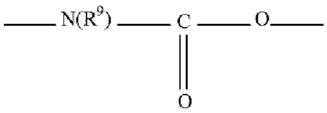 ; 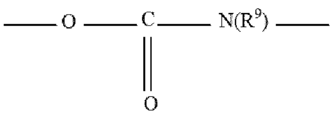 ; 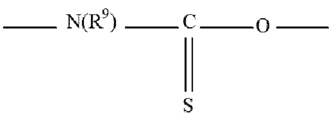 ;

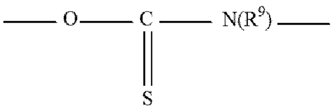 ; 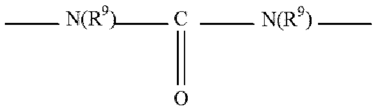 ,

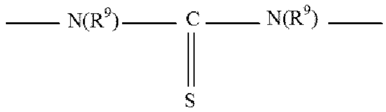 ,

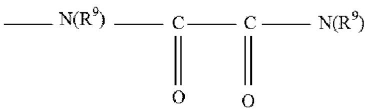 ; 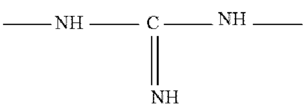 ; et

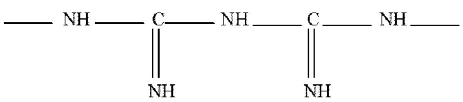

in which $R^9$ represents a hydrogen atom or a linear or branched $C_1$ to $C_{20}$ alkyl group, on condition that at least 50% of the groups $R^9$ of the polymer represent a hydrogen atom and that at least two of the groups G of the polymer are a group other than:

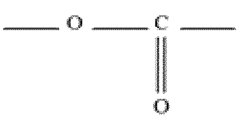 and 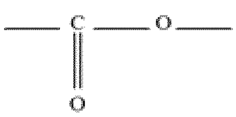 ;

n is an integer ranging from 2 to 500 and preferably from 2 to 200, and m is an integer ranging from 1 to 1000, preferably from 1 to 700 and better still from 6 to 200.

According to the invention, 80% of the groups $R^4$, $R^5$, $R^6$ and $R^7$ of the polymer are preferably chosen from methyl, ethyl, phenyl and 3,3,3-trifluoropropyl groups.

According to the invention, Y may represent various divalent groups, furthermore optionally comprising one or two free valencies to establish bonds with other units of the polymer or copolymer. Preferably, Y represents a group chosen from:

- linear $C_1$ to $C_{20}$ and preferably $C_1$ to $C_{10}$ alkylene groups,
- $C_{30}$ to $C_{56}$ branched alkylene groups possibly comprising rings and unconjugated unsaturations,
- $C_5$-$C_6$ cycloalkylene groups,
- phenylene groups optionally substituted with one or more $C_1$ to $C_{40}$ alkyl groups,
- $C_1$ to $C_{20}$ alkylene groups comprising from 1 to 5 amide groups,
- $C_1$ to $C_{20}$ alkylene groups comprising one or more substituents chosen from hydroxyl, $C_3$ to $C_8$ cycloalkane, $C_1$ to $C_3$ hydroxyalkyl and $C_1$ to $C_6$ alkylamine groups,
- polyorganosiloxane chains of formula:

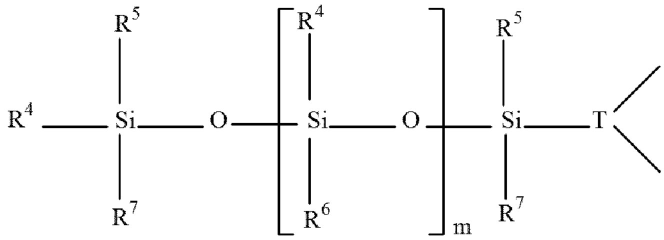

in which $R^4$, $R^5$, $R^6$, $R^7$, T and m are as defined above, and - polyorganosiloxane chains of formula:

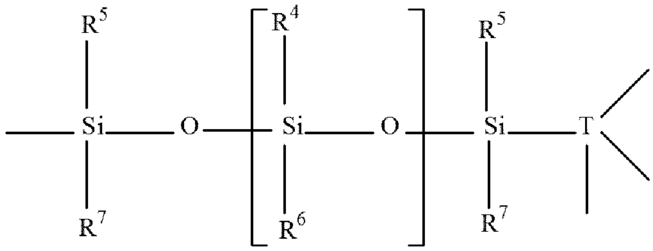

According to the second variant, the polyorganosiloxanes may be polymers comprising at least one unit corresponding to formula (II):

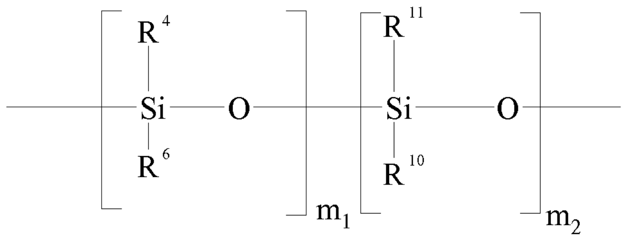

(II)

in which:

- $R^4$ and $R^6$, which may be identical or different, are as defined above for formula (I),
- $R^{10}$ represents a group as defined above for $R^4$ and $R^6$, or represents the group of formula -X-G-$R^{12}$ in which X and G are as defined above for formula (I) and $R^{12}$ represents a hydrogen atom or a linear, branched or cyclic, saturated or unsaturated $C_1$ to $C_{50}$ hydrocarbon-based group optionally comprising in its chain one or more atoms chosen from O, S and N, optionally substituted with one or more fluorine atoms and/or one or more hydroxyl groups, or a phenyl group optionally substituted with one or more $C_1$ to $C_4$ alkyl groups,
- $R^{11}$ represents a group of formula -X-G-$R^{12}$ in which X, G and $R^{12}$ are as defined above,
- $m_1$ is an integer ranging from 1 to 998, and
- $m_2$ is an integer ranging from 2 to 500.

According to the invention, the silicone polymer used as structuring agent may be a homopolymer, that is to say a polymer comprising several identical units, in particular units of formula (I) or of formula (II).

According to the invention, it is also possible to use a silicone polymer formed from a copolymer comprising several different units of formula (I), that is to say a polymer in which at least one of the groups $R^4$, $R^5$, $R^6$, $R^7$, X, G, Y, m and n is different in one of the units. The copolymer may also be formed from several units of formula (II), in which at least one of the groups $R^4$, $R^6$, $R^{10}$, $R^{11}$, $m_1$ and $m_2$ is different in at least one of the units.

It is also possible to use a polymer comprising at least one unit of formula (I) and at least one unit of formula (II), the units of formula (I) and the units of formula (II) possibly being identical to or different than each other.

According to one variant of the invention, it is also possible to use a polymer furthermore comprising at least one hydrocarbon-based unit comprising two groups capable of establishing hydrogen interactions, chosen from ester, amide, sulfonamide, carbamate, thiocarbamate, urea, urethane, thiourea, oxamido, guanidino and biguanidino groups, and combinations thereof.

These copolymers may be block polymers or grafted polymers.

According to a first advantageous embodiment of the invention, the groups capable of establishing hydrogen interactions are amide groups of formulae-C(O)NH- and -HN-C(O)-.

In this case, the structuring agent may be a polymer comprising at least one unit of formula (III) or (IV):

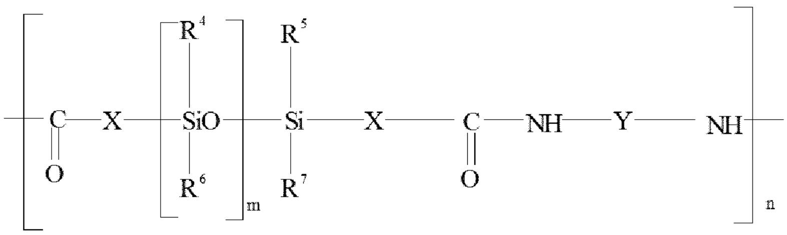

(III)

or

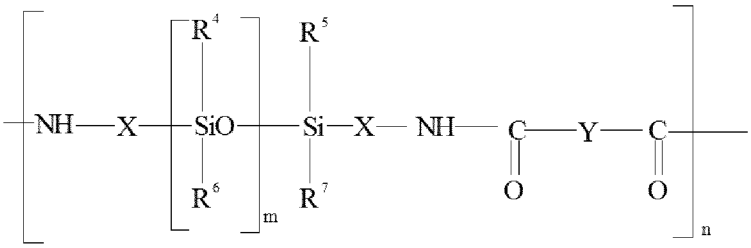

(IV)

in which $R^4$, $R^5$, $R^6$, $R^7$, X, Y, m and n are as defined above.

Such a unit may be obtained:

either by a condensation reaction between a silicone containing α,ω-carboxylic acid ends and one or more diamines, according to the following reaction scheme:

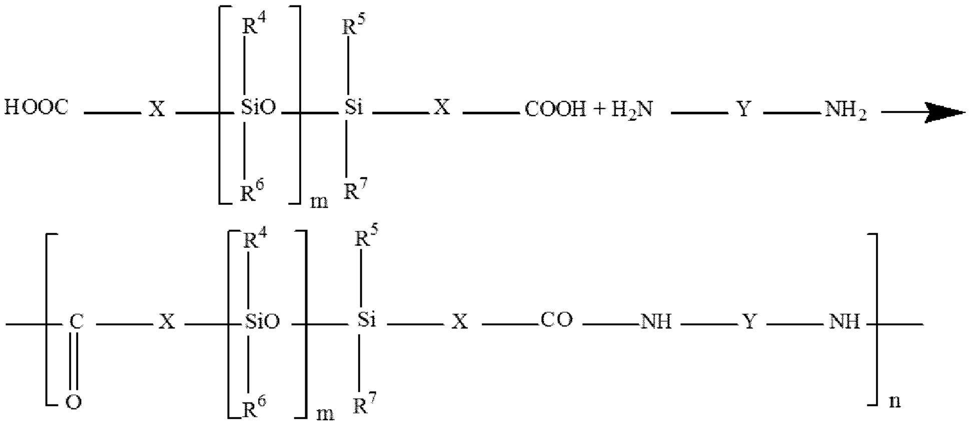

or by reaction of two molecules of α-unsaturated carboxylic acid with a diamine according to the following reaction scheme:

$$CH_2{=}CH\text{-}X^1\text{-}COOH + H_2N\text{-}Y\text{-}NH_2 \rightarrow CH_2\text{-}CH\text{-}X^1\text{-}CO\text{-}NH\text{-}Y\text{-}NH\text{-}CO\text{-}X^1\text{-}CH\text{-}CH_2$$

followed by the addition of a siloxane to the ethylenic unsaturations, according to the following scheme:

$$CH_2{=}CH\text{-}X^1\text{-}CO\text{-}NH\text{-}Y\text{-}NH\text{-}CO\text{-}X^1\text{-}CH{=}CH_2$$

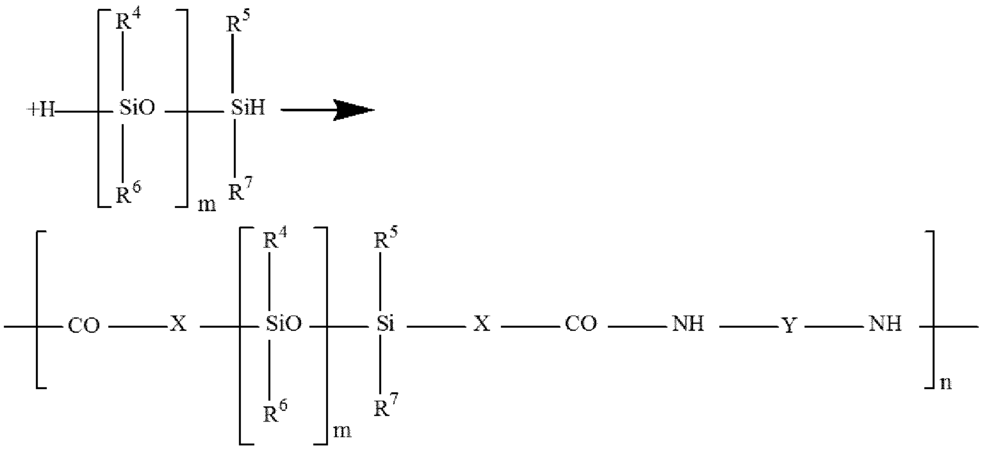

in which $X^1$-$(CH_2)_2$- corresponds to X defined above and Y, $R^4$, $R^5$, $R^6$, $R^7$ and m are as defined above;
or by reaction of a silicone containing α,ω-$NH_2$ ends and a diacid of formula HOOC-Y-COOH according to the following reaction scheme:

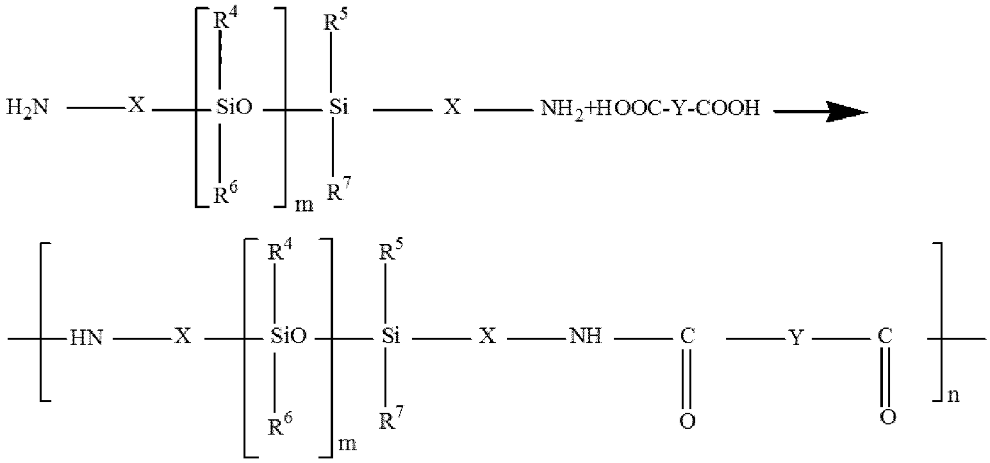

In these polyamides of formula (III) or (IV), m ranges from 1 to 700, in particular from 15 to 500 and especially from 50 to 200, and n ranges in particular from 1 to 500, preferably from 1 to 100 and better still from 4 to 25.
X is preferably a linear or branched alkylene chain containing from 1 to 30 carbon atoms, in particular 1 to 20 carbon atoms, especially from 5 to 15 carbon atoms and more particularly 10 carbon atoms, and Y is preferably an alkylene chain that is linear or branched, or which may comprise rings and/or unsaturations, containing from 1 to 40 carbon atoms, in particular 1 to 20 carbon atoms and better still from 2 to 6 carbon atoms, in particular 6 carbon atoms.
In formulae (III) and (IV), the alkylene group representing X or Y can optionally contain in its alkylene part at least one of the following components:

- one to five amide, urea, urethane or carbamate groups,
- a $C_5$ or $C_6$ cycloalkyl group, and
- a phenylene group optionally substituted with 1 to 3 identical or different $C_1$ to $C_3$ alkyl groups.

In formulae (III) and (IV), the alkylene groups may also be substituted with at least one component chosen from the group consisting of:

- a hydroxyl group,
- a $C_3$ to $C_8$ cycloalkyl group,
- one to three $C_1$ to $C_{40}$ alkyl groups,
- a phenyl group optionally substituted with one to three $C_1$ to $C_3$ alkyl groups,
- a $C_1$ to $C_3$ hydroxyalkyl group, and
- a $C_1$ to $C_6$ aminoalkyl group.

In these formulae (III) and (IV), Y may also represent:

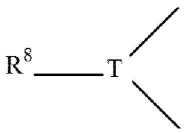

in which $R^8$ represents a polyorganosiloxane chain and T represents a group of formula:

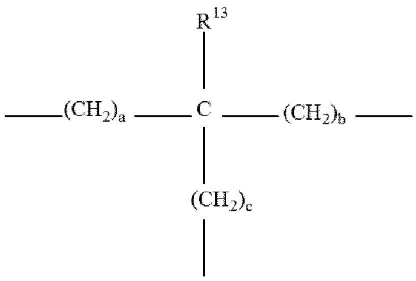

ou

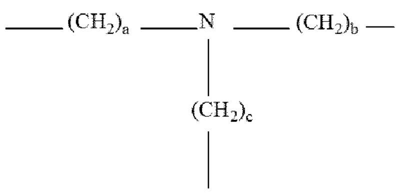

in which a, b and c are, independently, integers ranging from 1 to 10, and $R^{13}$ is a hydrogen atom or a group such as those defined for $R^4$, $R^5$, $R^6$ and $R^7$.

In formulae (III) and (IV), $R^4$, $R^5$, $R^6$ and $R^7$ preferably represent, independently, a linear or branched $C_1$ to $C_{40}$ alkyl group, preferably a $CH_3$, $C_2H_5$, n-$C_3H_7$ or isopropyl group, a polyorganosiloxane chain or a phenyl group optionally substituted with one to three methyl or ethyl groups.

As has been seen previously, the polymer may comprise identical or different units of formula (III) or (IV).

Thus, the polymer may be a polyamide containing several units of formula (III) or (IV) of different lengths, i.e. a polyamide corresponding to formula (V):

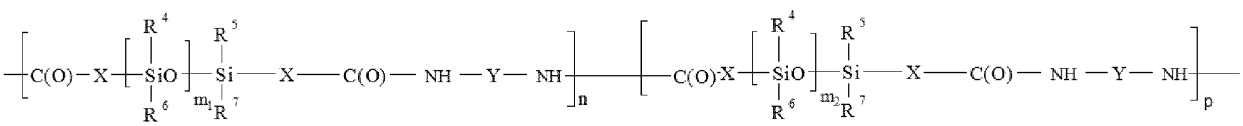

(V)

in which X, Y, n and $R^4$ to $R^7$ have the meanings given above, $m_1$ and $m_2$, which are different, are chosen in the range from 1 to 1000, and p is an integer ranging from 2 to 300.

In this formula, the units may be structured to form either a block copolymer, or a random copolymer or an alternating copolymer. In this copolymer, the units may be not only of different lengths, but also of different chemical structures, for example containing different groups Y. In this case, the polymer may correspond to formula VI:

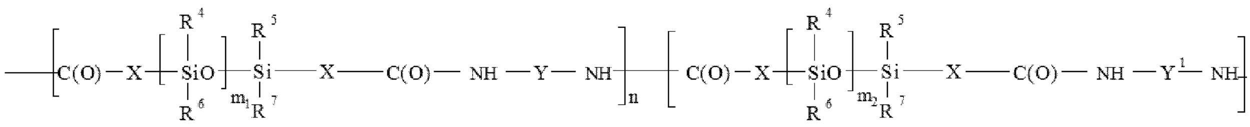

(VI)

in which $R^4$ to $R^7$, X, Y, $m_1$, $m_2$, n and p have the meanings given above and Y1 is different than Y but chosen from the groups defined for Y.

As previously, the various units may be structured to form either a block copolymer, or a random copolymer or an alternating copolymer.

In this first embodiment of the invention, the structuring agent may also consist of a grafted copolymer. Thus, the polyamide containing silicone units may be grafted and optionally crosslinked with silicone chains containing amide groups. Such polymers may be synthesized with trifunctional amines.

In this case, the polymer may comprise at least one unit of formula (VII):

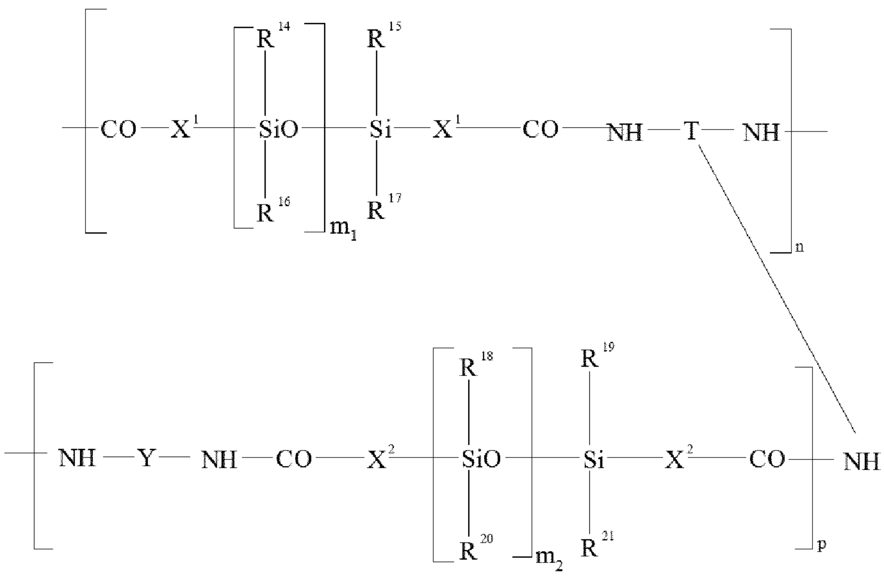

(VII)

in which $X^1$ and $X^2$, which are identical or different, have the meaning given for X in formula (I), n is as defined in formula (I), Y and T are as defined in formula (I), $R^{14}$ to $R^{21}$ are groups chosen from the same group as $R^4$ to $R^7$, $m_1$ and $m_2$ are numbers in the range from 1 to 1000, and p is an integer ranging from 2 to 500.

In formula (VII), it is preferred that:

- p is in the range from 1 to 25 and better still from 1 to 7,
- $R^{14}$ to $R^{21}$ are methyl groups,
- T corresponds to one of the following formulae:

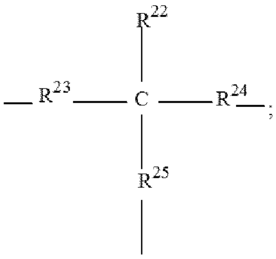 ; 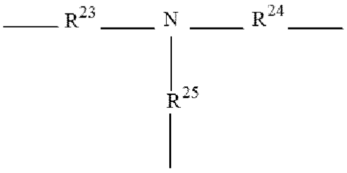 ; 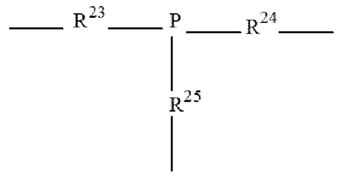

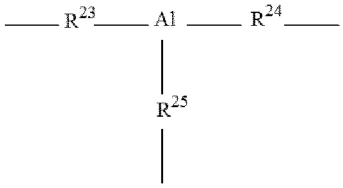

in which $R^{22}$ is a hydrogen atom or a group chosen from the groups defined for $R^4$ to $R^7$, and $R^{23}$, $R^{24}$ and $R^{25}$ are, independently, linear or branched alkylene groups, and more preferably correspond to the formula:

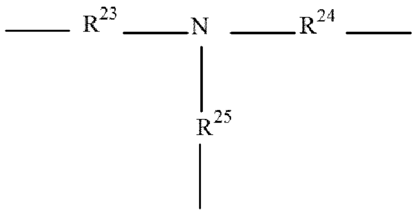

in particular with $R^{23}$, $R^{24}$ and $R^{25}$ representing $-CH_2-CH_2-$, $m_1$ and $m_2$ are from 15 to 500 and better still from 15 to 45, $X^1$ and $X^2$ represent $-(CH_2)_{10}-$, and Y represents $-CH_2-$.

These polyamides containing a grafted silicone unit of formula (VII) may be copolymerized with polyamide-silicones of formula (II) to form block copolymers, alternating copolymers or random copolymers. The weight percentage of grafted silicone units (VII) in the copolymer may range from 0.5% to 30% by weight.

According to the invention, as has been seen previously, the siloxane units may be in the main chain or backbone of the polymer, but they may also be present in grafted or pendent chains. In the main chain, the siloxane units may be in the form of segments as described above. In the pendent or grafted chains, the siloxane units may appear individually or in segments.

According to one embodiment variant of the invention, a copolymer of silicone polyamide and of hydrocarbon-based polyamide, or a copolymer comprising units of formula (III) or (IV) and hydrocarbon-based polyamide units, may be used. In this case, the polyamide-silicone units may be located at the ends of the hydrocarbon-based polyamide.

According to a preferred embodiment, the silicone polyamide comprises units of formula III, preferably in which the groups $R^4$, $R^5$, $R^6$ and $R^7$ represent methyl groups, one from among X and Y represents an alkylene group of 6 carbon atoms and the other represents an alkylene group of 11 carbon atoms, n representing the degree of polymerization, DP, of the polymer. As examples of such silicone polyamides, mention may be made of the compounds sold by the company Dow Corning under the names DC 2-8179® (DP 100®) and DC 2-8178® (DP 15®), the INCI name of which is Nylon-611/dimethicone copolymer.

Advantageously, the composition according to the invention comprises at least one polydimethylsiloxane block polymer of general formula (I) with an m value of about 15.

More preferably, the composition according to the invention comprises at least one polymer comprising at least one unit of formula (III) in which m ranges from 5 to 100, in particular from 10 to 75 and even more particularly is about 15; even more preferably, $R^4$, $R^5$, $R^6$ and $R^7$ independently represent a linear or branched $C_1$ to $C_{40}$ alkyl group, preferably a group $CH_3$, C2$H_5$, n-$C_3H_7$ or isopropyl in formula (III). According to a preferred mode, use is made of the polyamide silicone polymer sold by the company Dow Corning under the name DC 2-8179® (DP 100®).

As an example of silicone polymers that may be used, mention may be made of one of the silicone polyamides obtained in accordance with Examples 1 to 3 of document

US-A-5 981 680.

## IV. Vinyl polymer comprising at least one carbosiloxane dendrimer-based unit

According to one particular embodiment, a composition used according to the invention may comprise, as hydrophobic film-forming polymer, at least one vinyl polymer comprising at least one carbosiloxane dendrimer-based unit.

The vinyl polymer used according to the invention especially has a backbone and at least one side chain, which comprises a carbosiloxane dendrimer-based unit having a carbosiloxane dendrimer structure.

Vinyl polymers comprising at least one carbosiloxane dendrimer unit as described in patent applications WO 03/045 337 and EP 963 751 by the company Dow Corning may be used in particular.

In the context of the present invention, the term "carbosiloxane dendrimer structure" represents a molecular structure containing branched groups of high molecular masses, said structure having high regularity in the radial direction starting from the bond to the backbone. Such carbosiloxane dendrimer structures are described in the form of a highly branched siloxane-silylalkylene copolymer in laid-open Japanese patent application Kokai 9-171 154.

A vinyl polymer according to the invention may contain carbosiloxane dendrimer-based units that may be represented by the following general formula:

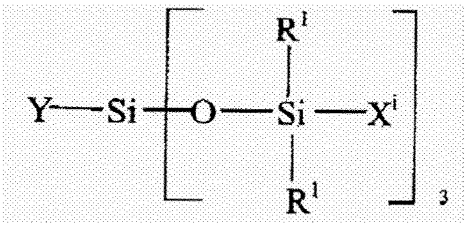

in which $R^1$ represents an aryl group or an alkyl group containing from 1 to 10 carbon atoms, and $X^i$ represents a silylalkyl group which, when i = 1, is represented by the formula:

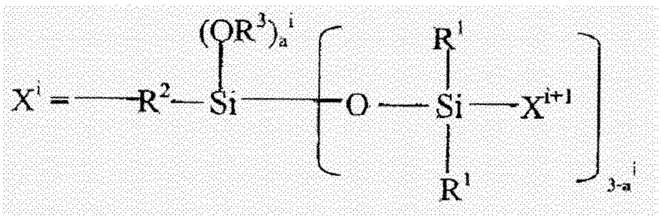

in which $R^1$ is the same as defined above, $R^2$ represents an alkylene group containing from 2 to 10 carbon atoms, $R^3$ represents an alkyl group containing from 1 to 10 carbon atoms, $X^{i+1}$ represents a hydrogen atom, an alkyl group containing from 1 to 10 carbon atoms, an aryl group or the silylalkyl group defined above with i = i + 1; i is an integer from 1 to 10 which represents the generation of said silylalkyl group, and ai is an integer from 0 to 3; Y represents a radical-polymerizable organic group chosen from:

- organic groups containing a methacrylic group or an acrylic group and that are represented by the formulae:

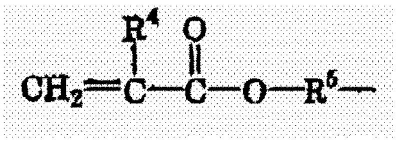

and

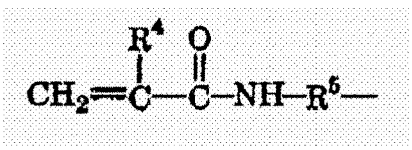

in which $R^4$ represents a hydrogen atom or an alkyl group, $R^5$ represents an alkylene group containing from 1 to 10

carbon atoms, such as a methylene group, an ethylene group, a propylene group or a butylene group, the methylene group and the propylene group being preferred; and

- organic groups containing a styryl group and that are represented by the formula:

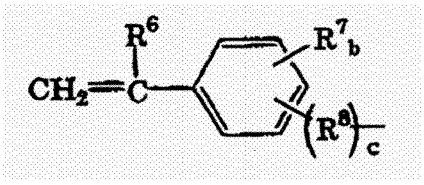

in which $R^6$ represents a hydrogen atom or an alkyl group, $R^7$ represents an alkyl group containing from 1 to 10 carbon atoms, such as a methyl group, an ethyl group, a propyl group or a butyl group, the methyl group being preferred, $R^8$ represents an alkylene group containing from 1 to 10 carbon atoms, such as a methylene group, an ethylene group, a propylene group or a butylene group, the ethylene group being preferred, b is an integer from 0 to 4, and c is 0 or 1 such that if c is 0, $-(R^8)c-$ represents a bond.

According to one embodiment, $R^1$ may represent an aryl group or an alkyl group containing from 1 to 10 carbon atoms. The alkyl group may preferably be represented by a methyl group, an ethyl group, a propyl group, a butyl group, a pentyl group, an isopropyl group, an isobutyl group, a cyclopentyl group or a cyclohexyl group. The aryl group can preferably be represented by a phenyl group and a naphthyl group. The methyl and phenyl groups are more particularly preferred, and the methyl group is most preferred.

A vinyl polymer containing at least one carbosiloxane dendrimer-based unit has a molecular side chain containing a carbosiloxane dendrimer structure, and may be the product of polymerization of:

from 0 to 99.9 parts by weight of a vinyl monomer; and
from 100 to 0.1 part by weight of a carbosiloxane dendrimer containing a radical-polymerizable organic group, represented by the general formula:

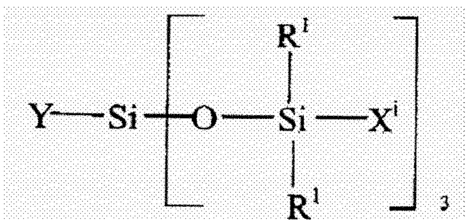

in which Y represents a radical-polymerizable organic group, $R^1$ represents an aryl group or an alkyl group containing from 1 to 10 carbon atoms, and $X^i$ represents a silylalkyl group which, when i = 1, is represented by the formula:

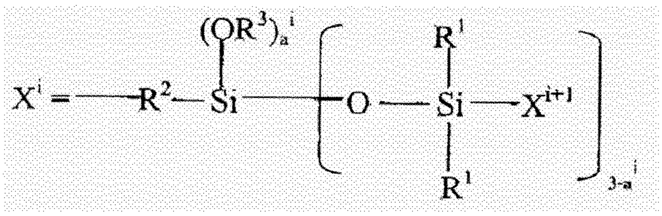

in which $R^1$ is the same as defined above, $R^2$ represents an alkylene group containing from 2 to 10 carbon atoms, $R^3$ represents an alkyl group containing from 1 to 10 carbon atoms, $X^{i+1}$ represents a hydrogen atom, an alkyl group containing from 1 to 10 carbon atoms, an aryl group, or the silylalkyl group defined above with i = i + 1; i is an integer from 1 to 10 that represents the generation of said silylalkyl group, and ai is an integer from 0 to 3;
in which said radical-polymerizable organic group contained in the component (B) is chosen from:

- organic groups containing a methacrylic group or an acrylic group and that are represented by the formulae:

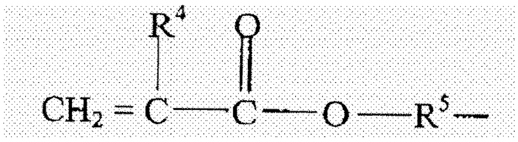

and

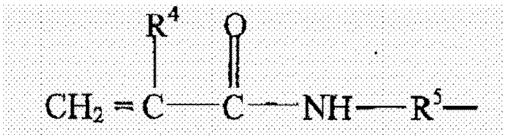

in which R4 represents a hydrogen atom or an alkyl group, R5 represents an alkylene group containing from 1 to 10 carbon atoms; and

- organic groups containing a styryl group and that are represented by the formula:

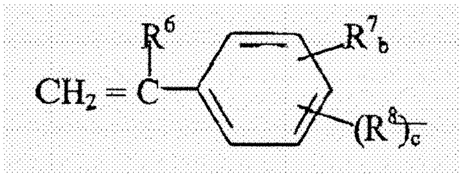

in which R6 represents a hydrogen atom or an alkyl group, R7 represents an alkyl group containing from 1 to 10 carbon atoms, R8 represents an alkylene group containing from 1 to 10 carbon atoms, b is an integer from 0 to 4, and c is 0 or 1, such that if c is 0, -(R8)c- represents a bond.

The monomer of vinyl type that is component (A) in the vinyl polymer is a monomer of vinyl type that contains a radical-polymerizable vinyl group.

There is no particular limitation as regards such a monomer.

The following are examples of this monomer of vinyl type: methyl methacrylate, ethyl methacrylate, n-propyl methacrylate, isopropyl methacrylate or a methacrylate of an analogous lower alkyl; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate or a higher-analogue methacrylate; vinyl acetate, vinyl propionate or a vinyl ester of an analogous lower fatty acid; vinyl caproate, vinyl 2-ethylhexoate, vinyl laurate, vinyl stearate or an ester of a higher fatty acid analogue; styrene, vinyltoluene, benzyl methacrylate, phenoxyethyl methacrylate, vinylpyrrolidone or similar vinylaromatic monomers; methacrylamide, N-methylolmethacrylamide, N-methoxymethylmethacrylamide, isobutoxymethoxymethacrylamide, N,N-dimethylmethacrylamide or similar monomers of vinyl type containing amide groups; hydroxyethyl methacrylate, hydroxypropyl alcohol methacrylate or similar monomers of vinyl type containing hydroxyl groups; acrylic acid, methacrylic acid, itaconic acid, crotonic acid, fumaric acid, maleic acid or similar monomers of vinyl type containing a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl vinyl ether, 2-ethylhexyl vinyl ether or a similar monomer of vinyl type with ether bonds; methacryloxypropyltrimethoxysilane, polydimethylsiloxane containing a methacrylic group on one of its molecular ends, polydimethylsiloxane containing a styryl group on one of its molecular ends, or a similar silicone compound containing unsaturated groups; butadiene; vinyl chloride; vinylidene chloride; methacrylonitrile; dibutyl fumarate; anhydrous maleic acid; anhydrous succinic acid; methacryl glycidyl ether; an organic salt of an amine, an ammonium salt, and an alkali metal salt of methacrylic acid, of itaconic acid, of crotonic acid, of maleic acid or of fumaric acid; a radical-polymerizable unsaturated monomer containing a sulfonic acid group such as a styrenesulfonic acid group; a quaternary ammonium salt derived from methacrylic acid, such as 2-hydroxy-3-methacryloxypropyltrimethylammonium chloride; and a methacrylic acid ester of an alcohol containing a tertiary amine group, such as a methacrylic acid ester of diethylamine.

Multifunctional monomers of vinyl type can also be used.

The following are examples of such compounds: trimethylolpropane trimethacrylate, pentaerythrityl trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, trimethylolpropane trioxyethylmethacrylate, tris(2-hydroxyethyl)isocyanurate dimethacrylate, tris(2-hydroxyethyl)isocyanurate trimethacrylate, polydimethylsiloxane capped with styryl groups possessing divinylbenzene groups on both ends, or analogous silicone compounds containing unsaturated groups.

A carbosiloxane dendrimer, which is the component (B), may be represented by the following formula:

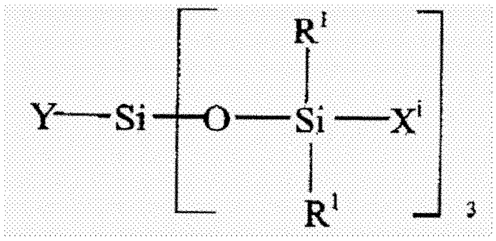

in which Y represents a radical-polymerizable organic group as defined previously.

The following are preferred examples of radical-polymerizable organic groups Y: an aryloxymethyl group, a 3-acryloxypropyl group, a methacryloxymethyl group, a 3-methacryloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group and a 5-hexenyl group.

R’ is as defined previously.

$X^i$ represents a silylalkyl group that is represented by the following formula, when i is equal to 1:

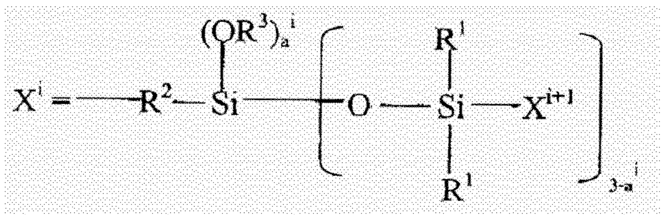

in which:

$R^1$ is as defined above;

$R^2$ represents an alkylene group containing from 2 to 10 carbon atoms, such as an ethylene group, a propylene group, a butylene group, a hexylene group or a similar linear alkylene group; a methylmethylene group, a methylethylene group, a 1-methylpentylene group, a 1,4-dimethylbutylene group or a similar branched alkylene group.

The ethylene, methylethylene, hexylene, 1-methylpentylene and 1,4-dimethylbutylene groups are most preferred. $R^3$ represents an alkyl group containing from 1 to 10 carbon atoms, such as methyl, ethyl, propyl, butyl and isopropyl groups.

$X^{i+1}$ represents a hydrogen atom, an alkyl group containing from 1 to 10 carbon atoms, an aryl group or the silylalkyl group with i = i + 1.

$a^i$ is an integer from 0 to 3, and i is an integer from 1 to 10 that indicates the generation number, which represents the number of repetitions of the silylalkyl group.

For example, when the generation number is equal to 1, the carbosiloxane dendrimer may be represented by the first general formula shown below, in which Y, $R^1$, $R^2$ and $R^3$ are the same as defined above, $R^{12}$ represents a hydrogen atom or is identical to $R^1$; $a^1$ is identical to ai. Preferably, the total average number of $OR^3$ groups in a molecule is within the range from 0 to 7.

When the generation number is equal to 2, the carbosiloxane dendrimer may be represented by the second general formula shown below, in which Y, $R^1$, $R^2$, $R^3$ and $R^{12}$ are the same as defined above; a1 and a2 represent the ai of the indicated generation. Preferably, the total mean number of groups OR3 in a molecule is within the range from 0 to 25.

When the generation number is equal to 3, the carbosiloxane dendrimer is represented by the third general formula shown below, in which Y, $R^1$, $R^2$, $R^3$ and $R^{12}$ are the same as defined above; $a^1$, $a^2$ and $a^3$ represent the ai of the indicated generation. Preferably, the total average number of $OR^3$ groups in a molecule is within the range from 0 to 79.

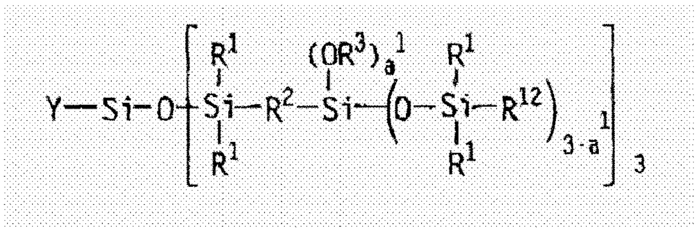

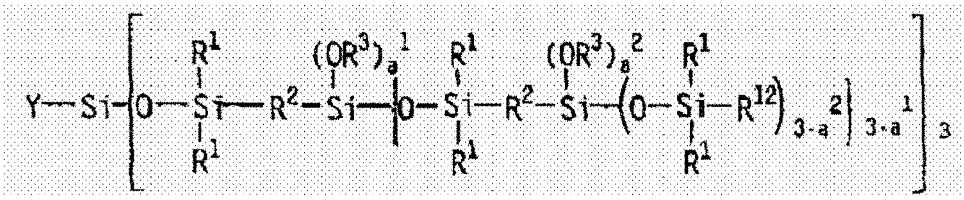

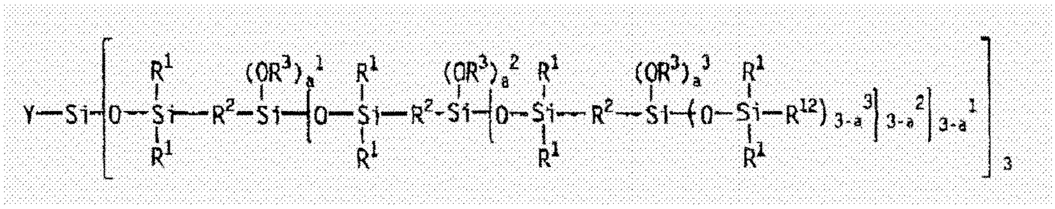

A carbosiloxane dendrimer that contains a radical-polymerizable organic group may be represented by the following mean structural formulae:

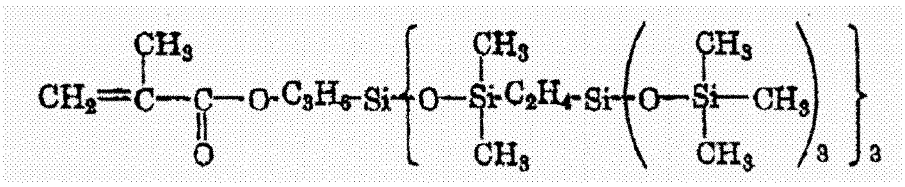

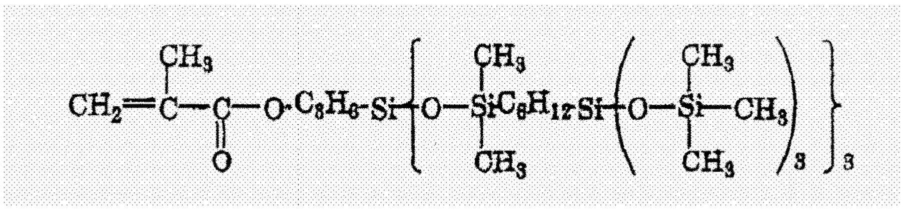

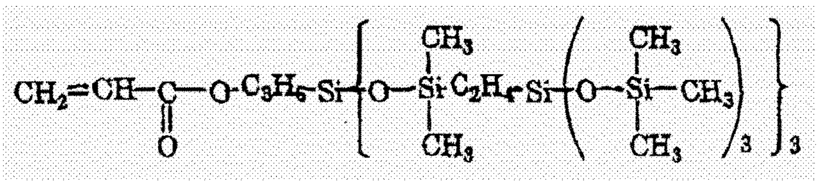

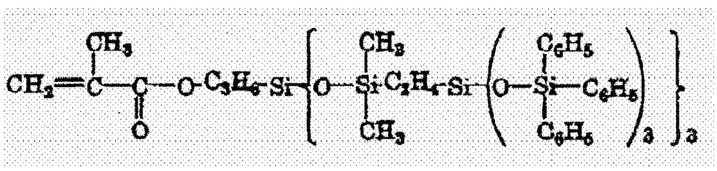

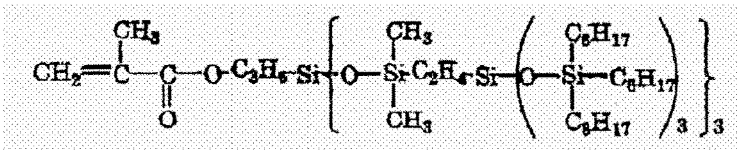

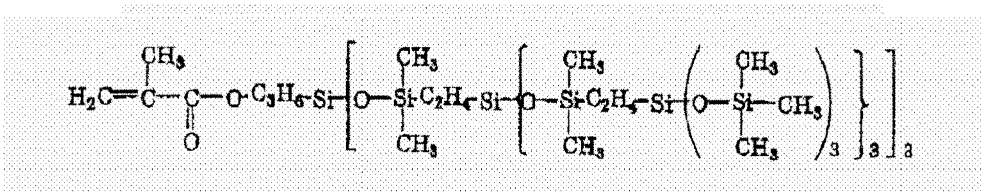

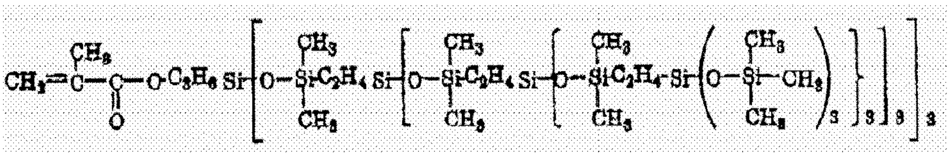

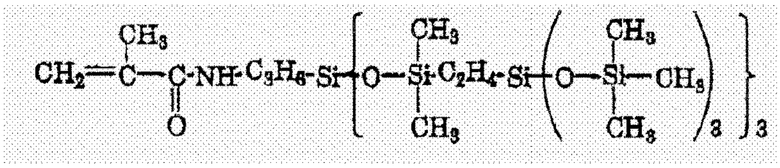

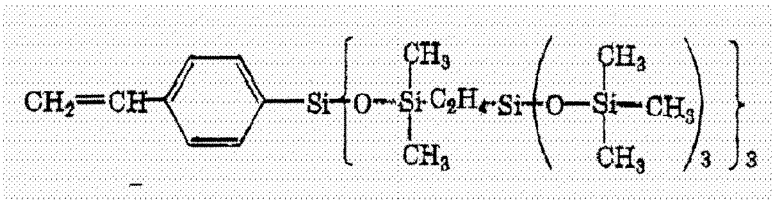

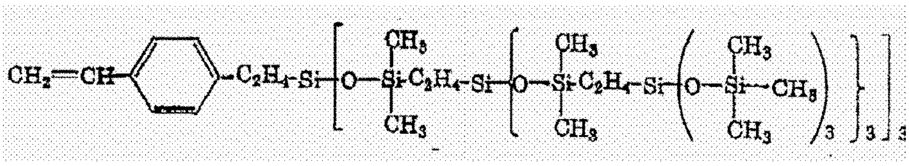

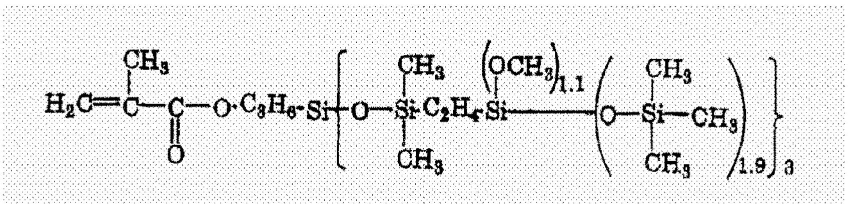

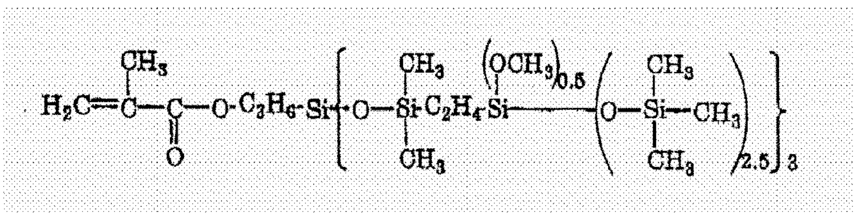

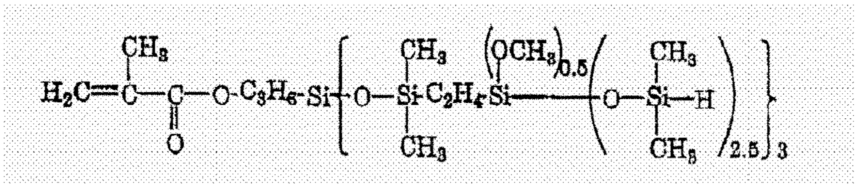

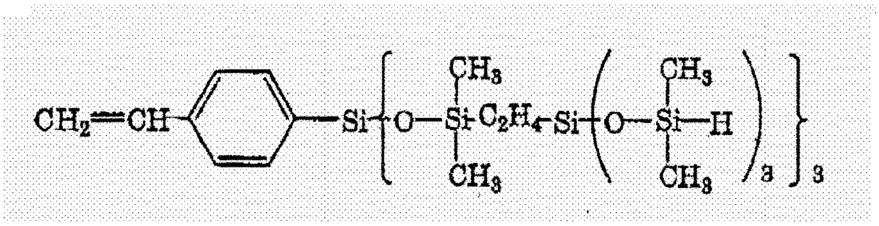

The carbosiloxane dendrimer may be manufactured according to the process for manufacturing a branched silalkylene siloxane described in Japanese patent application Hei 9-171 154.

For example, it may be produced by subjecting an organosilicon compound containing a hydrogen atom linked to a silicon atom, represented by the following general formula:

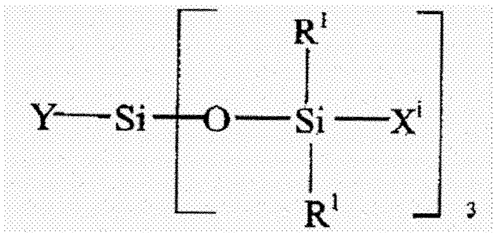

and an organosilicon compound containing an alkenyl group, to a hydrosilylation reaction.

In the above formula, the organosilicon compound may be represented by 3-methacryloxypropyltris(dimethylsiloxy)silane, 3-acryloxypropyltris- may be chosen from the polymers such that the carbosiloxane dendrimer-based unit is (dimethylsiloxy)silane, and 4-vinylphenyltris(dimethylsiloxy)silane. The organosilicon compound that contains an alkenyl group may be represented by vinyltris(trimethylsiloxy)silane, vinyltris(dimethylphenylsiloxy)silane, and 5-hexenyltris(trimethylsiloxy)silane.

The hydrosilylation reaction is performed in the presence of a chloroplatinic acid, a complex of vinylsiloxane and of platinum, or a similar transition metal catalyst.

A vinyl polymer containing at least one carbosiloxane dendrimer-based unit may be chosen from polymers such that the carbosiloxane dendrimer-based unit is a carbosiloxane dendritic structure represented by formula (I):

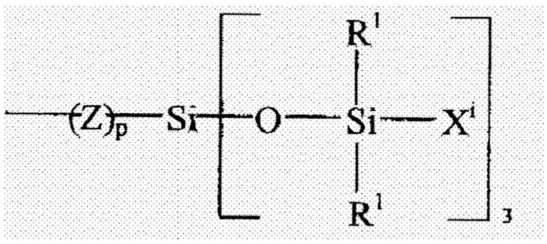

in which Z is a divalent organic group, p is 0 or 1, $R^1$ is an aryl or alkyl group of 1 to 10 carbon atoms and $X^i$ is a silylalkyl group represented by formula (II):

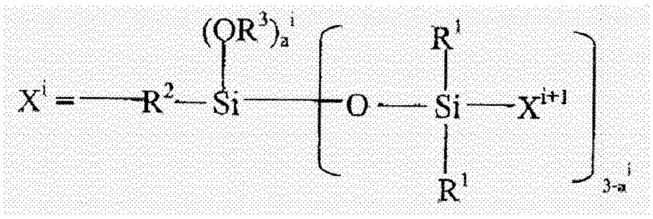

in which $R^1$ is as defined above, $R^2$ is an alkylene group containing from 1 to 10 carbon atoms, $R^3$ is an alkyl group containing from 1 to 10 carbon atoms and $X^{i+1}$ is a group chosen from the group comprising hydrogen atoms, aryl groups and alkyl groups containing up to 10 carbon atoms, and silylalkyl groups $X^i$ in which the power "i" is an integer from 1 to 10 indicating the generation of the starting silylalkyl group in each carbosiloxane dendritic structure with a value of 1 for the group $X^i$ in formula (I) and the index "$a^i$" is an integer from 0 to 3.

In a vinyl polymer bearing at least one carbosiloxane dendrimer-based unit, the polymerization ratio between the components (A) and (B), in terms of the weight ratio between (A) and (B), may be within a range from 0/100 to 99.9/0.1, or even from 0.1/99.9 to 99.9/0.1 and preferably within a range from 1/99 to 99/1. A ratio of the component (A) to the component (B) of 0/100 means that the compound becomes a homopolymer of component (B).

A vinyl polymer having at least one unit derived from carbosiloxane dendrimer can be obtained by the copolymerization of the components (A) and (B) or by the polymerization of the component (B) alone.

The polymerization can be a radical polymerization or an ionic polymerization; however, the radical polymerization is preferred.

The polymerization may be performed by bringing about a reaction between the components (A) and (B) in a solution for a period of from 3 to 20 hours in the presence of a radical initiator at a temperature of from 50°C to 150°C.

A suitable solvent for this purpose is hexane, octane, decane, cyclohexane or a similar aliphatic hydrocarbon; benzene, toluene, xylene or a similar aromatic hydrocarbon; diethyl ether, dibutyl ether, tetrahydrofuran, dioxane or similar ethers; acetone, methyl ethyl ketone, methyl isobutyl ketone, diisobutyl ketone or similar ketones; methyl acetate, ethyl acetate, butyl acetate, isobutyl acetate or similar esters; methanol, ethanol, isopropanol, butanol or similar alcohols; octamethylcyclotetrasiloxane, decamethylcyclopentasiloxane, hexamethyldisiloxane, octamethyltrisiloxane or a similar organosiloxane oligomer.

A radical initiator can be any compound known in the art for conventional radical polymerization reactions. The specific examples of such radical initiators are 2,2'-azobis(isobutyronitrile), 2,2'-azobis(2-methylbutyronitrile), 2,2'-azobis(2,4-dimethylvaleronitrile) or analogous compounds of azobis type; benzoyl peroxide, lauroyl peroxide, tert-butyl peroxybenzoate, tert-butyl peroxy-2-ethylhexanoate or an analogous organic peroxide. These radical initiators can be used alone or in a combination of two or more. The radical initiators can be used in an amount of 0.1 to 5 parts by weight per 100 parts by weight of the components (A) and (B). A chain-transfer agent can be added. The chain-transfer agent can be 2-mercaptoethanol, butyl mercaptan, n-dodecyl mercaptan, (3-mercaptopropyl)trimethoxysilane, a polydimethylsiloxane possessing a mercaptopropyl group or an analogous compound of mercapto type; methylene chloride, chloroform, carbon tetrachloride, butyl bromide, (3-chloropropyl)trimethoxysilane or an analogous halogenated compound.

In the manufacture of the polymer of vinyl type, after the polymerization, the unreacted residual vinyl monomer can be removed under conditions of heating under vacuum.

To facilitate the preparation of starting material mixture for cosmetic products, the number-average molecular weight of the vinyl polymer bearing a carbosiloxane dendrimer may be chosen within the range between 3000 and 2 000 000 and preferably between 5000 and 800 000. It can be a liquid, a gum, a paste, a solid, a powder or any other form. The preferred forms are solutions formed by the dilution of a dispersion or of a powder in solvents.

The vinyl polymer can be a dispersion of a polymer of vinyl type having a carbosiloxane dendrimer structure in its molecular side chain, in a liquid such as a silicone oil, an organic oil, an alcohol or water.

The silicone oil may be a dimethylpolysiloxane with the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar unreactive linear silicone oils, and also hexamethylcyclotrisiloxane, octamethylcyclotetrasiloxane, decamethylcyclopentasiloxane, dodecamethylcyclohexasiloxane or a similar cyclic compound. In addition to the unreactive silicone oils, modified polysiloxanes containing functional groups such as silanol groups, amino groups and polyether groups on the ends or within the molecular side chains may be used.

The organic oils can be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetearyl alcohol, oleyl alcohol, avocado oil, almond oil, olive oil, cocoa oil, jojoba oil, gum oil, sunflower oil, soybean oil, camelia oil, squalane, castor oil, cottonseed oil, coconut oil, egg yolk oil, polypropylene glycol monooleate, neopentyl glycol 2-ethylhexanoate or an

analogous glycol ester oil; triglyceryl isostearate, the triglyceride of a fatty acid of coconut oil, or an analogous oil of a polyhydric alcohol ester; polyoxyethylene lauryl ether, polyoxypropylene cetyl ether or an analogous polyoxyalkylene ether.

The alcohol may be any type that is suitable for use in combination with a cosmetic product starting material. For example, it can be methanol, ethanol, butanol, isopropanol or analogous lower alcohols.

A solution or a dispersion of the alcohol should have a viscosity within the range from 10 to $10^9$ mPa at 25°C. To improve the sensory use properties in a cosmetic product, the viscosity should be within the range from 100 to $5 \times 10^8$ mPa.s.

The solutions and dispersions can be easily prepared by mixing a vinyl polymer having at least one unit derived from carbosiloxane dendrimer with a silicone oil, an organic oil, an alcohol or water. The liquids may be present in the step of polymerization of a polymer of vinyl type bearing at least one carbosiloxane dendrimer-based unit. In this case, the unreacted residual vinyl monomer should be completely removed by heat treatment of the solution or dispersion under atmospheric pressure or reduced pressure.

In the case of a dispersion, the dispersity of the polymer of vinyl type can be improved by adding a surfactant.

Such an agent may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and nonionic surfactants of polyester type, and also mixtures.

In addition, the solvents and dispersions may be combined with iron oxide suitable for use with cosmetic products, or a similar pigment, and also zinc oxide, titanium oxide, silicon oxide, mica, talc or similar mineral oxides in powder form. In the dispersion, a mean particle diameter of the polymer of vinyl type can be within a range of between 0.001 and 100 microns and preferably between 0.01 and 50 microns. The reason for this is that, outside the recommended range, a cosmetic product mixed with the emulsion will not have a nice enough feel on the skin or to the touch, or sufficient spreading properties or a pleasant feel.

A vinyl polymer contained in the dispersion or the solution may have a concentration in the range between 0.1% and 95% by weight and preferably between 5% and 85% by weight. However, to facilitate the handling and the preparation of the mixture, the range should preferably be between 10% and 75% by weight.

According to a preferred mode, a vinyl polymer that is suitable for use in the invention may be one of the polymers described in the examples of patent application EP 0 963 751.

According to a preferred embodiment, a vinyl polymer grafted with a carbosiloxane dendrimer may be the product of polymerization of:

of 0.1 to 99 parts by weight of one or more acrylate or methacrylate monomer(s); and
of 100 to 0.1 part by weight of an acrylate or methacrylate monomer of a tris[tri(trimethylsiloxy)silylethyldimethylsiloxy] silylpropyl carbosiloxane dendrimer.

According to one embodiment, a vinyl polymer bearing at least one carbosiloxane dendrimer-based unit may comprise a tris[tri(trimethylsiloxy)silylethyldimethylsiloxy]silylpropyl carbosiloxane dendrimer-based unit corresponding to one of the formulae:

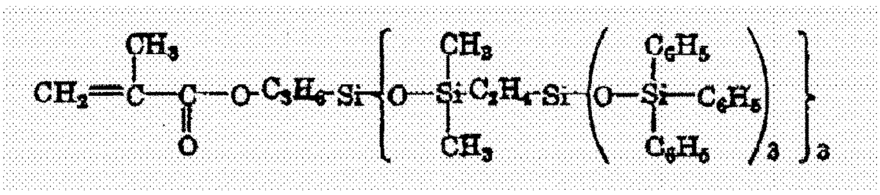

or

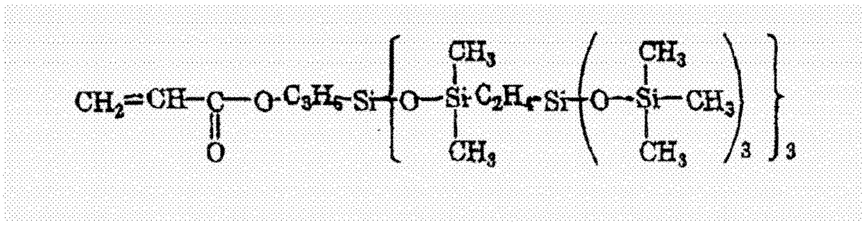

According to a preferred mode, a vinyl polymer bearing at least one carbosiloxane dendrimer-based unit used in the invention comprises at least one butyl acrylate monomer.

According to one embodiment, a vinyl polymer may also comprise at least one fluoro organic group. A fluorinated vinyl polymer can be one of the polymers described in the examples of application WO 03/045337.

According to a preferred embodiment, a vinyl polymer grafted in the sense of the present invention may be conveyed in an oil or a mixture of oils, which are preferably volatile, chosen in particular from silicone oils and hydrocarbon-based oils, and mixtures thereof.

According to a particular embodiment, a silicone oil that is suitable for use in the invention may be cyclopentasiloxane.

According to another particular embodiment, a hydrocarbon-based oil that is suitable for use in the invention may be isododecane.

Vinyl polymers grafted with at least one carbosiloxane dendrimer-based unit that may be particularly suitable for use in the present invention are the polymers sold under the names TIB 4-100®, TIB 4-101®, TIB 4-120®, TIB 4-130®, TIB 4-200®, FA 4002 ID® (TIB 4-202®), TIB 4-220 and FA 4001 CM® (TIB 4-230®) by the company Dow Corning. The polymers sold under the names FA 4002 ID® (TIB 4-202®) and FA 4001 CM® (TIB 4-230®) by the company Dow Corning will preferably be used.

Preferably, the vinyl polymer grafted with at least one carbosiloxane dendrimer-based unit that may be used in a composition of the invention is an acrylate/polytrimethyl siloxymethacrylate copolymer, especially the product sold in isododecane under the name Dow Corning FA 4002 ID® Silicone Acrylate by the company Dow Corning.

## V. Silicone acrylate copolymers

According to a particular embodiment, a composition used according to the invention may comprise, as hydrophobic film-forming polymer, at least one copolymer comprising carboxylate groups and polydimethylsiloxane groups.

In the present patent application, the term "copolymer comprising carboxylate groups and polydimethylsiloxane groups" means a copolymer obtained from (a) one or more carboxylic (acid or ester) monomers, and (b) one or more polydimethylsiloxane (PDMS) chains.

In the present patent application, the term "carboxylic monomer" means both carboxylic acid monomers and carboxylic acid ester monomers. Thus, the monomer (a) may be chosen, for example, from acrylic acid, methacrylic acid, maleic acid, fumaric acid, itaconic acid, crotonic acid, esters thereof and mixtures of these monomers. Mention may be made, as esters, of the following monomers: acrylate, methacrylate, maleate, fumarate, itaconate and/or crotonate. According to a preferred embodiment of the invention, the monomers in ester form are more particularly chosen from linear or branched, preferably $C_1$-$C_{24}$ and better still $C_1$-$C_{22}$ alkyl acrylates and methacrylates, the alkyl radical preferably being chosen from methyl, ethyl, stearyl, butyl and 2-ethylhexyl radicals, and mixtures thereof.

Thus, according to a particular embodiment of the invention, the copolymer comprises as carboxylate groups at least one group chosen from acrylic acid and methacrylic acid, and methyl, ethyl, stearyl, butyl or 2-ethylhexyl acrylate or methacrylate, and mixtures thereof.

In the present patent application, the term "polydimethylsiloxanes" (also known as organopolysiloxanes and abbreviated as PDMS) is intended to denote, in accordance with what is generally accepted, any organosilicon polymer or oligomer of linear structure, of variable molecular weight, obtained by polymerization and/or polycondensation of suitably functionalized silanes, and formed essentially from a repetition of main units in which the silicon atoms are linked together via oxygen atoms (siloxane bond ≡Si-O-Si≡), comprising trimethyl radicals directly linked via a carbon atom to said silicon atoms. The PDMS chains which can be used to obtain the copolymer used according to the invention comprise at least one radically polymerizable group, preferably located on at least one of the ends of the chain, that is to say that the PDMS can, for example, have a radically polymerizable group on the two ends of the chain or have a radically polymerizable group on one end of the chain and a trimethylsilyl end group on the other end of the chain. The polymerizable radical group may especially be an acrylic or methacrylic group, in particular a group $CH_2 = CR_1 - CO - O - R_2$, in which $R^1$ represents a hydrogen or a methyl group and $R_2$ represents $-CH_2-$,$-(CH_2)_n-$ with n = 3, 5, 8 or 10, $-CH_2-CH(CH_3)-CH_2-$, $-CH_2-CH_2-O-CH_2-CH_2-$, $-CH_2-CH_2-O-CH_2-CH_2-CH(CH_3)-CH_2-$, $-CH_2-CH_2-O-CH_2-$ $CH_2-O-CH_2-CH_2-CH_2-$.

The copolymers used in the composition of the invention are generally obtained according to the usual methods of polymerization and grafting, for example by radical polymerization (A) of a PDMS comprising at least one radically polymerizable group (for example on one of the ends of the chain or on both ends) and (B) of at least one carboxylic monomer, as described, for example, in the documents US-A-5 061 481 and US-A-5 219 560.
The copolymers obtained generally have a molecular weight ranging from about 3000 to 200 000 and preferably from about 5000 to 100 000.
The copolymer used in the composition of the invention can be provided as is or in dispersed form in a solvent, such as lower alcohols comprising from 2 to 8 carbon atoms, for instance isopropyl alcohol, or oils, for instance volatile silicone oils (for example cyclopentasiloxane).
As copolymers that may be used in the composition of the invention, mention may be made, for example, of copolymers of acrylic acid and of stearyl acrylate bearing polydimethylsiloxane grafts, copolymers of stearyl methacrylate bearing polydimethylsiloxane grafts, copolymers of acrylic acid and of stearyl methacrylate bearing polydimethylsiloxane grafts, copolymers of methyl methacrylate, butyl methacrylate, 2-ethylhexyl acrylate and stearyl methacrylate bearing polydimethylsiloxane grafts. As copolymers that may be used in the composition of the invention, mention may be made in particular of the copolymers sold by the company Shin-Etsu under the names KP-561® (CTFA name: acrylates/dimethicone), KP-541® in which the copolymer is dispersed at 60% by weight in isopropyl alcohol (CTFA name: acrylates/dimethicone and isopropyl alcohol), and KP-545® in which the copolymer is dispersed at 30% in cyclopentasiloxane (CTFA name: acrylates/dimethicone and cyclopentasiloxane). According to a preferred embodiment of the invention, KP561® is preferably used; this copolymer is not dispersed in a solvent, but is in waxy form, its melting point being about 30°C.
Mention may also be made of the grafted copolymer of polyacrylic acid and dimethylpolysiloxane dissolved in isododecane, sold by the company Shin-Etsu under the name KP-550®.
Advantageously, a composition according to the invention may comprise, as hydrophobic film-forming polymer, at least one trimethyl siloxysilicate resin.

## COSMETIC APPLICATIONS

It is a matter of routine operations for a person skilled in the art to adjust the natures and the amounts of the additives present in the compositions in accordance with the invention so that the cosmetic properties desired for the latter are not affected thereby.
According to one embodiment, a composition of the invention may advantageously be in the form of a composition for caring for the skin and/or keratin fibres, the body or the face, in particular the face.
According to another embodiment, a composition of the invention can advantageously be in the form of a composition for making up keratin materials, in particular the skin of the body or of the face, in particular of the face.
Thus, according to a sub-mode of this embodiment, a composition of the invention may advantageously be in the form of a makeup base composition.
A composition of the invention may advantageously be in the form of a foundation.
According to another sub-mode of this embodiment, a composition of the invention may advantageously be in the form of a composition for making up the skin and in particular the face. It may thus be an eyeshadow or a face powder.
According to yet another sub-mode of this embodiment, a composition of the invention may advantageously be in the form of a product for making up the lips, in particular a lipstick.
According to yet another sub-mode of this embodiment, a composition of the invention may advantageously be in the form of a product for making up and/or caring for the eyebrows.
Such compositions are prepared in particular according to the general knowledge of a person skilled in the art.
Throughout the description, including the claims, the term *"comprising a"* should be understood as being synonymous with *"comprising at least one"*, unless otherwise specified.
The terms *"between... and..."* and *"ranging from...* to..." should be understood as being inclusive of the limits, unless otherwise specified.
The invention is illustrated in more detail by the examples and figures presented below. Unless otherwise indicated, the amounts shown are expressed as percentages by weight.

## Methodology for the oscillating dynamic rheology measurements

These are harmonic-regime rheology measurements for measuring the elastic modulus.
The measurements are taken using a Haake RS600 rheometer on a product at rest, at 25°C with a plate-plate rotor Ø 60 mm and a 2 mm gap.
The harmonic-regime measurements make it possible to characterize the viscoelastic properties of the products. The technique consists in subjecting a material to a stress which varies sinusoidally over time and in measuring the response of the material to this stress. In a range in which the behaviour is linear viscoelastic behaviour (zone in which

the strain is proportional to the stress), the stress ($\tau$) and the strain ($\gamma$) are two sinusoidal functions of time which are written in the following manner:

$$\tau(t) = \tau_0 \sin(\omega t)$$

$$\gamma(t) = \gamma_0 \sin(\omega t + \delta)$$

in which:

$\tau_0$ represents the maximum amplitude of the stress (Pa);
$\gamma_0$ represents the maximum amplitude of the strain (-);
$\omega = 2\Pi N$ represents the angular frequency ($rad.s^{-1}$) with N representing the frequency (Hz); and
$\delta$ represents the phase shift of the stress relative to the strain (rad).

Thus, the two functions have the same angular frequency, but they are shifted by an angle $\delta$. Depending on the phase shift $\delta$ between $\tau(t)$ and $y(t)$, the behaviour of the system may be apprehended:

- if $\delta = 0$, the material is purely elastic;
- if $\delta = \Pi/2$, the material is purely viscous (Newtonian fluid); and
- if $0 < \delta < \Pi/2$, the material is viscoelastic.

In general, the stress and the strain are written in complex form:

$$\tau^*(t) = \tau_0 e^{i\omega t}$$

$$\gamma^*(t) = \gamma_0 e^{(i\omega t + \delta)}$$

A complex stiffness modulus, representing the overall resistance of the material to the strain, whether it is of elastic or viscous origin, is then defined by:

$$G^* = \tau^* / \gamma^* = G' + iG''$$

in which:

G' is the storage modulus or elastic modulus, which characterizes the energy stored and totally restituted during a cycle, $G' = (\tau_0/\gamma_0) \cos \delta$; and
G" is the loss modulus or viscous modulus, which characterizes the energy dissipated by internal friction during a cycle, $G'' = (\tau_0/\gamma_0) \sin \delta$.

The parameter retained is the mean stiffness modulus G* recorded at the plateau measured at a frequency of 1 Hz.

**Example 1 of foundation in gel-gel form**

| Phases | Ingredients | Example 1 (invention) | Example 1A (invention) |
|---|---|---|---|
| A | Trimethyl siloxysilicate | 5 | 5 |

(continued)

| Phases | Ingredients | Example 1 (invention) | Example 1A (invention) |
|---|---|---|---|
| B | Cyclohexasiloxane | 7 | 7 |
| | Titanium dioxide Isopropyl Titanium Triisostearate (BTD-401® from Kobo) | 11.91 | 11.91 |
| | Iron oxides (and) Isopropyl titanium Triisostearate (BRO-I2® from Kobo) | 0.43 | 0.43 |
| | Iron oxides (and) Isopropyl titanium Triisostearate (BYO-12® from Kobo) | 1.46 | 1.46 |
| | Iron oxides (and) Isopropyl Titanium Triisostearate (BBO-I2® from Kobo) | 0.2 | 0.2 |
| B1 | Polysilicone-11 (Gransil RPS-D6® from Grant Industries) | 11.5 | 11.5 |
| C | Water | qsp 100 | qsp 100 |
| | Butylene glycol | 5 | 5 |
| | Glycerol | 5 | 5 |
| | Isostearic acid (Prisorine 3505-LQ-(GD)® from Croda) | 0.2 | 0.2 |
| | Phenoxyethanol | 0.7 | 0.7 |
| | Glyceryl caprylate (Dermosoft GMCY®- Dr Straetemans) | 0.5 | 0.5 |
| | Propanediol | 3 | 3 |
| C1 | Hydroxyethyl acrylate/sodium acryloyldimethyltaurate copolymer (Sepinov EMT 10® from SEPPIC) | 1.25 | 1.25 |
| D | Polymethyl methacrylate (Microsphere M-310 from Matsumoto Yushi-Seiyaku) | 1.8 | 1.8 |
| | Lauryl methacrylate/Glycol Dimethacrylate Crosspolymer (Polytrap 6603 Adsorber® from Amcol Health & Beauty Solutions) | 0.5 | 0.5 |
| | Silica silylate (Dow Corning VM-2270 Aerogel Fine Particles) | 0.35 | 0.35 |

**Procedure:**

C was placed in a beaker with moderate stirring using a Rayneri blender. C1 was added, and the gel thickened and became homogeneous after 5 minutes of vigorous stirring with the Rayneri blender. Phase B was ground in 3 treatments (large, medium and then small aperture) using a three-roll mill. The mixture was then placed under slow stirring with the Rayneri blender, followed by addition of B1. When the mixture was homogeneous, A was added very slowly by sprinkling with slow stirring using the Rayneri blender for about 45 minutes until the mixture was homogeneous. Mixture A+B+B1 was added to C+C1 with vigorous stirring using the Rayneri blender for 2 x 2 minutes. The composition then became homogeneous. D was added with vigorous stirring using the Rayneri blender for 2 x 10 minutes until the fillers were fully dispersed in the mixture.

The macroscopic appearance of formulae 1 and 1A was measured with the naked eye after 2 months at room temperature (25°C). There were placed on glass plmates. A smooth and glossy aspect of a formulation corresponds to a homogeneous macroscopic composition.

Exemples 3 to 5

| Phase | Ingredients | Exemple 3 (outside the invention) | Exemple 4 (invention) | Exemple 5 (invention) |
|---|---|---|---|---|
| A | WATER | qsp 100 | qsp 100 | qsp 100 |
| | GLYCERIN | 5,28 | 5,28 | 5,28 |
| | PHENOXYETHANOL | 0,78 | 0,78 | 0,78 |
| B | DIMETHICONE | 8 | 8 | 8 |
| | DIMETHICONE (and) DIMETHICONE CROSSPOLYMER | 14 | 14 | 14 |
| | ISOSTEARIC ACID | 0 | 0,2 | 0 |
| | OLEIC ACID | 0 | 0 | 0,2 |
| C | HYDROXYETHYL ACRYLATE/SODIUM ACRYLOYLDIMETHYL TAURATE COPOLYMER | 2,4 | 2,4 | 2,4 |
| D | IRON OXIDES (and) DISODIUM STEAROYL GLUTAMATE (and) ALUMINUM HYDROXIDE (CI 77492) | 1,899 | 1,899 | 1,899 |
| | IRON OXIDES (and) DISODIUM STEAROYL GLUTAMATE (and) ALUMINUM HYDROXIDE (CI 77491) | 0,396 | 0,396 | 0,396 |
| | IRON OXIDES (and) DISODIUM STEAROYL GLUTAMATE (and) ALUMINUM HYDROXIDE (CI 77499) | 0,099 | 0,099 | 0,099 |
| | TITANIUM DIOXIDE (and) DISODIUM STEAROYL GLUTAMATE (and) ALUMINUM HYDROXIDE (CI 77891) | 15,597 | 15,597 | 15,597 |
| | SYNTHETIC FLUORPHLOGOPITE | 1,999 | 1,999 | 1,999 |

The ingredients of the aqueous phase A were weighed out and stirred using a Rayneri blender at room temperature. Then, the ingredients of the oily phase B and hydrophilic gelling agent phase C were added to the aqueous phase and mixed under Rayneri stirring. A white and translucid gel gel was formed. When the gel gel was smooth and uniform, the phase D was added under strong stirring until complete homogeneization using a spatula. The walls and base of the beaker were scraped using a spatula and the mixture was left to homogenize with vigorous stirring at room temperature until completely homogeneous. The macroscopic appearance of formulae **3 to 5** was measured with the naked eye after 24 hours at room temperature (25°C). There were placed on glass plates. A smooth and glossy aspect of a formulation corresponds to a homogeneous macroscopic composition.

| Examples | Macroscopic appearance after 24 hours at 25°C |
|---|---|
| **Ex. 3 (outside the invention) Without liquid fatty acid** | Less glossy and less homogeneous |
| **Ex. 4 (invention) With Isostearic acid** | Smooth and glossy homogeneous texture |
| **Ex. 5 (invention) With Oleic acid** | Smooth and glossy homogeneous texture |

**Claims**

1. Composition **of gel-gel type,** especially comprising a physiologically acceptable medium, especially for coating keratin materials, more particularly for making up and/or caring for keratin materials, containing:

- at least one aqueous phase gelled with at least one **synthetic** hydrophilic gelling agent selected **from 2-**

**acrylamido-2-methylpropanesulfonic acid polymers and copolymers**; and
- at least one oily phase gelled with at least one lipophilic gelling agent **which is an organopolysiloxane elastomer;** said phases forming therein a macroscopically homogeneous mixture and said composition also comprising:
- at least one hydrophobic-coated pigment; and
- at least one liquid fatty acid of formula (1) below:

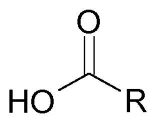

in which R is chosen from:

a) a saturated, branched $C_{14}$-$C_{22}$, preferably $C_{18}$, alkyl group, or
b) an alkyl group comprising at least one linear or branched $C_{14}$-$C_{22}$, preferably $C_{18}$, double bond; and

- **optionally** at least one glycol compound of formula (2) below:

$$CH_3\text{--}[CH_2CH_2]_m\text{--}[COO]_n\text{-}CH_2\text{-}CH(OH)\text{-}CH_2OH \qquad (2)$$

in which:

- m is between 2 and 4
- n is equal to 0 or 1;

said gelled oily phase comprising at least one polar oil when the composition comprises at least one glycol compound of formula (2).

**2.** Composition according to **claim 1,** comprising as lipophilic gelling agent at least one organopolysiloxane elastomer chosen from Dimethicone Crosspolymer, Dimethicone (and) Dimethicone Crosspolymer, Vinyl Dimethicone Crosspolymer, Dimethicone/Vinyl Dimethicone Crosspolymer, Dimethicone Crosspolymer-3, and in particular Dimethicone Crosspolymer and Dimethicone (and) Dimethicone Crosspolymer.

**3.** Composition according to any one of the preceding claims, containing the aqueous and oily phases in an aqueous phase/oily phase weight ratio of from 95/5 to 5/95 and preferably from 30/70 to 80/20.

**4.** Composition according to any one of the preceding claims, in which the hydrophobic coated pigments are hydrophobic coated pigments of iron oxide and/or of titanium dioxide.

**5.** Composition according to any one of the preceding claims, in which the hydrophobic-coated pigments are coated with at least one compound chosen from N-acylamino acids or salts thereof, isopropyl titanium triisostearate; silicone surface agents; natural plant or animal waxes; hydrogenated lecithin, fatty esters; and mixtures thereof.

**6.** Composition according to any one of the preceding claims, in which the hydrophobic-coated pigments are titanium dioxides and/or iron oxides coated:

- with an N-acylamino acid and/or a salt thereof, more particularly with a glutamic acid derivative and/or a salt thereof, in particular aluminium stearoyl glutamate; or
- with isopropyl titanium triisostearate.

**7.** Composition according to any one of the preceding claims, in which the liquid fatty acid of formula (1) is chosen from:

- isostearic acid
- oleic acid
- linoleic acid

- linolenic acid; and
- mixtures thereof, and more particularly is isostearic acid.

**8.** Composition according to any one of the preceding claims, in which the compound of formula (2) is chosen from caprylyl glycol, glyceryl caprylate, glyceryl caprylate/caprate mixtures, and mixtures thereof, and more particularly is chosen from caprylyl glycol, glyceryl caprylate, and mixtures thereof.

**9.** Composition according to any one of the preceding claims, in which the polar oil is chosen from liquid lipophilic organic UV-screening agents, preferably chosen from:

- liquid lipophilic β,β-diphenylacrylate compounds
- liquid lipophilic salicylate compounds
- liquid lipophilic cinnamate compounds
- mixtures thereof, and more particularly is ethylhexyl methoxycinnamate.

**10.** Composition according to any one of the preceding claims, in which the polar oil is chosen from:

- volatile cyclic silicone oils having a viscosity at room temperature of less than 8 cSt and containing in particular from 4 to 7 silicon atoms, these silicones optionally comprising alkyl or alkoxy groups containing from 1 to 10 carbon atoms, such as hexamethylcyclotrisiloxane, octamethylcyclotetrasiloxane, decamethylcyclopentasiloxane and dodecamethylcyclohexasiloxane (cyclohexasiloxane);
- polydimethylsiloxanes comprising aliphatic groups, in particular alkyl or alkoxy groups, which are pendent and/or at the end of the silicone chain, in particular caprylyl methicone.

**11.** Composition according to any one of the preceding claims, in which the polar oil is chosen from phenyl silicone oils.

**12.** Composition according to any one of the preceding claims, in which the polar oil is chosen from ethylhexyl methoxycinnamate, dodecamethylcyclohexasiloxane, caprylyl methicone, and mixtures thereof.

**13.** Process for preparing a composition as defined in any one of the preceding claims, comprising at least one step of mixing:

- aqueous phase gelled with at least one synthetic hydrophilic gelling agent **selected from 2-acrylamido-2-methylpropanesulfonic acid polymers and copolymers;** and
- oily phase gelled with at least one lipophilic gelling agent **which is an organopolysiloxane elastomer** under conditions suitable for obtaining a macroscopically homogeneous mixture; said composition also comprising at least one hydrophobic-coated pigment and at least one compound of formula (1) **and optionally at least one compound of formula (2).**

**14.** Process according to Claim **13,** comprising a step of mixing at least three or even more gelled phases.

**15.** Process according to either of Claims **13 and 14,** in which the mixing is performed at room temperature.

**16.** Cosmetic process for making up and/or caring for a keratin material, in particular the skin and/or the lips and/or the eyebrows, and keratin fibres, especially the eyebrows, comprising at least one step which consists in applying to said keratin material a composition as defined according to any one of Claims 1 to **12.**

**Patentansprüche**

**1.** Zusammensetzung vom Gel-Gel-Typ, die insbesondere ein physiologisch unbedenkliches Medium umfasst, insbesondere zum Beschichten von Keratinmaterialien, spezieller zum Schminken und/oder Pflegen von Keratinmaterialien, enthaltend:

- mindestens eine wässrige Phase, die mit mindestens einem synthetischen hydrophoben Gelierungsmittel, das aus 2-Acrylamido-2-methyl-propansulfonsäure-Polymeren und -Copolymeren ausgewählt ist, geliert ist; und
- mindestens eine ölige Phase, die mit mindestens einem lipophilen Gelierungsmittel, bei dem es sich um ein Organopolysiloxan-Elastomer handelt, geliert ist; wobei die Phasen darin eine makroskopisch homogene Mischung bilden und die Zusammensetzung außerdem Folgendes umfasst:
- mindestens ein hydrophob beschichtetes Pigment und
- mindestens eine flüssige Fettsäure der nachstehenden Formel (1):

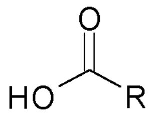

wobei R aus

a) einer gesättigten, verzweigten $C_{14}$-$C_{22}$- und vorzugsweise $C_{18}$-Alkylgruppe oder
b) einer Alkylgruppe mit mindestens einer linearen oder verzweigten $C_{14}$-$C_{22}$- und vorzugsweise $C_{18}$-Doppelbindung
ausgewählt ist; und

- gegebenenfalls mindestens eine Glykolverbindung der nachstehenden Formel (2):

$$CH_3\text{--}[CH_2CH_2]_m\text{--}[COO]_n\text{-}CH_2\text{-}CH(OH)\text{-}CH_2OH \qquad (2)$$

wobei:

- m zwischen 2 und 4 liegt;
- n gleich 0 oder 1 ist;

wobei die pegylierte ölige Phase mindestens ein polares Öl umfasst, wenn die Zusammensetzung mindestens eine Glykolverbindung der Formel (2) umfasst.

**2.** Zusammensetzung nach Anspruch 1, die als lipophiles Gelierungsmittel mindestens ein Organopolysiloxan-Elastomer umfasst, das aus Dimethicone Crosspolymer, Dimethicone (und) Dimethicone Crosspolymer, Vinyl Dimethicone Crosspolymer, Dimethicone/Vinyl Dimethicone Crosspolymer, Dimethicone Crosspolymer-3 und insbesondere Dimethicone Crosspolymer und Dimethicone (und) Dimethicone Crosspolymer ausgewählt ist.

**3.** Zusammensetzung nach einem der vorhergehenden Ansprüche, die die wässrige Phase und die ölige Phase in einem Gewichtsverhältnis von wässriger Phase zu öliger Phase von 95/5 bis 5/95 und vorzugsweise von 30/70 bis 80/20 enthält.

**4.** Zusammensetzung nach einem der vorhergehenden Ansprüche, wobei es sich bei den hydrophob beschichteten Pigmenten um hydrophob beschichtete Pigmente von Eisenoxid und/oder von Titandioxid handelt.

**5.** Zusammensetzung nach einem der vorhergehenden Ansprüche, wobei die hydrophob beschichteten Pigmente mit mindestens einer Verbindung, die aus N-Acylaminosäuren oder Salzen davon, Isopropyltitantriisostearat, Silikon-Oberflächenmitteln, natürlichen pflanzlichen oder tierischen Wachsen, hydriertem Lecithin, Fettsäureestern und Mischungen davon ausgewählt ist, beschichtet sind.

**6.** Zusammensetzung nach einem der vorhergehenden Ansprüche, wobei es sich bei den hydrophob beschichteten Pigmenten um Titandioxide und/oder Eisenoxide, die

- mit einer N-Acylaminosäure oder einem Salz davon, spezieller mit einem Glutaminsäurederivat und/oder einem Salz davon, insbesondere Aluminiumstearoylglutamat, oder
- mit Isopropyltitantriisostearat
beschichtet sind, handelt.

**7.** Zusammensetzung nach einem der vorhergehenden Ansprüche, wobei die flüssige Fettsäure der Formel (1) aus

- Isostearinsäure,
- Ölsäure,

- Linolsäure,
- Linolensäure und
- Mischungen davon

ausgewählt ist und spezieller Isostearinsäure ist.

**8.** Zusammensetzung nach einem der vorhergehenden Ansprüche, wobei die Verbindung der Formel (2) aus Caprylylglykol, Glycerylcaprylat, Glycerylcaprylat/caprat-Mischungen und Mischungen davon ausgewählt ist und spezieller aus Caprylylglykol, Glycerylcaprylat und Mischungen davon ausgewählt ist.

**9.** Zusammensetzung nach einem der vorhergehenden Ansprüche, wobei das polare Öl aus flüssigen lipophilen organischen UV-Filtersubstanzen ausgewählt ist, vorzugsweise aus

- flüssigen lipophilen β,β-Diphenylacrylat-Verbindungen,
- flüssigen lipophilen Salicylat-Verbindungen,
- flüssigen lipophilen Cinnamat-Verbindungen,
- Mischungen davon

ausgewählt ist und spezieller Ethylhexylmethoxycinnamat ist.

**10.** Zusammensetzung nach einem der vorhergehenden Ansprüche, wobei das polare Öl aus

- flüchtigen cyclischen Silikonölen, die bei Raumtemperatur eine Viskosität von weniger als 8 cSt aufweisen und insbesondere 4 bis 7 Siliciumatome enthalten, wobei diese Silikone gegebenenfalls Alkyl- oder Alkoxygruppen mit 1 bis 10 Kohlenstoffatomen umfassen, wie Hexamethylcyclotrisiloxan, Octamethylcyclotetrasiloxan, Decamethylcyclopentasiloxan und Dodecamethylcyclohexasiloxan (Cyclohexasiloxan);
- Polydimethylsiloxanen mit aliphatischen Gruppen, insbesondere Alkyl- oder Alkoxygruppen, die seitenständig sind und/oder am Ende der Silikonkette vorliegen, insbesondere Caprylylmethicon, ausgewählt ist.

**11.** Zusammensetzung nach einem der vorhergehenden Ansprüche, wobei das polare Öl aus Phenylsilikonölen ausgewählt ist.

**12.** Zusammensetzung nach einem der vorhergehenden Ansprüche, wobei das polare Öl aus Ethylhexylmethoxycinnamat, Dodecamethylcyclohexasiloxan, Caprylylmethicon und Mischungen davon ausgewählt ist.

**13.** Verfahren zur Herstellung einer Zusammensetzung gemäß einem der vorhergehenden Ansprüche, umfassend mindestens einen Schritt des Mischens von

- wässriger Phase, die mit mindestens einem synthetischen hydrophoben Gelierungsmittel, das aus 2-Acrylamido-2-methylpropansulfonsäure-Polymeren und -Copolymeren ausgewählt ist, geliert ist; und
- öliger Phase, die mit mindestens einem lipophilen Gelierungsmittel, bei dem es sich um ein Organopolysiloxan-Elastomer handelt, geliert ist, unter Bedingungen, die zum Erhalt einer makroskopisch homogenen Mischung geeignet sind; wobei die Zusammensetzung außerdem mindestens ein hydrophob beschichtetes Pigment und mindestens eine Verbindung der Formel (1) und gegebenenfalls mindestens eine Verbindung der Formel (2) umfasst.

**14.** Verfahren nach Anspruch 13, umfassend einen Schritt des Mischens von mindestens drei oder noch mehr gelierten Phasen.

**15.** Verfahren nach einem der Ansprüche 13 und 14, wobei das Mischen bei Raumtemperatur durchgeführt wird.

**16.** Kosmetisches Verfahren zum Schminken und/oder Pflegen für ein Keratinmaterial, insbesondere die Haut und/oder die Lippen und/oder die Augenbrauen, und Keratinfasern, insbesondere die Augenbrauen, umfassend mindestens einen Schritt, der darin besteht, dass man auf das Keratinmaterial eine Zusammensetzung gemäß einem der Ansprüche 1 bis 12 aufbringt.

## Revendications

**1.** Composition de type gel-gel, notamment comprenant un milieu physiologiquement acceptable, notamment pour le

revêtement de matériaux de kératine, plus particulièrement pour le maquillage et/ou l'entretien de matériaux de kératine, contenant :

- au moins une phase aqueuse gélifiée avec au moins un agent gélifiant hydrophile synthétique choisi parmi des polymères et des copolymères d'acide 2-acrylamido-2-méthylpropanesulfonique ; et
- au moins une phase huileuse gélifiée avec au moins un agent gélifiant lipophile qui est un élastomère d'organopolysiloxane ; lesdites phases formant dans celle-ci un mélange macroscopiquement homogène et ladite composition comprenant également :
- au moins un pigment à revêtement hydrophobe ; et
- au moins un acide gras liquide de formule (1) ci-dessous :

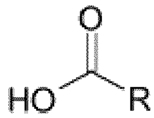

dans laquelle R est choisi parmi :

a) un groupe alkyle saturé, ramifié en $C_{14-22}$, préférablement en $C_{18}$, ou
b) un groupe alkyle comprenant au moins une double liaison linéaire ou ramifiée en $C_{14-22}$, préférablement en $C_{18}$ ; et
- éventuellement au moins un composé de glycol de formule (2) ci-dessous :

$$CH_3\text{--}[CH_2CH_2]_m\text{--}[COO]_n\text{-}CH_2\text{-}CH(OH)\text{-}CH_2OH \qquad (2)$$

dans laquelle :

- m est compris entre 2 et 4
- n est égal à 0 ou 1 ;

ladite phase huileuse gélifiée comprenant au moins une huile polaire lorsque la composition comprend au moins un composé de glycol de formule (2).

**2.** Composition selon la revendication 1, comprenant en tant qu'agent gélifiant lipophile au moins un élastomère d'organopolysiloxane choisi parmi Dimethicone Crosspolymer, Dimethicone (and) Dimethicone Crosspolymer, Vinyl Dimethicone Crosspolymer, Dimethicone/Vinyl Dimethicone Crosspolymer, Dimethicone Crosspolymer-3, et en particulier Dimethicone Crosspolymer et Dimethicone (and) Dimethicone Crosspolymer.

**3.** Composition selon l'une quelconque des revendications précédentes, contenant les phases aqueuses et huileuses en un rapport pondéral phase aqueuse/phase huileuse allant de 95/5 à 5/95 et préférablement de 30/70 à 80/20.

**4.** Composition selon l'une quelconque des revendications précédentes, dans laquelle les pigments à revêtement hydrophobe sont des pigments à revêtement hydrophobe d'oxyde de fer et/ou de dioxyde de titane.

**5.** Composition selon l'une quelconque des revendications précédentes, dans laquelle les pigments à revêtement hydrophobe sont revêtus avec au moins un composé choisi parmi des acides aminés N-acylés ou des sels correspondants, du triisostéarate d'isopropyltitane ; des agents de surface de type silicone ; des cires naturelles végétales ou animales ; de la lécithine hydrogénée, des esters gras ; et des mélanges correspondants.

**6.** Composition selon l'une quelconque des revendications précédentes, dans laquelle les pigments à revêtement hydrophobe sont des dioxydes de titane et/ou des oxydes de fer revêtus :

- avec un acide aminé N-acylé et/ou un sel correspondant, plus particulièrement avec un dérivé d'acide glutamique et/ou un sel correspondant, en particulier du glutamate de stéaroyle d'aluminium ; ou
- avec du triisostéarate d'isopropyltitane.

**7.** Composition selon l'une quelconque des revendications précédentes, dans laquelle l'acide gras liquide de formule

(1) est choisi parmi :

- l'acide isostéarique,
- l'acide oléique,
- l'acide linoléique,
- l'acide linolénique ; et
- des mélanges correspondants, et est plus particulièrement l'acide isostéarique.

**8.** Composition selon l'une quelconque des revendications précédentes, dans lequel le composé de formule (2) est choisi parmi le caprylyle glycol, le caprylate de glycéryle, des mélanges caprylate/caprate de glycéryle, et des mélanges correspondants, et est choisi plus particulièrement parmi le caprylyle glycol, le caprylate de glycéryle, et des mélanges correspondants.

**9.** Composition selon l'une quelconque des revendications précédentes, dans laquelle l'huile polaire est choisie parmi des agents anti-UV organiques lipophiles liquides, préférablement choisie parmi :

- des composés de type $\beta,\beta$-diphénylacrylate lipophiles liquides
- des composés de type salicylate lipophiles liquides
- des composés de type cinnamate lipophiles liquides
- des mélanges correspondants, et est plus particulièrement un méthoxycinnamate d'éthylhexyle.

**10.** Composition selon l'une quelconque des revendications précédentes, dans laquelle l'huile polaire est choisie parmi :

- des huiles de silicone cyclique volatiles possédant une viscosité à température ambiante inférieure à 8 cSt et contenant en particulier de 4 à 7 atomes de silicium, ces silicones comprenant éventuellement des groupes alkyle ou alcoxy contenant de 1 à 10 atome(s) de carbone, telles que l'hexaméthylcyclotrisiloxane, l'octaméthylcyclotétrasiloxane, le décaméthylcyclopentasiloxane et le dodécaméthylcyclohexasiloxane (cyclohexasiloxane) ;
- des polydiméthylsiloxanes comprenant des groupes aliphatiques, en particulier des groupes alkyle ou alcoxy, qui sont pendants et/ou au niveau de la terminaison de la chaîne silicone, en particulier un caprylyle méthicone.

**11.** Composition selon l'une quelconque des revendications précédentes, dans laquelle l'huile polaire est choisie parmi des huiles de phényle silicone.

**12.** Composition selon l'une quelconque des revendications précédentes, dans laquelle l'huile polaire est choisie parmi un méthoxycinnamate d'éthylhexyle, un dodécaméthylcyclohexasiloxane, un caprylyle méthicone, et des mélanges correspondants.

**13.** Procédé pour la préparation d'une composition telle que définie selon l'une quelconque des revendications précédentes, comprenant au moins une étape de mélange :

- d'une phase aqueuse gélifiée avec au moins un agent gélifiant hydrophile synthétique choisi parmi des polymères et des copolymères d'acide 2-acrylamido-2-méthylpropanesulfonique ; et
- d'au moins une phase huileuse gélifiée avec au moins un agent gélifiant lipophile qui est un élastomère d'organopolysiloxane dans des conditions appropriées pour l'obtention d'un mélange macroscopiquement homogène ; ladite composition comprenant également au moins un pigment à revêtement hydrophobe et au moins un composé de formule (1) et éventuellement au moins un composé de formule (2).

**14.** Procédé selon la revendication 13, comprenant une étape de mélange d'au moins trois phases gélifiées ou encore plus.

**15.** Procédé selon l'une ou l'autre des revendications 13 et 14, dans lequel le mélange est réalisé à température ambiante.

**16.** Procédé cosmétique pour le maquillage et/ou l'entretien d'un matériau de kératine, en particulier de la peau et/ou des lèvres et/ou des sourcils, et de fibres de kératine, notamment les sourcils, comprenant au moins une étape qui consiste à appliquer audit matériau de kératine une composition telle que définie selon l'une quelconque des revendications 1 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9965455 A
- WO 9962497 A
- JP 2005112834 A
- WO 2008081175 A
- FR 2984736
- FR 3002444
- FR 3004343
- WO 14128680 A
- WO 2014128679 A
- WO 14128678 A
- EP 0815928 B1
- EP 295886 A
- EP 242219 A
- EP 285886 A
- EP 765656 A
- JP 61194009 A
- US 5538793 A
- WO 2008155059 A
- FR 0853634
- EP 0955039 A
- US 2004175338 A
- US 4578266 A
- JP H07196946 B
- US 5725882 A
- US 5209924 A
- US 4972037 A
- US 4981903 A
- US 4981902 A
- US 5468477 A
- US 5219560 A
- EP 0388582 A
- US 5246694 A
- EP 0486135 A
- JP H0586984 B
- JP 09188830 A
- JP 10158450 A
- JP 10158541 A
- JP 07258460 A
- JP 05017710 A
- JP 2003128788 B
- JP 2000191789 B
- US 2676182 A
- US 3627851 A
- US 3772247 A
- US 5248739 A
- US 5082706 A
- US 5319040 A
- US 5302685 A
- US 4935484 A
- US 5817302 A
- US 5110890 A
- WO 2005075542 A
- WO 04055081 A
- EP 749747 A
- EP 748746 A
- EP 923928 A
- EP 930060 A
- US 5874069 A
- US 5919441 A
- US 6051216 A
- US 5981680 A
- WO 03045337 A
- EP 963751 A
- JP 9171154 A
- JP HEI9171154 B
- EP 0963751 A
- US 5061481 A


### Non-patent literature cited in the description


- **ALMEIDA et al.** *Pharmaceutical Development and Technology,* 2008, vol. 13 (487), 488
- Remington: The Science and Practice of Pharmacy. 1995, 282
- **C.M. HANSEN.** The three-dimensional solubility parameters. *J. Paint Technol.,* 1967, vol. 39, 105
- **WITUCKI.** A silane primer, Chemistry and applications of alkoxy silanes. *Journal of Coatings Technology,* 1993, vol. 65 (822), 57-60
- **C. M. HANSEN.** The three-dimensional solubility parameters. *J. Paint Technol.,* 1967, vol. 39, 105
- Encyclopedia of Polymer Science and Engineering. John Wiley and Sons, 1989, vol. 15, 265-270
- a reference manual such as the Polymer Handbook. John Wiley, 1989